# EUROPEAN PATENT SPECIFICATION

(11) **EP 2 493 917 B1**
(45) Date of publication and mention of the grant of the patent: **11.12.2019**
(21) Application number: 10787709.4
(22) Date of filing: 26.10.2010
(51) Int. Cl.: C07K 14/47, G01N 33/50, G01N 33/574

(54) **BREAST TUMOR MARKERS AND METHODS OF USE THEREOF**
BRUSTTUMORMARKER UND DEREN VERWENDUNG
MARQUERS DE LA TUMEUR DU SEIN ET LEUR UTILISATION

(30) Priority: 26.10.2009 EP 09174060
(43) Date of publication of application: 05.09.2012
(60) Divisional application: 17209384.1
(73) Proprietor: EXTERNAUTICS S.P.A., 53100 Siena (IT)
(72) Inventor: GRIFANTINI, Renata, 53100 Siena (IT); PILERI, Piero, 53100 Siena (IT); CAMPAGNOLI, Susanna, 53100 Siena (IT); GRANDI, Alberto, 53100 Siena (IT); PARRI, Matteo, 53100 Siena (IT); PIERLEONI, Andrea, 53100 Siena (IT); NOGAROTTO, Renzo, 53100 Siena (IT)
(74) Representative: Banfi, Paolo
(86) International application number: PCT/EP2010/066146
(87) International publication number: WO 2011/051277

(56) References cited:
- WO-A2-2007/109376
- US-A1- 2006 204 503
- GARCIA-RUDAZ CECILIA ET AL: "Fxna, a novel gene differentially expressed in the rat ovary at the time of folliculogenesis, is required for normal ovarian histogenesis." DEVELOPMENT (CAMBRIDGE, ENGLAND) MAR 2007 LNKD- PUBMED:17267443, vol. 134, no. 5, March 2007 (2007-03), pages 945-957, XP002618324 ISSN: 0950-1991

## Description

The present invention relates to detection of breast tumors through antibodies capable of selectively interacting with the ERMP1 marker, as well as methods for tumor diagnosis using such ligand.

### Background of the invention

### Tumor markers (or biomarkers)

Tumor markers are substances that can be produced by tumor cells or by other cells of the body in response to cancer. In particular, a protein biomarker is either a single protein or a panel of different proteins, that could be used to unambiguously distinguish a disease state. Ideally, a biomarker would have both a high specificity and sensitivity, being represented in a significant percentage of the cases of given disease and not in healthy state.

Biomarkers can be identified in different biological samples, like tissue biopsies or preferably biological fluids (saliva, urine, blood-derivatives and other body fluids), whose collection does not necessitate invasive treatments. Tumor marker levels may be categorized in three major classes on the basis of their clinical use. Diagnostic markers can be used in the detection and diagnosis of cancer. Prognostics markers are indicative of specific outcomes of the disease and can be used to define predictive models that allow the clinicians to predict the likely prognosis of the disease at time of diagnosis. Moreover, prognosis markers are helpful to monitor the patient response to a drug therapy and facilitate a more personalized patient management. A decrease or return to a normal level may indicate that the cancer is responding to therapy, whereas an increase may indicate that the cancer is not responding. After treatment has ended, tumor marker levels may be used to check for recurrence of the tumor. Finally, therapeutic markers can be used to develop tumor-specific drugs or affinity ligand (i.e. antibodies) for a prophylactic intervention.

Currently, although an abnormal tumor marker level may suggest cancer, this alone is usually not enough to accurately diagnose cancer and their measurement in body fluids is frequently combined with other tests, such as a biopsy and radioscopic examination. Frequently, tumor marker levels are not altered in all of people with a certain cancer disease, especially if the cancer is at early stage. Some tumor marker levels can also be altered in patients with noncancerous conditions. Most biomarkers commonly used in clinical practice do not reach a sufficiently high level of specificity and sensitivity to unambiguously distinguish a tumor from a normal state.

To date the number of markers that are expressed abnormally is limited to certain types/subtypes of cancer, some of which are also found in other diseases. (http://www.cancer.gov/cancertopics/factsheet).

For instance, the human epidermal growth factor receptor (HER2) is a marker protein overproduced in about 20% of breast cancers, whose expression is typically associated with a more aggressive and recurrent tumors of this class.

### Routine screening test for tumor diagnosis

Screening tests are a way of detecting cancer early, before there are any symptoms. For a screening test to be helpful, it should have high sensitivity and specificity. Sensitivity refers to the test's ability to identify people who have the disease. Specificity refers to the test's ability to identify people who do not have the disease. Different molecular biology approaches such as analysis of DNA sequencing, small nucleotide polymorphyms, in situ hybridization and whole transcriptional profile analysis have done remarkable progresses to discriminate a tumor state from a normal state and are accelerating the knowledge process in the tumor field. However so far different reasons are delaying their use in the common clinical practice, including the higher analysis complexity and their expensiveness. Other diagnosis tools whose application is increasing in clinics include in situ hybridization and gene sequencing.

Currently, Immuno-HistoChemistry (IHC), a technique that allows the detection of proteins expressed in tissues and cells using specific antibodies, is the most commonly used method for the clinical diagnosis of tumor samples. This technique enables the analysis of cell morphology and the classification of tissue samples on the basis of their immunoreactivity. However, at present, IHC can be used in clinical practice to detect cancerous cells of tumor types for which protein markers and specific antibodies are available. In this context, the identification of a large panel of markers for the most frequent cancer classes would have a great impact in the clinical diagnosis of the disease.

### Anti-cancer therapies

In the last decades, an overwhelming number of studies remarkably contributed to the comprehension of the molecular mechanisms leading to cancer. However, this scientific progress in the molecular oncology field has not been paralleled by a comparable progress in cancer diagnosis and therapy. Surgery and/or radiotherapy are the still the main modality of local treatment of cancer in the majority of patients. However, these treatments are effective only at initial phases of the disease and in particular for solid tumors of epithelial origin, as is the case of colon, lung, breast, prostate and others, while they are not effective for distant recurrence of the disease. In some tumor classes, chemotherapy treatments have been developed, which generally relies on drugs, hormones and antibodies, targeting specific biological processes used by cancers to grow and spread. However, so far many cancer therapies had limited efficacy due to severity of side effects and overall toxicity. Indeed, a major effort in cancer therapy is the development of treatments able to target specifically tumor cells causing limited damages to surrounding normal cells thereby decreasing adverse side effects. Recent developments in cancer therapy in this direction are encouraging, indicating that in some cases a cancer specific therapy is feasible. In particular, the development and commercialization of humanized monoclonal antibodies that recognize specifically tumor-associated markers and promote the elimination of cancer is one of the most promising solutions that appears to be an extremely favorable market opportunity for pharmaceutical companies. However, at present the number of therapeutic antibodies available on the market or under clinical studies is very limited and restricted to specific cancer classes. So far licensed monoclonal antibodies currently used in clinics for the therapy of specific tumor classes, show only a partial efficacy and are frequently associated with chemotherapies to increase their therapeutic effect. Administration of Trastuzumab (Herceptin), a commercial monoclonal antibody targeting HER2, a protein overproduced in about 20% of breast cancers, in conjunction with Taxol adjuvant chemotherapy induces tumor remission in about 42% of the cases. Bevacizumab (Avastin) and Cetuximab (Erbitux) are two monoclonal antibodies recently licensed for use in humans, targeting the endothelial and epithelial growth factors respectively that, combined with adjuvant chemotherapy, proved to be effective against different tumor diseases. Bevacizumab proved to be effective in prolonging the life of patients with metastatic colorectal, breast and lung cancers. Cetuximab demostrated efficacy in patients with tumor types refractory to standard chemotherapeutic treatments (Adams G.P. and Weiner L.M. (2005) Monoclonal antibody therapy cancer. Nat Biotechnol. 23:1147-57). WO 2007/109376 discloses assays for detecting expression of ERMP1 in tissue samples from breast cancer patients.

In summary, available screening tests for tumor diagnosis are uncomfortable or invasive and this sometimes limits their applications. Moreover tumor markers available today have a limited utility in clinics due to either their incapability to detect all tumor subtypes of the defined cancers types and/or to distinguish unambiguously tumor vs. normal tissues. Similarly, licensed monoclonal antibodies combined with standard chemotherapies are not effective against the majority of cases. Therefore, there is a great demand for new tools to advance the diagnosis and treatment of cancer.

### Experimental approaches commonly used to identify tumor markers

Most popular approaches used to discover new tumor markers are based on genome-wide transcription profile or total protein content analyses of tumor. These studies usually lead to the identification of groups of mRNAs and proteins which are differentially expressed in tumors. Validation experiments then follow to eventually single out, among the hundreds of RNAs/proteins identified, the very few that have the potential to become useful markers. Although often successful, these approaches have several limitations and often, do not provide firm indications on the association of protein markers with tumor. A first limitation is that, since frequently mRNA levels not always correlate with corresponding protein abundance (approx. 50% correlation), studies based on transcription profile do not provide solid information regarding the expression of protein markers in tumor (1, 2, 3, 4).

A second limitation is that neither transcription profiles nor analysis of total protein content discriminate post-translation modifications, which often occur during oncogenesis. These modifications, including phosphorylations, acetylations, and glycosylations, or protein cleavages influence significantly protein stability, localization, interactions, and functions (5).

As a consequence, large scale studies generally result in long lists of differentially expressed genes that would require complex experimental paths in order to validate the potential markers. However, large scale genomic/proteomic studies reporting novel tumor markers frequently lack of confirmation data on the reported potential novel markers and thus do not provide solid demonstration on the association of the described protein markers with tumor.

The approach that we used to identify the protein markers included in the present invention is based on an innovative immuno-proteomic technology. In essence, a library of recombinant human proteins has been produced from E. coli and is being used to generate polyclonal antibodies against each of the recombinant proteins.

The screening of the antibodies library on Tissue microarrays (TMAs) carrying clinical samples from different patients affected by the tumor under investigation leads to the identification of specific tumor marker proteins. Therefore, by screening TMAs with the antibody library, the tumor markers are visualized by immuno-histochemistry, the classical technology applied in all clinical pathology laboratories. Since TMAs also include healthy tissues, the specificity of the antibodies for the tumors can be immediately appreciated and information on the relative level of expression and cellular localization of the markers can be obtained. In our approach the markers are subjected to a validation process consisting in a molecular and cellular characterization.

Altogether, the detection the marker proteins disclosed in the present invention selectively in tumor samples and the subsequent validation experiments leads to an unambiguous confirmation of the marker identity and confirm its association with defined tumor classes. Moreover this process provides an indication of the possible use of the proteins as tools for diagnostic or therapeutic intervention. For instance, markers showing a surface cellular localization could be both diagnostic and therapeutic markers against which both chemical and antibody therapies can be developed. Differently, markers showing a cytoplasmic expression could be more likely considered for the development of tumor diagnostic tests and chemotherapy/small molecules treatments.

### Summary of the invention

The present invention provides new means for the detection and treatment of breast tumors, based on the use of antibodies to a protein marker specific for these tumor types, namely the endoplasmic reticulum metallopeptidase 1 (ERMP1).

The invention also provides a method for the diagnosis of breast tumor, comprising a step of detecting the ERMP1 marker in a biological sample, e.g. in a tissue sample of a subject suspected of having or at risk of developing malignancies or susceptible to cancer recurrences.

Also disclosed are antibodies capable of selectively interacting with the ERMP1 marker.

### Detailed disclosure of the invention

The present invention provides antibodies that specifically bind to ERMP1 protein and their use in the diagnosis of breast cancer.

Endoplasmic reticulum metallopeptidase 1 (ERMP1, synonyms: FLJ23309, FXNA, KIAA1815; GENE ID: ENSG00000099219; Transcript IDs: ENST00000214893, ENST00000339450, ENST00000381506; Protein IDs: ENSP00000214893, ENSP00000340427, ENSP00000370917) is a transmembrane metallopeptidase, so far described as localized to the endoplasmic reticulum. ERMP1 transcript has been found differentially expressed in the rat ovary at the time of folliculogenesis. A lower level of ERMP1 transcript in the rat ovary resulted in substantial loss of primordial, primary and secondary follicles, and structural disorganization of the ovary, suggesting that is required for normal ovarian histogenesis (9). ERMP1 has been also included in a patent application (Publication number US 2003064439) on novel nucleic acid sequences encoding melanoma associated antigen molecules, however no solid data documented the expression of ERMP1 protein in tumor. As described below, an antibody generated towards the amino acid region 1 to 204 of the ERMP1 protein SEQ ID NO:74 showed a selective immunoreactivity in histological preparation of breast cancer tissues, which indicates the presence of this protein in this cancer type. In particular our immunoistochemistry analysis indicates that the protein shows plasma membrane localization in tumor cells.

Moreover, localization analysis of ovary tumor cell lines showed that this proteins is exposed on the cell surface and accessible to the binding of specific antibodies.

Therefore, the invention provides a method of screening a breast tissue sample for malignancy, which comprises determining the presence in said sample of the tumor marker protein ERMP1 SEQ ID NO:74 by means of an antibody against the amino acid region 1 to 204 of said ERMP1 protein. The marker protein can be detected by means of labeled monoclonal or polyclonal antibodies that specifically bind to the target protein. The methods for detecting proteins in a tissue sample are known to one skilled in the art and include immunoradiometric, immunoenzymatic or immunohistochemical techniques, such as radioimmunoassays, immunofluorescent assays or enzyme-linked immunoassays. Other known protein analysis techniques, such as polyacrylamide gel electrophoresis (PAGE), Western blot or Dot blot are suitable as well. Preferably, the detection of the protein marker is carried out with the immune-histochemistry technology, particularly by means of High Through-Put methods that allow the analyses of the antibody immune-reactivity simultaneously on different tissue samples immobilized on a microscope slide. Briefly, each Tissue Micro Array (TMA) slide includes tissue samples suspected of malignancy taken from different patients, and an equal number of normal tissue samples from the same patients as controls. The direct comparison of samples by qualitative or quantitative measurement, e.g. by enzimatic or colorimetric reactions, allows the identification of tumors.

A further aspect of the invention is a method *in vitro* for determining the presence of a breast tumor in a subject, which comprises the steps of:
- providing a sample of the tissue suspected of containing tumor cells;
- determining the presence of a tumor marker protein ERMP1 SEQ ID NO:74 in said tissue sample by detecting the expression of the marker protein by means of an antibody raised against the amino acid region 1 to 204 of said ERMP1 protein;
wherein the detection of the tumor marker in the tissue sample is indicative of the presence of tumor in said subject.

The methods and techniques for carrying out the assay are known to one skilled in the art and are preferably based on immunoreactions for detecting proteins

The term "antibody" as used herein refers to all types of immunoglobulins, including IgG, IgM, IgA, IgD and IgE. Such antibodies may include polyclonal, monoclonal, chimeric, single chain, antibodies or fragments such as Fab or scFv. The antibodies may be of various origin, including human, mouse, rat, rabbit and horse, or chimeric antibodies. The production of antibodies is well known in the art. For the production of antibodies in experimental animals, various hosts including goats, rabbits, rats, mice, and others, may be immunized by injection with polypeptides of the present invention or any fragment or oligopeptide or derivative thereof which has immunogenic properties or forms a suitable epitope. Monoclonal antibodies may be produced following the procedures described in Kohler and Milstein, Nature 265:495 (1975) or other techniques known in the art.

The antibodies to the tumor marker of the invention can be used to detect the presence of the marker in histologic preparations or to distinguish tumor cells from normal cells. To that purpose, the antibodies may be labeled with radioactive, fluorescent or enzyme labels.

The invention further discloses a diagnostic kit containing suitable means for detection, in particular the polypeptides or polynucleotides, antibodies or fragments or derivatives thereof described above, reagents, buffers, solutions and materials needed for setting up and carrying out the immunoassays. Parts of the kit of the invention can be packaged individually in vials or bottles or in combination in containers or multicontainer units.

### Description of the Figures

**Figure 1****. Analysis of purified ERMP1 recombinant protein**
   Left panel: Comassie staining of purified His-tag ERMP1 fusion protein separated by SDS-PAGE; Right panel: WB on the recombinant protein stained with anti- ERMP1 antibody. Arrow marks the protein band of the expected size. Molecular weight markers are reported on the left.
**Figure 2****. Staining of breast tumor TMA with anti-ERMP1 antibodies**
   Examples of TMA of tumor (lower panel) and normal tissue samples (upper panel) stained with anti-ERMP1 antibodies. The antibody stains specifically tumor cells (in dark gray).
**Figure 3****. Expression and localization of ERMP1 in tumor cell lines**
   **Panel A:** Western blot analysis of ERMP1 expression in total protein extracts separated by SDS-PAGE from HEK-293T cells (corresponding to 1x10⁶ cells) transfected with the empty pcDNA3 vector (lane 1) or with the plasmid construct encoding the ERMP1 gene (lane 2);
   **Panel B:** Western blot analysis of ERMP1 expression in total protein extracts separated by SDS-PAGE from MCF-7 (lane 1) and SKBR-3 (lane 2) tumor cells (corresponding to 2x10⁵ cells). Arrow marks the expected ERMP1 band. Molecular weight markers are reported on the left.
   **Panel C:** Flow cytometry analysis of ERMP1 cell surface localization in SKBR-3 tumor cells stained with a negative control antibody (filled curve or with anti-ERMP1 antibody (empty curve). X axis, Fluorescence scale; Y axis, Cells (expressed as % relatively to major peaks).

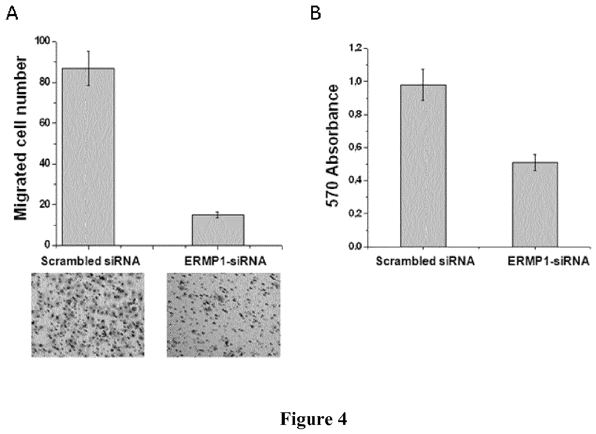

**Figure 4****. ERMP1 confers malignant cell phenotype** - The proliferation and the invasiveness properties of the MCF7 cell line were assessed after transfection with ERMP1-siRNA and a scramble siRNA control using the MTT and the Boyden in vitro invasion assays, respectively.
   **Panel A.** Cell migration/invasiveness measured by the Boyden migration assay. The graph represents the reduced migration/invasiveness of MCF7 treated with the ERMP1-specific siRNA. Small boxes under the columns show the visual counting of the migrated cells.
   **Panel B.** Cell proliferation determined by the MTT incorporation assay. The graph represents the reduced proliferation of the MCF7 tumor cells upon treatment with ERMP1-siRNA, as determined by spectrophotometric reading.

### EXAMPLES

### Example 1. Generation of recombinant human protein antigens and antibodies to identify tumor markers

### Methods

The entire coding region or suitable fragments of the genes encoding the target proteins, were designed for cloning and expression using bioinformatic tools with the human genome sequence as template (Lindskog M et al (2005). Where present, the leader sequence for secretion was replaced with the ATG codon to drive the expression of the recombinant proteins in the cytoplasm of E. coli. For cloning, genes were PCR-amplified from templates derived from Mammalian Gene Collection (http://mgc.nci.nih.gov/) clones using specific primers. Clonings were designed so as to fuse a 10 histidine tag sequence at the 5' end, annealed to in house developed vectors, derivatives of vector pSP73 (Promega) adapted for the T4 ligation independent cloning method (Nucleic Acids Res. 1990 October 25; 18(20): 6069-6074) and used to transform *E.coli* NovaBlue cells recipient strain. *E. coli* tranformants were plated onto selective LB plates containing 100 µg/ml ampicillin (LB Amp) and positive E.coli clones were identified by restriction enzyme analysis of purified plasmid followed by DNA sequence analysis. For expression, plasmids were used to transform BL21-(DE3) *E.coli* cells and BL21-(DE3) E. coli cells harbouring the plasmid were inoculated in ZYP-5052 growth medium (Studier, 2005) and grown at 37°C for 24 hours. Afterwards, bacteria were collected by centrifugation, lysed into B-Per Reagent containing 1 mM MgCl2, 100 units DNAse I (Sigma), and 1 mg/ml lysozime (Sigma). After 30 min at room temperature under gentle shaking, the lysate was clarified by centrifugation at 30.000 g for 40 min at 4°C. All proteins were purified from the inclusion bodies by resuspending the pellet coming from lysate centrifugation in 40 mM TRIS-HCl, 1 mM TCEP {Tris(2-carboxyethyl)-phosphine hydrochloride, Pierce} and 6M guanidine hydrochloride, pH 8 and performing an IMAC in denaturing conditions. Briefly, the resuspended material was clarified by centrifugation at 30.000 g for 30 min and the supernatant was loaded on 0.5 ml columns of Ni-activated Chelating Sepharose Fast Flow (Pharmacia). The column was washed with 50 mM TRIS-HCl buffer, 1 mM TCEP, 6M urea, 60 mM imidazole, 0.5M NaCl, pH 8. Recombinant proteins were eluted with the same buffer containing 500 mM imidazole. Proteins were analysed by SDS-Page and their concentration was determined by Bradford assay using the BIORAD reagent (BIORAD) with a bovine serum albumin standard according to the manufacturer's recommendations. The identity of recombinant affinity purified proteins was further confirmed by mass spectrometry (MALDI-TOF), using standard procedures.

To generate antisera, the purified proteins were used to immunize CD1 mice (6 week-old females, Charles River laboratories, 5 mice per group) intraperitoneally, with 3 protein doses of 20 micrograms each, at 2 week-interval. Freund's complete adjuvant was used for the first immunization, while Freund's incomplete adjuvant was used for the two booster doses. Two weeks after the last immunization animals were bled and sera collected from each animal was pooled.

### Results

Gene fragments of the expected size were obtained by PCR from specific clones of the Mammalian Gene Collection or, alternatively, from cDNA generated from pools of total RNA derived from Human testis, Human placenta, Human bone marrow, Human fetal brain, using primers specific for each gene.

For the ERMP1 gene, a fragment corresponding to nucleotides 55-666 of the transcript ENST00000339450 and encoding a protein of 204 residues, corresponding to the amino acid region from 1 to 204 of ENSP00000340427 sequence was obtained.

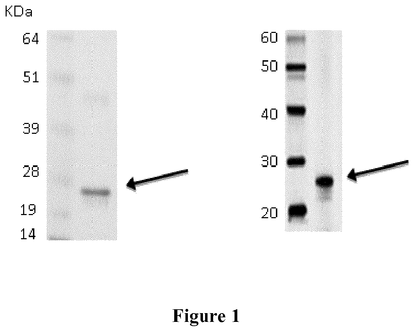

A clone encoding the correct amino acid sequence was identified for the gene/gene-fragment and, upon expression in *E. coli,* a protein of the correct size was produced and subsequently purified using affinity chromatography (Figure 1 left panels). As shown in the figures, in some case SDS-PAGE analysis of affinity-purified recombinant proteins revealed the presence of extra bands, of either higher and/or lower masses. Mass spectrometry analysis confirmed that they corresponded to either aggregates or degradation products of the protein under analysis.

Antibodies generated by immunization specifically recognized their target proteins in Western blot (WB) (Figure 1 right panel).

### Example 2. Tissue profiling by immune-histochemistry

### Methods

The analysis of the antibodies' capability to recognize their target proteins in tumor samples was carried out by Tissue Micro Array (TMA), a miniaturized immuno-histochemistry technology suitable for HTP analysis that allows to analyse the antibody immuno-reactivity simultaneously on different tissue samples immobilized on a microscope slide.

Since the TMAs include both tumor and healthy tissues, the specificity of the antibodies for the tumors can be immediately appreciated. The use of this technology, differently from approaches based on transcription profile, has the important advantage of giving a first hand evaluation on the potential of the markers in clinics. Conversely, since mRNA levels not always correlate with protein levels (approx. 50% correlation), studies based on transcription profile do not provide solid information regarding the expression of protein markers.

A tissue microarray was prepared containing formalin-fixed paraffin-embedded cores of human tissues from patients affected by breast cancer and corresponding normal tissues as controls and analyzed using the specific antibody sample. In total, the TMA design consisted in 10 tumor breast tumor samples and 10 normal tissues from 5 well pedigreed patients (equal to two tumor samples and 2 normal tissues from each patient) to identify promising target molecules differentially expressed in cancer and normal cells. The direct comparison between tumor and normal tissues of each patient allowed the identification of antibodies that stain specifically tumor cells and provided indication of target expression in breast tumor.

To further confirm the association of each protein with breast tumors a tissue microarray was prepared containing 100 formalin-fixed paraffin-embedded cores of human breast tissues from 50 patients (equal to two tissue samples from each patient).

All formalin fixed, paraffin embedded tissues used as donor blocks for TMA production were selected from the archives at the IEO (Istituto Europeo Oncologico, Milan). Corresponding whole tissue sections were examined to confirm diagnosis and tumour classification, and to select representative areas in donor blocks. Normal tissues were defined as microscopically normal (non- neoplastic) and were generally selected from specimens collected from the vicinity of surgically removed tumors. The TMA production was performed essentially as previously described (Kononen J et al (1998) Nature Med. 4:844-847; Kallioniemi OP et al (2001) Hum. Mol. Genet. 10:657-662). Briefly, a hole was made in the recipient TMA block. A cylindrical core tissue sample (1 mm in diameter) from the donor block was acquired and deposited in the recipient TMA block. This was repeated in an automated tissue arrayer "Galileo TMA CK 3500" (BioRep, Milan) until a complete TMA design was produced. TMA recipient blocks were baked at 42 <0>C for 2 h prior to sectioning. The TMA blocks were sectioned with 2-3 mm thicknes using a waterfall microtome (Leica), and placed onto poly-L-lysinated glass slides for immunohistochemical analysis. Automated immunohistochemistry was performed as previously described (Kampf C. et al (2004) Clin. Proteomics 1:285-300). In brief, the glass slides were incubated for 30' min in 60°C, de-paraffinized in xylene (2 x 15 min) using the Bio-Clear solution (Midway. Scientific, Melbourne, Australia), and re-hydrated in graded alcohols. For antigen retrieval, slides were immersed 0.01 M Na-citrate buffer, pH 6.0 at 99°C for 30 min Slides were placed in the Autostainer (R) (DakoCytomation) and endogenous peroxidase was initially blocked with 3% H2O2, for 5 min. Slides were then blocked in Dako Cytomation Wash Buffer containing 5% Bovine serum albumin (BSA) and subsequently incubated with mouse antibodies for 30' (dilution 1:200 in Dako Real ™ dilution buffer). After washing with DakoCytomation wash buffer, slides were incubated with the goat anti-mouse peroxidase conjugated Envision(R) for 30 min each at room temperature (DakoCytomation). Finally, diaminobenzidine (DakoCytomation) was used as chromogen and Harris hematoxylin (Sigma- Aldrich) was used for counterstaining. The slides were mounted with Pertex(R) (Histolab).

The staining results have been evaluated by a trained pathologist at the light microscope, and scored according to both the percentage of immunostained cells and the intensity of staining. The individual values and the combined score (from 0 to 300) were recorded in a custom-tailored database. Digital images of the immunocytochemical findings have been taken at a Leica DM LB light microscope, equipped with a Leica DFC289 color camera.

### Results

A TMA design was obtained, representing tumor tissue samples and normal tissues, derived from patients affected by breast tumor. The results from tissue profiling showed that the antibodies specific for the recombinant proteins (see Example 1) are strongly immunoreactive on breast tumor cancer tissues, while no or poor reactivity was detected in normal tissues, indicating the presence of the target proteins in breast tumors. Based on this finding, the detection of target proteins in tissue samples can be associated with breast tumor. In some cases immunoreactivity accumulated at the cell membrane of tumor cells providing a first-hand indication on the surface localization of the target proteins.

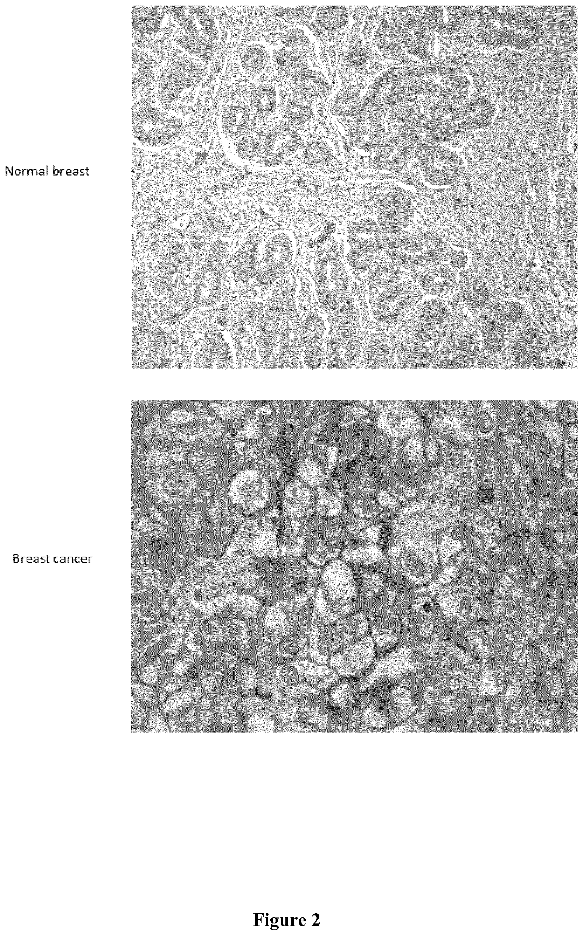

The capability of target-specific antibodies to stain breast tumor tissues is summarized in Table I. Representative examples of microscopic enlargements of tissue samples stained by each antibody are reported in Figure 2).

Table reports the percentage of positive breast tumor tissue samples after staining with the target specific antibodies.

| **Marker name** | **Percentage of Breast tumor tissues showing positive IHC staining** |
|---|---|
| ERMP1 | 45** |

| | |
|---|---|
| (**) The antibody stains the cell membrane of tumor cells | |

### Example 3. Expression and localization of target protein in transfected mammalian cells

### Methods

The specificity of the antibodies for each target proteins was assessed by Western blot analysis on total protein extracts from eukaryotic cells transiently transfected with plasmid constructs containing the complete sequences of the genes encoding the target proteins. Where indicated, expression and localization of target proteins were investigated by confocal microscopy analysis of transfected cells. Such experiments were carried out on ERMP1 (cloned sequence corresponding to Transcripts ENST00000339450).

For clonings, cDNA were generated from pools of total RNA derived from Human testis, Human placenta, Human bone marrow, Human fetal brain, in reverse transcription reactions and the entire coding regions were PCR-amplified with specific primers pairs. PCR products were cloned into plasmid pcDNA3 (Invitrogen). HeLa or Hek-293T cells were grown in DMEM-10% FCS supplemented with 1 mM Glutamine were transiently transfected with preparation of the resulting plasmids and with the empty vector as negative control using the Lipofectamine-2000 transfection reagent (Invitrogen). After 48 hours, cells were collected and analysed by Western blot or confocal microscopy. For Western blot, cells were lysed with PBS buffer containing 1% Triton X100 and total cell extracts (corresponding to 2x10⁵ cells) were separated on pre-cast SDS-PAGE gradient gels (NuPage 4-12% Bis-Tris gel, Invitrogen) under reducing conditions, followed by electro-transfer to nitrocellulose membranes (Invitrogen) according to the manufacturer's recommendations. The membranes were blocked in blocking buffer composed of 1xPBS-0.1% Tween 20 (PBST) added with 10% dry milk, for 1 h at room temperature, incubated with the antibody diluted 1:2500 in blocking buffer containing 1% dry milk and washed in PBST-1%. The secondary HRP-conjugated antibody (goat anti-mouse immunoglobulin/HRP, Perkin Elmer) was diluted 1:5000 in blocking buffer and chemiluminescence detection was carried out using a Chemidoc-IT UVP CCD camera (UVP) and the Western lightning™cheminulescence Reagent Plus (Perkin Elmer), according to the manufacturer's protocol.

For confocal microscopy analysis, the cells were plated on glass cover slips and after 48 h were washed with PBS and fixed with 3% *p*-formaldheyde solution in PBS for 20 min at RT. For surface staining, cells were incubated overnight at 4°C with polyclonal antibodies (1:200). The cells were then stained with Alexafluor 488-labeled goat anti-mouse antibodies (Molecular Probes). DAPI (Molecular Probes) was used to visualize nuclei; Live/Dead® red fixable (Molecular Probes) was used to visualize membrane. The cells were mounted with glycerol plastine and observed under a laser-scanning confocal microscope (LeicaSP5).

### Results

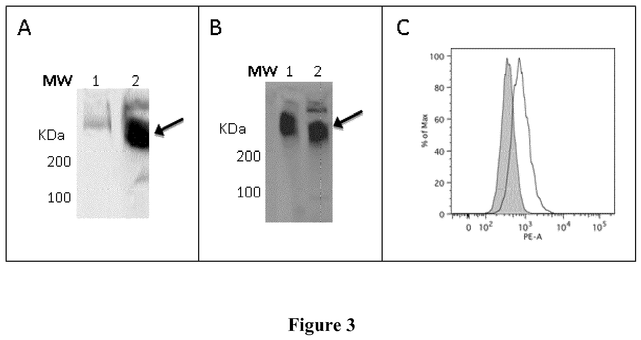

The selected coding sequence for ERMP1 was cloned in a eukaryotic expression vector and the derived plasmid used for transient transfection of HeLa or HEK293T cells. Expression of ERMP1 was analysed in trasnfected HEK-293T cells. Overall the data confirmed that the marker-specific antibodies recognized specifically their target proteins. For cells transfected with ERMP1-encoding plasmid, a band of high molecular mass was specifically detected by the anti-ERMP1 antibody indicating that the protein forms stable aggregates (Figure 3A).

### Example 4. Detection of target protein in tumor tissue homogenates

The presence of protein bands corresponding to the marker protein was also investigated in tissue homogenates of breast tumor biopsies as compared to normal tissues from patients. Homogenates were prepared by mechanic tissue disruption in buffer containing 40 mM TRIS-HCl, 1 mM TCEP {Tris(2-carboxyethyl)-phosphine hydrochloride, Pierce} and 6M guanidine hydrochloride, pH 8. Western blot was performed by separation of the total protein extracts (20 µg/lane) proteins were detected by specific antibodies.

### Example 5. Expression of target protein in tumor cell lines

Expression of target proteins was also assessed by WB and/or Flow cytometry on total extracts from breast tumor cell lines, including BT549, MCF7, MDA-MB231 and SKBR-3.

In each analysis, cells were cultured in under ATCC recommended conditions, and sub-confluent cell monolayers were detached with PBS-0.5 mM EDTA. For Western blot analysis, cells were lysed by several freeze-thaw passages in PBS-1% Triton. Total protein extracts were loaded on SDS-PAGE (2x10⁵ cells/lane), and subjected to WB with specific antibodies as described above.

For flow cytometry analysis, cells (2x10⁴ per well) were pelletted in 96 U-bottom microplates by centrifugation at 200 x g for 5 min at 4°C and incubated for 1 hour at 4°C with the appropriate dilutions of the marker-specific antibodies. Cells were washed twice in PBS-5% FCS and incubated for 20 min with the appropriate dilution of R-Phycoerythrin (PE)-conjugated secondary antibodies (Jackson Immuno Research, PA, USA) at 4°C. After washing, cells were analysed by a FACS Canto II flow cytometer (Becton Dickinson). Data were analyzed with FlowJo 8.3.3 program.

### Results

Western blot and flow cytometry analysis of ERMP1 are represented. Western blot analysis shows a band of high molecular mass detected in the breast cell lines MCF7 and SKBR-3, showing an electrophoretic pattern similar to that reported in transfected cells (see Example 3). This further confirms the existence of stable aggregates for this protein confirming its expression in cell lines derived from breast tumor (Figure 29B). Flow cytometry analysis indicates that ERMP1 is detected on the surface of the SKBR-3 cell line (Figure 3C).

### Example 6. Expression of the marker proteins confers malignant cell phenotype

To verify that the proteins included in the present invention can be exploited as targets for therapeutic applications, the effect of marker depletion was evaluated *in vitro* in cellular studies generally used to define the role of newly discovered proteins in tumor development. Marker-specific knockdown and control tumor cell lines were assayed for their proliferation and the migration/invasiveness phenotypes using the MTT and the Boyden in vitro invasion assay, respectively.

### Method

Expression of marker genes were silenced in tumor cell lines by the siRNA technology and the influence of the reduction of marker expression on cell parameters relevant for tumor development was assessed in *in vitro* assays. The expression of marker genes was knocked down in a panel of epithelial tumor cell lines previously shown to express the tumor markers using a panel of marker-specific siRNAs (whose target sequences are reported in the Table II) using the HiPerfect transfection reagent (QIAGEN) following the manufacturer's protocol. As control, cells treated with irrelevant siRNA (scrambled siRNA) were analysed in parallel. At different time points (ranging from 24 to 72 hours) post transfection, the reduction of gene transcription was assessed by quantitative RT-PCR (Q-RT-PCR) on total RNA, by evaluating the relative marker transcript level, using the beta-actin, GAPDH or MAPK genes as internal normalization control. Afterwards, cell proliferation and migration/invasiveness assays were carried out to assess the effect of the reduced marker expression. Cell proliferation was determined using the MTT assay, a colorimetric assay based on the cellular conversion of a tetrazolium salt into a purple colored formazan product. Absorbance of the colored solution can be quantified using a spectrophotometer to provide an estimate of the number of attached living cells. Approximately 5 x 10³ cells/100µl were seeded in 96-well plates in DMEM with 10% FCS to allow cell attachment. After overnight incubation with DMEM without FCS, the cells were treated with 2,5% FBS for 72 hours. Four hours before harvest 15 µL of the MTT dye solution (Promega) were added to each well. After 4-hour incubation at 37°C, the formazan precipitates were solubilized by the addition of 100 µL of solubilization solution (Promega) for 1h at 37°C,. Absorbance at 570 nm was determined on a multiwell plate reader (SpectraMax, Molecular Devices).

Cell migration/invasiveness was tested using the Boyden *in vitro* invasion assay, as compared to control cell lines treated with a scramble siRNA. This assay is based on a chamber of two medium-filled compartments separated by a microporous membrane. Cells are placed in the upper compartment and are allowed to migrate through the pores of the membrane into the lower compartment, in which chemotactic agents are present. After an appropriate incubation time, the membrane between the two compartments is fixed and stained, and the number of cells that have migrated to the lower side of the membrane is determined. For this assay, a transwell system, equipped with 8-µm pore polyvinylpirrolidone-free polycarbonate filters, was used. The upper sides of the porous polycarbonate filters were coated with 50 µg/cm² of reconstituted Matrigel basement membrane and placed into six-well culture dishes containing complete growth medium. Cells (1x10⁴ cells/well) were loaded into the upper compartment in serum-free growth medium. After 16 h of incubation at 37°C, non-invading cells were removed mechanically using cotton swabs, and the microporous membrane was stained with Diff-Quick solution. Chemotaxis was evaluated by counting the cells migrated to the lower surface of the polycarbonate filters (six randomly chosen fields, mean ± SD).

### Results

Examples of this analysis are reported for ERMP1 in the breast tumor cell line MCF7. Gene silencing experiments with marker-specific siRNA reduced the marker transcripts (approximately 30-40 fold reduction), as determined by Q-RT_PCR. T II reports the sequences targeted by the siRNA molecules. The reduction of the expression of either of the two genes significantly impairs the proliferation and the invasiveness phenotypes of the MCF7 breast tumor cell line (Figures 30 and 36). This indicates that the protein is involved in tumor development and are therefore likey targets for the development of anti-cancer therapies.

| **Table II** | |
|---|---|
| **NCBI gene** | **siRNA Target Sequence** |
| ERMP1 | CCCGTGGTTCATCTGATATAA |
| | AAGGACTTTGCTCGGCGTTTA |
| | TACGTGGATGTTTGTAACGTA |
| | CTCGTATTGGCTCAATCATAA |

### References

1) Anderson, L., and Seilhamer, J. (1997). A comparison of selected mRNA and protein abundances in human liver. Electrophoresis 18, 533 - 537;
2) Chen, G., Gharib, T. G., Wang, H., Huang, C. C., Kuick, R., Thomas, D. G., Shedden, K. A., Misek, D. E., Taylor, J. M., Giordano, T. J., Kardia, S. L., Iannettoni, M. D., Yee, J., Hogg, P. J., Orringer, M. B., Hanash, S. M., and Beer, D. G. (2003) Protein profiles associated with survival in lung adenocarcinoma. Proc. Natl. Acad. Sci. U. S. A 100, 13537 - 13542;
3) Ginestier, C., Charafe-Jauffret, E., Bertucci, F., Eisinger, F., Geneix, J., Bechlian, D., Conte, N., Adelaide, J., Toiron, Y., Nguyen, C., Viens, P., Mozziconacci, M. J., Houlgatte, R., Birnbaum, D., and Jacquemier, J. (2002) Distinct and complementary information provided by use of tissue and DNA microarrays in the study of breast tumor markers. Am. J. Pathol. 161, 1223 - 1233;
4) Gygi, S. P., Rochon, Y., Franza, B. R., and Aebersold, R. (1999) Correlation between protein and mRNA abundance in yeast. Mol. Cell. Biol. 19, 1720 - 1730; Nishizuka, S., Charboneau, L., Young, L., Major, S., Reinhold, W. C., Waltham, M., Kouros-Mehr, H., Bussey, K. J., Lee, J. K., Espina, V., Munson, P. J., Petricoin, E., III, Liotta, L. A., and Weinstein, J. N. (2003) Proteomic profiling of the NCI-60 cancer cell lines using new high-density reverse-phase lysate microarrays. Proc. Natl. Acad. Sci. U. S. A 100, 14229 - 14234;
5) Tyers, M., and Mann, M. (2003) From genomics to proteomics. Nature 422, 193 - 197;
6) Kagara N, Tanaka N, Noguchi S, Hirano T. (2007) Zinc and its transporter ZIP10 are involved in invasive behavior of breast cancer cells. Cancer Sci. 98:692 - 697;
7) Kasper G, Weiser AA, Rump A, Sparbier K, Dahl E, Hartmann A, Wild P, Schwidetzky U, Castaños-Vélez E, Lehmann K. (2005) Expression levels of the putative zinc transporter LIV-1 are associated with a better outcome of breast cancer patients. Int J Cancer. 117:961 - 973;
8) Nguyen ST, Hasegawa S, Tsuda H, Tomioka H, Ushijima M, Noda M, Omura K, Miki Y, (2007) Identification of a predictive gene expression signature of cervical lymph node metastasis in oral squamous cell carcinoma., Cancer Sci. 98:740 - 746;
9) Garcia-Rudaz,C., Luna,F., Tapia,V., Kerr,B., Colgin,L., Galimi,F., Dissen,G.A., Rawlings,N.D. and Ojeda,S.R. (2007) Fxna, a novel gene differentially expressed in the rat ovary at the time of folliculogenesis, is required for normal ovarian histogenesis. Development. 134, 945-957;
10) Kratzschmar J, Haendler B, Eberspaecher U, Roosterman D, Donner P, Schleuning WD. (1996) The human cysteine-rich secretory protein (CRISP) family. Primary structure and tissue distribution of CRISP-1, CRISP-2 and CRISP-3. Eur J Biochem. 236:827 - 836
11) Bjartell,A., Johansson,R., Bjork,T., adaleanu,V., Lundwall,A., Lilja,H., Kjeldsen,L. and Udby,L. (2006) Immunohistochemical detection of cysteine-rich secretory protein 3 in tissue and in serum from men with cancer or benign enlargement of the prostate gland, Prostate. 66: 591 - 603;
12) Bjartell,A.S., Al-Ahmadie,H., Serio,A.M., Eastham,J.A., Eggener,S.E., Fine,S.W., Udby,L., Gerald,W.L., Vickers,A.J., Lilja,H., Reuter,V.E. and Scardino,P.T. (2007), Association of cysteine-rich secretory protein 3 and beta-microseminoprotein with outcome after radical prostatectomy. Clin. Cancer Res. 13: 4130-4138.

## Claims

1. A method of screening a breast tissue sample for malignancy, said method comprising determining the presence in said sample of the tumor marker protein ERMP1 SEQ ID NO:74 by means of an antibody against the amino acid region 1 to 204 of said ERMP1 protein.

2. A method according to claim 1, which is based on immunoradiometric, immunoenzymatic or immunohistochemical techniques.

3. A method *in vitro* for determining the presence of a breast tumor in a subject, which comprises the steps of:
(a) providing a sample of the tissue suspected of containing tumor cells;
(b) determining the presence of the tumor marker protein ERMP1 SEQ ID NO:74 in said tissue sample by detecting the expression of the marker protein by means of an antibody raised against the amino acid region 1 to 204 of said ERMP1 protein;
wherein the detection of the tumor marker in the tissue sample is indicative of the presence of tumor in said subject.

## Patentansprüche

1. Verfahren zum Screening einer Brustgewebeprobe auf Malignität, wobei das Verfahren Bestimmen des Vorliegens des Tumormarkerproteins ERMP1, SEQ ID NO:74, mit Hilfe eines Antikörpers gegen die Aminosäureregion 1 bis 204 des ERMP1-Proteins in der Probe umfasst.

2. Verfahren gemäß Anspruch 1, das auf immunoradiometrischen, immunoenzymatischen oder immunohistochemischen Techniken basiert.

3. In-vitro-Verfahren zum Bestimmen des Vorliegens eines Brusttumors in einem Subjekt, das die folgenden Schritte umfasst:
(a) Bereitstellen einer Probe des Gewebes, von dem vermutet wird, dass es Tumorzellen enthält;
(b) Bestimmen des Vorliegens des Tumormarkerproteins ERMP1, SEQ ID NO:74, in der Gewebeprobe durch Detektieren der Expression des Markerproteins mittels eines Antikörpers, der gegen die Aminosäureregion 1 bis 204 des ERMP1-Proteins gerichtet ist;
wobei die Detektion des Tumormarkers in der Gewebeprobe für das Vorliegen des Tumors in dem Subjekt indikativ ist.

## Revendications

1. Procédé de criblage d'un échantillon de tissu de sein pour la malignité, ledit procédé comprenant de déterminer la présence dans ledit échantillon de la protéine marqueur de tumeur ERMP1 SEQ ID n° 74 au moyen d'un anticorps contre la région d'acides aminés 1 à 204 de la protéine ERMP1.

2. Procédé selon la revendication 1, qui est basé sur des techniques immunoradiométriques, immunoenzymatiques ou immunohistochimiques.

3. Procédé *in vitro* de détermination de la présence d'une tumeur du sein chez un sujet, qui comprend les étapes consistant à :
(a) fournir un échantillon du tissu suspecté de contenir des cellules tumorales ;
(b) déterminer la présence de la protéine marqueur de tumeur ERMP1 SEQ ID n° 74 dans ledit échantillon de tissu en détectant l'expression de la protéine marqueur au moyen d'un anticorps élevé contre la région d'acides aminés 1 à 204 de ladite protéine ERMP1 ;
où la détection du marqueur de tumeur dans l'échantillon de tissu est indicatrice de la présence d'une tumeur chez ledit sujet.


### SEQUENCE LISTING

<110> Externautics S.p.A.
<120> breast tumor markers and methos of use thereof
<130> 1298PCT
<160> 94
<170> Patent In version 3.3
<210> 1
   <211> 132
   <212> PRT
   <213> Homo sapiens
<400> 1
   Met Ser Gln Gly Arg His Leu Leu Glu Phe Leu Pro Leu Tyr Ile Ala
   1               5                   10                  15
   Phe Met Leu Arg Gly Val Cys Arg Ile Asp Ala Gly Ser Leu Asn Pro
               20                  25                  30
   Glu Leu Phe Leu Pro Met Leu His Glu Glu Asp Trp Cys Trp Glu Ile
           35                  40                  45
   Ala Gly His Val Asp Ser Gln Glu Leu Phe Val Gly Leu Phe Ser Ser
       50                  55                  60
   Thr Ser Thr Gly His Ala Glu Leu Asp Lys Lys Val Asn Gly Leu Tyr
   65                  70                  75                  80
   Tyr Asp Ser Val Phe Gln Leu Ser Leu Asp Arg Met Arg His Thr Arg
                   85                  90                  95
   Ser Met Ala Arg Val Glu Arg Leu Arg His Arg Lys Ala Ile Gln Lys
               100                 105                 110
   Lys Thr Gln Leu Val His His Leu Leu Phe Lys Gly Trp Ala Ser Asp
           115                 120                 125
   Glu Thr Glu Ile
       130
<210> 2
   <211> 399
   <212> DNA
   <213> Homo sapiens
<400> 2
   atgtcacaag gcaggcatct tcttgagttt cttccattgt acatagcttt catgttacgt      60
   ggggtttgta ggatagacgc tggaagcctt aatccagaac tgtttttgcc aatgttacat    120
   gaagaggatt ggtgttggga gatagctggc catgtggact cccaagagtt attcgttggt    180
   ttgttttcta gtacctctac tgggcatgca gagctggaca aaaaggttaa tggactttat    240
   tatgactctg tattccagtt gtctctggac cgtatgcgtc atacaaggag tatggctaga    300
   gtagagaggc tgagacacag gaaagcgatc cagaaaaaga ctcagttagt ccatcatctg    360
   ctatttaaag gatgggcttc tgatgaaact gaaatttag                           399
<210> 3
   <211> 147
   <212> PRT
   <213> Homo sapiens
<400> 3
   Met Gly Phe Ile Phe Ser Lys Ser Met Asn Glu Ser Met Lys Asn Gln
   1               5                   10                  15
   Lys Glu Phe Met Leu Met Asn Ala Arg Leu Gln Leu Glu Arg Gln Leu
               20                  25                  30
   Ile Met Gln Ser Glu Met Arg Glu Arg Gln Met Ala Met Gln Ile Ala
           35                  40                  45
   Trp Ser Arg Glu Phe Leu Lys Tyr Phe Gly Thr Phe Phe Gly Leu Ala
       50                  55                  60
   Ala Ile Ser Leu Thr Ala Gly Ala Ile Lys Lys Lys Lys Pro Ala Phe
   65                  70                  75                  80
   Leu Val Pro Ile Val Pro Leu Ser Phe Ile Leu Thr Tyr Gln Tyr Asp
                   85                  90                  95
   Leu Gly Tyr Gly Thr Leu Leu Glu Arg Met Lys Gly Glu Ala Glu Asp
               100                 105                 110
   Ile Leu Glu Thr Glu Lys Ser Lys Leu Gln Leu Pro Arg Gly Met Ile
           115                 120                 125
   Thr Phe Glu Ser Ile Glu Lys Ala Arg Lys Glu Gln Ser Arg Phe Phe
       130                 135                 140
   Ile Asp Lys
   145
<210> 4
   <211> 932
   <212> DNA
   <213> Homo sapiens
<400> 4
   gagcgaggcc cggtccctgc agcgggcgaa aggagcccgg gcctggaggt ttgcgtaccg      60
   gtcgcctggt cccggcacca gcgccgccca gtgtggtttc ccataaggaa gctcttcttc     120
   ctgcttggct tccaccttta acccttccac ctgggagcgt cctctaacac attcagacta     180
   caagtccaga cccaggagag caaggcccag aaagaggtca aaatggggtt tatattttca     240
   aaatctatga atgaaagcat gaaaaatcaa aaggagttca tgcttatgaa tgctcgactt     300
   cagctggaaa ggcagctcat catgcagagt gaaatgaggg aaagacaaat ggccatgcag     360
   attgcgtggt ctcgggaatt cctcaaatat tttggaactt tttttggcct tgcagccatc     420
   tctttaacag ctggagcgat taaaaaaaag aagccagcct tcctggtccc gattgttcca     480
   ttaagcttta tcctcaccta ccagtatgac ttgggctatg gaaccctttt agaaagaatg    540
   aaaggtgaag ctgaggacat actggaaaca gaaaagagta aattgcagct gccaagagga     600
   atgatcactt ttgaaagcat tgaaaaagcc agaaaggaac agagtagatt cttcatagac     660
   aaatgaaatc atgcttacca atcaaatctc aaagcacaga attattgact tgaatcatgg     720
   tttttacagt tttttaaatg ctcaagattt tgatattata gattttattt taaaatatta     780
   aaatgcaaga tagttttgag ctattttaaa ataaaattta taacattcaa cacaaaatca     840
   tggaggtgct ctaaataact tttagatttc ctctctctgt gtgcattacc aatatctaag     900
   tgtaaaatta ataaattgtt ttgaattcct gg                                   932
<210> 5
   <211> 131
   <212> PRT
   <213> Homo sapiens
<400> 5
   Met Phe Leu Gly Leu Val Gly Leu Arg Thr Lys Gly Arg Arg Trp Ile
   1               5                   10                  15
   Ser Ser Trp Ser Glu Gly Glu Asp Arg Gly Gln Ser Pro Glu Gly Val
               20                  25                  30
   Leu Leu Thr Trp Val Phe Gly Thr Lys Cys Val Met His Pro Cys Glu
           35                  40                  45
   Glu Thr Thr Lys Gln Ala Leu Cys Glu Gln Gln Gly Cys Leu Phe His
       50                  55                  60
   Leu Gly Ala Asp Glu Leu Ser Pro Lys Arg Glu Ser Ala Gln Ser Ile
   65                  70                  75                  80
   Ser Phe Lys Trp Glu Asn Ser Ile Tyr Leu His Ala Thr Leu Phe Leu
                   85                  90                  95
   Ile Gly Glu Tyr Leu His Leu Ala Phe Tyr Tyr Phe Leu Leu Val Leu
               100                 105                 110
   Tyr Ile Leu Cys Ser Phe Leu Ser Tyr Cys Leu Leu Leu Trp Leu Gly
           115                 120                 125
   Ser Phe Leu
       130
<210> 6
   <211> 2950
   <212> DNA
   <213> Homo sapiens
<400> 6
   attatctagg tctcggagga tggagaaatc aaaagtgcca ttttctggcc atttagaacc      60
   attgtcgagt ttgtattggg gccaagcagt gttgcagaag aaaataagac atttagattt     120
   tagttcaggt gatagttgaa gaaattttaa gttcttgaga acacaggcta agggagaaga     180
   aggaggaatg gagggtggaa gtttgcccat agtgaaggag gcaagtttaa agagaaaggt     240
   agagacatgg agaaagggtt ggggagcagc cctgggctgc aatgtgggtg agcagccaaa     300
   gcaggcatcc ccgcaattga cttgccacca agggaatgtg gttgaatgac caaggcaggc     360
   atccctgaag atatcagacg ccaatggaat gtgggtgaat aatcaggcag gcatccccgg     420
   aatgattaaa cactaaggga aggctgcctt cctgagtaca tgaccagcac cagagttttg     480
   ggtccatgga taaaatgtgt ctcctttgtc tctactagaa aatgaaagga attgaaatta     540
   agagaagaga gggagtgaag ggtggcacca agaatgaaag gagaaagagg ttgagggata     600
   gtgagaaagg ttggagaaga gagtaaaaag aggccactta cccgatttaa aatttgtgag     660
   atgttccttg ggctggttgg tctgaggacc aaaggtcgta ggtggatctc ttcatggagt     720
   gagggtgagg acaggggaca gtctcctgaa ggagtcctgc tgacctgggt ctttggcacc     780
   aaatgtgtca tgcatccatg tgaagagacc accaaacagg ctttgtgtga gcaacagggc     840
   tgtttgtttc acctgggtgc agacgagttg agtccaaaaa gagagtcagc ccagtctata     900
   tcttttaaat gggaaaattc aatctattta catgcaacgt tattcttgat aggtgagtac     960
   ttacatctag cattctatta ttttctgctt gttttgtata tcctttgttc cttcctctct    1020
   tactgtttac ttttgtggtt gggcagtttt ctgtagggat aagatttgag tcttatctct    1080
   ttctccctat gtgttagctt taccagtgag tttttatagt ttcacatatt tttatgatgc    1140
   tggttatcat cttctctgtg gggaacaggc cccccaaaac ctggccataa actggcccca 1200
   aaactggcca taaacaaaat ctctgcagca ctgtgacatg tacatgatgg tcttaacgcc 1260
   cacgctggaa ggttgtgggt ttaccagaat gagggcaagg aacacctggc ccacccaggg 1320
   tggaaaaccg cttaaaggca ttcttaaacc acaaacaata gcatgagtga tctgtgcctt 1380
   aaggccatgc tcctgctgca gatagctagt ccaacccatc cctttatttc agcccatctc 1440
   ttcatttccc ataaggaata attttagtta atctaatatc tatagaaaga atgctaatga 1500
   ctagcttgct gttaataaat acatgggtaa acctctgttg gaggctctca gctctgaagg 1560
   ctgtgagacc cttgatttcc tacttcactc ctctatattt ctgtgtcttt aattcctcta 1620
   gtgccactgg gttagagtct ccccgaccaa gctggtctca gcaagtggtc tccatcatgg 1680
   gggctcgaat ccaggttgaa gggtcaccag agtgatggtt ggagaacatg gaactagctg 1740
   gaggacacct gagtactctt aaagcaaacc ccgtggtgag taagaagggg agctcagaag 1800
   catcagggta acaatgggac aagtgtgggg tctggttcgt tccatcttgg aactttttca 1860
   cactgatgat gaggaagaag gagagtataa tgaagtaaca gaagaggtta tagagcaggt 1920
   ttatttgcca gctaaagcta aagtggcaaa ggagggagag gttcatccct acccttctgc 1980
   accccctcat tattattttg aagaaaaaga gtggcctgac cctccagatc tttcttttcc 2040
   agaggacagt gggcaaaaat tagttgcccc agtgactgtt caagcagcac ctcgagcgac 2100
   tgctcttagt tctattcagt caggaattca gcaagctaga tgagaaggtg attaagaggc 2160
   ttggcagttc cctgttagac tacactgccc agaccaacag ggaaatattg tagctacatt 2220
   tgagcctttt tgttttaaat tactcaaaga atttaaacaa gctattaatc agtatggacc 2280
   aggttctcct tttgtaatgg gactattaaa gaacattgct gtttccagtc agatgattcc 2340
   tactgactgg gacgctctta ctcaagcttg tctaactcct gcttagttct tacaatttaa 2400
   aacttggtgg gcagatgaag cttccattca ggcttctcac aacacgcagg accaacctca 2460
   aattaatata actgcagacc aacttttggg ggttggcagt tgggctggtt tagatgcaca 2520
   aatggtcatg caggatgatg ccatagaaca gcttagagga gcgtgcatta gagcttgggg 2580
   aaaaaaaatc acttcaagtg gagaacaata ccctttcttt agtgctataa aacagggacc 2640
   agaagaatca tatgtggatt ttatagctca gttacaggag tctcttaaaa agatgactgc 2700
   agatttggct gctcaggata tagtgttgca attattagct ttcaacaatg ctaatcctga 2760
   ttgccaggct gctctgtgac ctatcagagg gaaagcacat ttagttgatt atatcaaggc 2820
   ctgtggtggt atcagaggta atctgcatca ggccacctgc tagcacgggc aatggcagga 2880
   ctgagagtgg atacagaaag tactccattt cctggagctt gttttaactg tgggaagcat 2940
   ggtcatactg 2950
<210> 7
   <211> 316
   <212> PRT
   <213> Homo sapiens
<400> 7
   Met Ala Ser Ala Asp Glu Leu Thr Phe His Glu Phe Glu Glu Ala Thr
   1               5                   10                  15
   Asn Leu Leu Ala Asp Thr Pro Asp Ala Ala Thr Thr Ser Arg Ser Asp
               20                  25                  30
   Gln Leu Thr Pro Gln Gly His Val Ala Val Ala Val Gly Ser Gly Gly
           35                  40                  45
   Ser Tyr Gly Ala Glu Asp Glu Val Glu Glu Glu Ser Asp Lys Ala Ala
       50                  55                  60
   Leu Leu Gln Glu Gln Gln Gln Gln Gln Gln Pro Gly Phe Trp Thr Phe
   65                  70                  75                  80
   Ser Tyr Tyr Gln Ser Phe Phe Asp Val Asp Thr Ser Gln Val Leu Asp
                   85                  90                  95
   Arg Ile Lys Gly Ser Leu Leu Pro Arg Pro Gly His Asn Phe Val Arg
               100                 105                 110
   His His Leu Arg Asn Arg Pro Asp Leu Tyr Gly Pro Phe Trp Ile Cys
           115                 120                 125
   Ala Thr Leu Ala Phe Val Leu Ala Val Thr Gly Asn Leu Thr Leu Val
       130                 135                 140
   Leu Ala Gln Arg Arg Asp Pro Ser Ile His Tyr Ser Pro Gln Phe His
   145                 150                 155                 160
   Lys Val Thr Val Ala Gly Ile Ser Ile Tyr Cys Tyr Ala Trp Leu Val
                   165                 170                 175
   Pro Leu Ala Leu Trp Gly Phe Leu Arg Trp Arg Lys Gly Val Gln Glu
               180                 185                 190
   Arg Met Gly Pro Tyr Thr Phe Leu Glu Thr Val Cys Ile Tyr Gly Tyr
           195                 200                 205
   Ser Leu Phe Val Phe Ile Pro Met Val Val Leu Trp Leu Ile Pro Val
       210                 215                 220
   Pro Trp Leu Gln Trp Leu Phe Gly Ala Leu Ala Leu Gly Leu Ser Ala
   225                 230                 235                 240
   Ala Gly Leu Val Phe Thr Leu Trp Pro Val Val Arg Glu Asp Thr Arg
                   245                 250                 255
   Leu Val Ala Thr Val Leu Leu Ser Val Val Val Leu Leu His Ala Leu
               260                 265                 270
   Leu Ala Met Gly Cys Lys Leu Tyr Phe Phe Gln Ser Leu Pro Pro Glu
           275                 280                 285
   Asn Val Ala Pro Pro Pro Gln Ile Thr Ser Leu Pro Ser Asn Ile Ala
       290                 295                 300
   Leu Ser Pro Thr Leu Pro Gln Ser Leu Ala Pro Ser
   305                 310                 315
<210> 8
   <211> 316
   <212> PRT
   <213> Homo sapiens
<400> 8
   Met Ala Ser Ala Asp Glu Leu Thr Phe His Glu Phe Glu Glu Ala Thr
   1               5                   10                  15
   Asn Leu Leu Ala Asp Thr Pro Asp Ala Ala Thr Thr Ser Arg Ser Asp
               20                  25                  30
   Gln Leu Thr Pro Gln Gly His Val Ala Val Ala Val Gly Ser Gly Gly
           35                  40                  45
   Ser Tyr Gly Ala Glu Asp Glu Val Glu Glu Glu Ser Asp Lys Ala Ala
       50                  55                  60
   Leu Leu Gln Glu Gln Gln Gln Gln Gln Gln Pro Gly Phe Trp Thr Phe
   65                  70                  75                  80
   Ser Tyr Tyr Gln Ser Phe Phe Asp Val Asp Thr Ser Gln Val Leu Asp
                   85                  90                  95
   Arg Ile Lys Gly Ser Leu Leu Pro Arg Pro Gly His Asn Phe Val Arg
               100                 105                 110
   His His Leu Arg Asn Arg Pro Asp Leu Tyr Gly Pro Phe Trp Ile Cys
           115                 120                 125
   Ala Thr Leu Ala Phe Val Leu Ala Val Thr Gly Asn Leu Thr Leu Val
       130                 135                 140
   Leu Ala Gln Arg Arg Asp Pro Ser Ile His Tyr Ser Pro Gln Phe His
   145                 150                 155                 160
   Lys Val Thr Val Ala Gly Ile Ser Ile Tyr Cys Tyr Ala Trp Leu Val
                   165                 170                 175
   Pro Leu Ala Leu Trp Gly Phe Leu Arg Trp Arg Lys Gly Val Gln Glu
               180                 185                 190
   Arg Met Gly Pro Tyr Thr Phe Leu Glu Thr Val Cys Ile Tyr Gly Tyr
           195                 200                 205
   Ser Leu Phe Val Phe Ile Pro Met Val Val Leu Trp Leu Ile Pro Val
       210                 215                 220
   Pro Trp Leu Gln Trp Leu Phe Gly Ala Leu Ala Leu Gly Leu Ser Ala
   225                 230                 235                 240
   Ala Gly Leu Val Phe Thr Leu Trp Pro Val Val Arg Glu Asp Thr Arg
                   245                 250                 255
   Leu Val Ala Thr Val Leu Leu Ser Val Val Val Leu Leu His Ala Leu
               260                 265                 270
   Leu Ala Met Gly Cys Lys Leu Tyr Phe Phe Gln Ser Leu Pro Pro Glu
           275                 280                 285
   Asn Val Ala Pro Pro Pro Gln Ile Thr Ser Leu Pro Ser Asn Ile Ala
       290                 295                 300
   Leu Ser Pro Thr Leu Pro Gln Ser Leu Ala Pro Ser
   305                 310                 315
<210> 9
   <211> 1210
   <212> DNA
   <213> Homo sapiens
<400> 9
   aggtgagggg gggcggagcg cacctgtggg gacgggacga cgagttcaag cctccgtggg      60
   tgcagttggt cgccagcgag ggatgcggag acgcccctga acgaccatgg catcggccga     120
   cgagctgacc ttccatgaat tcgaggaggc cactaatctt ctggctgaca ccccagatgc     180
   agccaccacc agcagaagcg atcagctgac cccacaaggg cacgtggctg tggccgtggg     240
   ctcaggtggc agctatggag ccgaggatga ggtggaggag gagagtgaca aggccgcgct     300
   cctgcaggag cagcagcagc agcagcagcc gggattctgg accttcagct actatcagag     360
   cttctttgac gtggacacct cacaggtcct ggaccggatc aaaggctcac tgctgccccg     420
   gcctggccac aactttgtgc ggcaccatct gcggaatcgg ccggatctgt atggcccctt     480
   ctggatctgt gccacgttgg cctttgtcct ggccgtcact ggcaacctga cgctggtgct     540
   ggcccagagg agggacccct ccatccacta cagcccccag ttccacaagg tgaccgtggc     600
   aggcatcagc atctactgct atgcgtggct ggtgcccctg gccctgtggg gcttcctgcg     660
   gtggcgcaag ggtgtccagg agcgcatggg gccctacacc ttcctggaga ctgtgtgcat     720
   ctacggctac tccctctttg tcttcatccc catggtggtc ctgtggctca tccctgtgcc     780
   ttggctgcag tggctctttg gggcgctggc cctgggcctg tcagccgccg ggctggtatt     840
   caccctctgg cccgtggtcc gtgaggacac caggctggtg gccacagtgc tgctgtccgt     900
   ggtcgtgctg ctccacgccc tcctggccat gggctgtaag ttgtacttct tccagtcgct     960
   gcctccggag aacgtggctc ctccacccca aatcacatct ctgccctcaa acatcgcgct    1020
   gtcccctacc ttgccgcagt ccctggcccc ctcctaggaa ggcccgggtc ccacaggcaa    1080
   cacctaagtg gaccaacccc tctgcctgtc ctgcccccca gacgatgact gaaggctcct    1140
   ttgacacctt gagatgattc tgctactttc cagacttttc ttacaaagca aacactttta    1200
   ttttctatgc                                                            1210
<210> 10
   <211> 1502
   <212> DNA
   <213> Homo sapiens
<400> 10
   ggatgttgct gtcaggtctg agtcggttgg agtctgacgg gtaggcgaga cgcgcaggcg      60
   cagagagccc cagccacgcc ggcccaggtg gcctcagcga gggatgcgga gacgcccctg     120
   aacgaccatg gcatcggccg acgagctgac cttccatgaa ttcgaggagg ccactaatct     180
   tctggctgac accccagatg cagccaccac cagcagaagc gatcagctga ccccacaagg     240
   gcacgtggct gtggccgtgg gctcaggtgg cagctatgga gccgaggatg aggtggagga     300
   ggagagtgac aaggccgcgc tcctgcagga gcagcagcag cagcagcagc cgggattctg     360
   gaccttcagc tactatcaga gcttctttga cgtggacacc tcacaggtcc tggaccggat     420
   caaaggctca ctgctgcccc ggcctggcca caactttgtg cggcaccatc tgcggaatcg     480
   gccggatctg tatggcccct tctggatctg tgccacgttg gcctttgtcc tggccgtcac     540
   tggcaacctg acgctggtgc tggcccagag gagggacccc tccatccact acagccccca     600
   gttccacaag gtgaccgtgg caggcatcag catctactgc tatgcgtggc tggtgcccct     660
   ggccctgtgg ggcttcctgc ggtggcgcaa gggtgtccag gagcgcatgg ggccctacac     720
   cttcctggag actgtgtgca tctacggcta ctccctcttt gtcttcatcc ccatggtggt     780
   cctgtggctc atccctgtgc cttggctgca gtggctcttt ggggcgctgg ccctgggcct     840
   gtcagccgcc gggctggtat tcaccctctg gcccgtggtc cgtgaggaca ccaggctggt     900
   ggccacagtg ctgctgtccg tggtcgtgct gctccacgcc ctcctggcca tgggctgtaa     960
   gttgtacttc ttccagtcgc tgcctccgga gaacgtggct cctccacccc aaatcacatc    1020
   tctgccctca aacatcgcgc tgtcccctac cttgccgcag tccctggccc cctcctagga    1080
   aggcccgggt cccacaggca acacctaagt ggaccaaccc ctctgcctgt cctgcccccc    1140
   agacgatgac tgaaggctcc tttgacacct tgagatgatt ctgctacttt ccagactttt    1200
   cttacaaagc aaacactttt attttctatg caaaggtgat tcagagaatt tatataaagg    1260
   cgggcgaggg gcagccgagc agggagcttt gggacagggc tggggccccc atatcccccc    1320
   cgggccacct gctttccctc ctatggctcc cctggaacag gagggagagc caagggggcg     1380
   gcccagcctg gacagcgccc gctcctgcct gggtgcacac acggcgggcc tgagctccag    1440
   catctgagtt tgggggtatg agaaacaggg gagcagaagg agaagaaaac tgcctgtgct    1500
   gc                                                                   1502
<210> 11
   <211> 157
   <212> PRT
   <213> Homo sapiens
<400> 11
   Met Leu Pro Glu Gln Gly Pro Gln Pro Ser Thr Met Pro Leu Trp Cys
   1               5                   10                  15
   Leu Leu Ala Ala Cys Thr Ser Leu Pro Arg Gln Ala Ala Thr Met Leu
               20                  25                  30
   Glu Glu Ala Ala Ser Pro Asn Glu Ala Val His Ala Ser Thr Ser Gly
           35                  40                  45
   Ser Gly Ala Leu Thr Asp Gln Thr Phe Thr Asp Leu Ser Ala Ala Glu
       50                  55                  60
   Ala Ser Ser Glu Glu Val Pro Asp Phe Met Glu Val Pro His Ser Val
   65                  70                  75                  80
   His His Lys Ile Asn Cys Phe Phe Tyr Leu Glu Lys Gln Leu Cys Gln
                   85                  90                  95
   Leu Pro Ser Pro Leu Cys Leu Ser Ser Leu Leu Thr Leu Lys Leu Lys
               100                 105                 110
   Thr Thr Val Pro Ala Pro Gly Arg Trp Trp Ser Phe Gln Pro His Lys
           115                 120                 125
   Ala Phe Pro Leu Leu Val Gly Thr Pro Gly Ser Trp Gln Ser Thr Ile
       130                 135                 140
   Asp Pro Ala Trp Ala Ala Pro Ser Gln Pro Ser Pro Gly
   145                 150                 155
<210> 12
   <211> 474
   <212> DNA
   <213> Homo sapiens
<400> 12
   atgcttccag agcaagggcc ccagccttcc acgatgccgc tctggtgcct cctcgccgcc      60
   tgcaccagcc tcccaaggca ggcagccacc atgctggagg aagctgcttc tcccaacgag     120
   gctgtccacg catcaacatc aggcagtggc gcactcactg atcagacatt tacagacctc     180
   tcagctgccg aggcctcctc agaggaggtt cctgacttca tggaggtgcc acactctgtt     240
   caccataaaa ttaactgctt tttctactta gaaaaacaac tctgccaact gccgtcccca     300
   ctgtgtctaa gcagcttgct tactttaaaa ttaaaaacaa cggtcccagc tcctggcagg     360
   tggtggagct tccagcctca caaggcattc ccacttctgg tgggcactcc tggaagctgg     420
   cagagcacaa tcgatcccgc gtgggcggcc ccctctcagc caagcccagg gtga           474
<210> 13
   <211> 241
   <212> PRT
   <213> Homo sapiens
<400> 13
   Met Lys Phe Ile Leu Leu Trp Ala Leu Leu Asn Leu Thr Val Ala Leu
   1               5                   10                  15
   Ala Phe Asn Pro Asp Tyr Thr Val Ser Ser Thr Pro Pro Tyr Leu Val
               20                  25                  30
   Tyr Leu Lys Ser Asp Tyr Leu Pro Cys Ala Gly Val Leu Ile His Pro
           35                  40                  45
   Leu Trp Val Ile Thr Ala Ala His Cys Asn Leu Pro Lys Leu Arg Val
       50                  55                  60
   Ile Leu Gly Val Thr Ile Pro Ala Asp Ser Asn Glu Lys His Leu Gln
   65                  70                  75                  80
   Val Ile Gly Tyr Glu Lys Met Ile His His Pro His Phe Ser Val Thr
                   85                  90                  95
   Ser Ile Asp His Asp Ile Met Leu Ile Lys Leu Lys Thr Glu Ala Glu
               100                 105                 110
   Leu Asn Asp Tyr Val Lys Leu Ala Asn Leu Pro Tyr Gln Thr Ile Ser
           115                 120                 125
   Glu Asn Thr Met Cys Ser Val Ser Thr Trp Ser Tyr Asn Val Cys Asp
       130                 135                 140
   Ile Tyr Lys Glu Pro Asp Ser Leu Gln Thr Val Asn Ile Ser Val Ile
   145                 150                 155                 160
   Ser Lys Pro Gln Cys Arg Asp Ala Tyr Lys Thr Tyr Asn Ile Thr Glu
                   165                 170                 175
   Asn Met Leu Cys Val Gly Ile Val Pro Gly Arg Arg Gln Pro Cys Lys
               180                 185                 190
   Glu Val Ser Ala Ala Pro Ala Ile Cys Asn Gly Met Leu Gln Gly Ile
           195                 200                 205
   Leu Ser Phe Ala Asp Gly Cys Val Leu Arg Ala Asp Val Gly Ile Tyr
       210                 215                 220
   Ala Lys Ile Phe Tyr Tyr Ile Pro Trp Ile Glu Asn Val Ile Gln Asn
   225                 230                 235                 240
   Asn
<210> 14
   <211> 912
   <212> DNA
   <213> Homo sapiens
<400> 14
   aaggctggca aaaaggagac cagacaggag gcgtctgtag agatatcatg aacttcaact      60
   tagctttgtt ttccagagac tggagctaaa ctgggctttc aacatcatca tgaagtttat     120
   cctcctctgg gccctcttga atctgactgt tgctttggcc tttaatccag attacacagt     180
   cagctccact cccccttact tggtctattt gaaatctgac tacttgccct gcgctggagt     240
   cctgatccac ccgctttggg tgatcacagc tgcacactgc aatttaccaa agcttcgggt     300
   gatattgggg gttacaatcc cagcagactc taatgaaaag catctgcaag tgattggcta     360
   tgagaagatg attcatcatc cacacttctc agtcacttct attgatcatg acatcatgct     420
   aatcaagctg aaaacagagg ctgaactcaa tgactatgtg aaattagcca acctgcccta     480
   ccaaactatc tctgaaaata ccatgtgctc tgtctctacc tggagctaca atgtgtgtga     540
   tatctacaaa gagcccgatt cactgcaaac tgtgaacatc tctgtaatct ccaagcctca     600
   gtgtcgcgat gcctataaaa cctacaacat cacggaaaat atgctgtgtg tgggcattgt     660
   gccaggaagg aggcagccct gcaaggaagt ttctgctgcc ccggcaatct gcaatgggat     720
   gcttcaagga atcctgtctt ttgcggatgg atgtgttttg agagccgatg ttggcatcta     780
   tgccaaaatt ttttactata taccctggat tgaaaatgta atccaaaata actgagctgt     840
   ggcagttgtg gaccatatga cacagcttgt ccccatcgtt cacctttaga attaaatata     900
   aattaactcc tc                                                         912
<210> 15
   <211> 279
   <212> PRT
   <213> Homo sapiens
<400> 15
   Met Met Leu Met Gln Ala Leu Val Leu Phe Thr Leu Asp Ser Leu Asp
   1               5                   10                  15
   Met Leu Pro Ala Val Lys Ala Thr Trp Leu Tyr Gly Ile Gln Ile Thr
               20                  25                  30
   Ser Leu Leu Leu Val Cys Ile Leu Gln Phe Phe Asn Ser Met Ile Leu
           35                  40                  45
   Gly Ser Leu Leu Ile Ser Phe Asn Leu Ser Val Phe Ile Ala Arg Lys
       50                  55                  60
   Leu Gln Lys Asn Leu Lys Thr Gly Ser Phe Leu Asn Arg Leu Gly Lys
   65                  70                  75                  80
   Leu Leu Leu His Leu Phe Met Val Leu Cys Leu Thr Leu Phe Leu Asn
                   85                  90                  95
   Asn Ile Ile Lys Lys Ile Leu Asn Leu Lys Ser Asp Glu His Ile Phe
               100                 105                 110
   Lys Phe Leu Lys Ala Lys Phe Gly Leu Gly Ala Thr Arg Asp Phe Asp
           115                 120                 125
   Ala Asn Leu Tyr Leu Cys Glu Glu Ala Phe Gly Leu Leu Pro Phe Asn
       130                 135                 140
   Thr Phe Gly Arg Leu Ser Asp Thr Leu Leu Phe Tyr Ala Tyr Ile Phe
   145                 150                 155                 160
   Val Leu Ser Ile Thr Val Ile Val Ala Phe Val Val Ala Phe His Asn
                   165                 170                 175
   Leu Ser Asp Ser Thr Asn Gln Gln Ser Val Gly Lys Met Glu Lys Gly
               180                 185                 190
   Thr Val Asp Leu Lys Pro Glu Thr Ala Tyr Asn Leu Ile His Thr Ile
           195                 200                 205
   Leu Phe Gly Phe Leu Ala Leu Ser Thr Met Arg Met Lys Tyr Leu Trp
       210                 215                 220
   Thr Ser His Met Cys Val Phe Ala Ser Phe Gly Leu Cys Ser Pro Glu
   225                 230                 235                 240
   Ile Trp Glu Leu Leu Leu Lys Ser Val His Leu Tyr Asn Pro Lys Arg
                   245                 250                 255
   Ile Cys Ile Met Arg Tyr Ser Val Pro Ile Leu Ile Leu Leu Tyr Leu
               260                 265                 270
   Cys Tyr Lys Asn Gln Lys Ser
           275
<210> 16
   <211> 102
   <212> PRT
   <213> Homo sapiens
<400> 16
   Met Met Ser Ile Arg Gln Arg Arg Glu Ile Arg Ala Thr Glu Val Ser
   1               5                   10                  15
   Glu Asp Phe Pro Ala Gln Glu Glu Asn Val Lys Leu Glu Asn Lys Leu
               20                  25                  30
   Pro Ser Gly Cys Thr Ser Arg Arg Leu Trp Lys Ile Leu Ser Leu Thr
           35                  40                  45
   Ile Gly Gly Thr Ile Ala Leu Cys Ile Gly Leu Leu Thr Ser Val Tyr
       50                  55                  60
   Leu Ala Thr Leu His Glu Asn Asp Leu Trp Phe Ser Asn Ile Lys Val
   65                  70                  75                  80
   Trp Ser Phe Phe Asp His Cys Ile Ile His Ser Val Gly Ser Pro Val
                   85                  90                  95
   Val Ser His Val Asp Glu
               100
<210> 17
   <211> 716
   <212> PRT
   <213> Homo sapiens
<400> 17
   Met Met Ser Ile Arg Gln Arg Arg Glu Ile Arg Ala Thr Glu Val Ser
   1               5                   10                  15
   Glu Asp Phe Pro Ala Gln Glu Glu Asn Val Lys Leu Glu Asn Lys Leu
               20                  25                  30
   Pro Ser Gly Cys Thr Ser Arg Arg Leu Trp Lys Ile Leu Ser Leu Thr
           35                  40                  45
   Ile Gly Gly Thr Ile Ala Leu Cys Ile Gly Leu Leu Thr Ser Val Tyr
       50                  55                  60
   Leu Ala Thr Leu His Glu Asn Asp Leu Trp Phe Ser Asn Ile Lys Glu
   65                  70                  75                  80
   Val Glu Arg Glu Ile Ser Phe Arg Thr Glu Cys Gly Leu Tyr Tyr Ser
                   85                  90                  95
   Tyr Tyr Lys Gln Met Leu Gln Ala Pro Thr Leu Val Gln Gly Phe His
               100                 105                 110
   Gly Leu Ile Tyr Asp Asn Lys Thr Glu Ser Met Lys Thr Ile Asn Leu
           115                 120                 125
   Leu Gln Arg Met Asn Ile Tyr Gln Glu Val Phe Leu Ser Ile Leu Tyr
       130                 135                 140
   Arg Val Leu Pro Ile Gln Lys Tyr Leu Glu Pro Val Tyr Phe Tyr Ile
   145                 150                 155                 160
   Tyr Thr Leu Phe Gly Leu Gln Ala Ile Tyr Val Thr Ala Leu Tyr Ile
                   165                 170                 175
   Thr Ser Trp Leu Leu Ser Gly Thr Trp Leu Ser Gly Leu Leu Ala Ala
               180                 185                 190
   Phe Trp Tyr Val Thr Asn Arg Ile Asp Thr Thr Arg Val Glu Phe Thr
           195                 200                 205
   Ile Pro Leu Arg Glu Asn Trp Ala Leu Pro Phe Phe Ala Ile Gln Ile
       210                 215                 220
   Ala Ala Ile Thr Tyr Phe Leu Arg Pro Asn Leu Gln Pro Leu Ser Glu
   225                 230                 235                 240
   Arg Leu Thr Leu Leu Ala Ile Phe Ile Ser Thr Phe Leu Phe Ser Leu
                   245                 250                 255
   Thr Trp Gln Phe Asn Gln Phe Met Met Leu Met Gln Ala Leu Val Leu
               260                 265                 270
   Phe Thr Leu Asp Ser Leu Asp Met Leu Pro Ala Val Lys Ala Thr Trp
           275                 280                 285
   Leu Tyr Gly Ile Gln Ile Thr Ser Leu Leu Leu Val Cys Ile Leu Gln
       290                 295                 300
   Phe Phe Asn Ser Met Ile Leu Gly Ser Leu Leu Ile Ser Phe Asn Leu
   305                 310                 315                 320
   Ser Val Phe Ile Ala Arg Lys Leu Gln Lys Asn Leu Lys Thr Gly Ser
                   325                 330                 335
   Phe Leu Asn Arg Leu Gly Lys Leu Leu Leu His Leu Phe Met Val Leu
               340                 345                 350
   Cys Leu Thr Leu Phe Leu Asn Asn Ile Ile Lys Lys Ile Leu Asn Leu
           355                 360                 365
   Lys Ser Asp Glu His Ile Phe Lys Phe Leu Lys Ala Lys Phe Gly Leu
       370                 375                 380
   Gly Ala Thr Arg Asp Phe Asp Ala Asn Leu Tyr Leu Cys Glu Glu Ala
   385                 390                 395                 400
   Phe Gly Leu Leu Pro Phe Asn Thr Phe Gly Arg Leu Ser Asp Thr Leu
                   405                 410                 415
   Leu Phe Tyr Ala Tyr Ile Phe Val Leu Ser Ile Thr Val Ile Val Ala
               420                 425                 430
   Phe Val Val Ala Phe His Asn Leu Ser Asp Ser Thr Asn Gln Gln Ser
           435                 440                 445
   Val Gly Lys Met Glu Lys Gly Thr Val Asp Leu Lys Pro Glu Thr Ala
       450                 455                 460
   Tyr Asn Leu Ile His Thr Ile Leu Phe Gly Phe Leu Ala Leu Ser Thr
   465                 470                 475                 480
   Met Arg Met Lys Tyr Leu Trp Thr Ser His Met Cys Val Phe Ala Ser
                   485                 490                 495
   Phe Gly Leu Cys Ser Pro Glu Ile Trp Glu Leu Leu Leu Lys Ser Val
               500                 505                 510
   His Leu Tyr Asn Pro Lys Arg Ile Cys Ile Met Arg Tyr Ser Val Pro
           515                 520                 525
   Ile Leu Ile Leu Leu Tyr Leu Cys Tyr Lys Phe Trp Pro Gly Met Met
       530                 535                 540
   Asp Glu Leu Ser Glu Leu Arg Glu Phe Tyr Asp Pro Asp Thr Val Glu
   545                 550                 555                 560
   Leu Met Asn Trp Ile Asn Ser Asn Thr Pro Arg Lys Ala Val Phe Ala
                   565                 570                 575
   Gly Ser Met Gln Leu Leu Ala Gly Val Lys Leu Cys Thr Gly Arg Thr
               580                 585                 590
   Leu Thr Asn His Pro His Tyr Glu Asp Ser Ser Leu Arg Glu Arg Thr
           595                 600                 605
   Arg Ala Val Tyr Gln Ile Tyr Ala Lys Arg Ala Pro Glu Glu Val His
       610                 615                 620
   Ala Leu Leu Arg Ser Phe Gly Thr Asp Tyr Val Ile Leu Glu Asp Ser
   625                 630                 635                 640
   Ile Cys Tyr Glu Arg Arg His Arg Arg Gly Cys Arg Leu Arg Asp Leu
                   645                 650                 655
   Leu Asp Ile Ala Asn Gly His Met Met Asp Gly Pro Gly Glu Asn Asp
               660                 665                 670
   Pro Asp Leu Lys Pro Ala Asp His Pro Arg Phe Cys Glu Glu Ile Lys
           675                 680                 685
   Arg Asn Leu Pro Pro Tyr Val Ala Tyr Phe Thr Arg Val Phe Gln Asn
       690                 695                 700
   Lys Thr Phe His Val Tyr Lys Leu Ser Arg Asn Lys
   705                 710                 715
<210> 18
   <211> 112
   <212> PRT
   <213> Homo sapiens
<400> 18
   Met Met Ser Ile Arg Gln Arg Arg Glu Ile Arg Ala Thr Glu Val Ser
   1               5                   10                  15
   Glu Asp Phe Pro Ala Gln Glu Glu Asn Val Lys Leu Glu Asn Lys Leu
               20                  25                  30
   Pro Ser Gly Cys Thr Ser Arg Arg Leu Trp Lys Ile Leu Ser Leu Thr
           35                  40                  45
   Ile Gly Gly Thr Pro Phe Ala Leu Asp Phe Leu His Leu Ser Thr Leu
       50                  55                  60
   Pro Arg Tyr Met Lys Met Ile Tyr Gly Phe Leu Ile Leu Arg Lys Trp
   65                  70                  75                  80
   Ser Glu Lys Ser His Ser Glu Gln Ser Val Ala Cys Ile Thr Pro Thr
                   85                  90                  95
   Thr Ser Arg Cys Cys Arg Leu Gln Pro Ser Cys Lys Val Ile Thr Thr
               100                 105                 110
<210> 19
   <211> 1841
   <212> DNA
   <213> Homo sapiens
<400> 19
   aagtttgcgg agcggcttct gctcgtcggc cgtgcggcga ggcagggcct gggctgcgac      60
   cccggcggcc gctcgcggtc ttgggagagc tggggcgcgt gcctgaactt cccggctgcc     120
   cctgtccttg gagacctacc tgatggggac gccaggtgtg caggggcgtg gcgcgtagga     180
   gtgatttgga gaacaatgca tgtaagtctg acatcatgat gtccatccgg caaagaagag     240
   aaataagagc cacagaagtt tctgaagact ttccagccca agaagaaaat gtgaagttgg     300
   aaaataaatt gccatctggt tgtaccagta gaagattatg gaagattttg tcattgacaa     360
   ttggtggaac cattgccctt tgcattggac ttcttacatc tgtctacctt gccacgttac     420
   atgaaaatga tttatggttt tctaatatta aggaagtgga gcgagaaatc tcattcagaa     480
   cagagtgtgg cctgtattac tcctactaca agcagatgct gcaggctcca accctcgtgc     540
   aaggttttca tggcctaata tatgataata aaactgaatc tatgaagaca attaacctcc     600
   ttcagcgaat gaatatttac caagaggttt ttctcagtat tttatataga gttctaccca     660
   tacagaaata tttagagcca gtttattttt atatttacac cttatttggg ctccaggcga     720
   tctatgtcac agctctctac ataaccagct ggctactcag tggtacatgg ctgtcaggac     780
   tgttggcagc tttctggtat gtcacaaata gaatagatac cacaagagtt gagtttacca     840
   tcccactgag ggagaactgg gcgctgccat tctttgcaat tcagatagca gcaattacat     900
   atttcctgag accaaactta cagcctcttt ctgaaaggct gacacttctt gccattttca     960
   tatcaacttt tctctttagt ctgacatggc aatttaatca atttatgatg ctgatgcaag    1020
   cattagtgct gttcacactg gactccctgg acatgctgcc agcagtgaag gcgacatggc    1080
   tgtatggaat acagataaca agtttactcc tggtctgcat tcttcagttt tttaattcca    1140
   tgattcttgg atcactgctt atcagtttta acctttcagt attcattgca agaaaacttc    1200
   agaaaaatct gaaaactgga agcttcctta ataggcttgg gaaacttttg ttacatttat    1260
   ttatggtttt atgtttgaca ctttttctca acaacataat taagaaaatt cttaacctga    1320
   agtcagatga acacatattt aaatttctga aggcaaaatt tgggcttgga gcaacaaggg    1380
   attttgatgc aaatctctat ctgtgtgaag aagcttttgg cctcctgcct tttaatacat    1440
   ttggaaggct ttcagatact ctgctttttt atgcttacat attcgttctg tccatcacag    1500
   tgattgtagc attcgttgtt gcctttcata atctcagtga ttctacaaat caacaatccg    1560
   tgggtaaaat ggaaaaaggc acagttgacc tgaaaccaga aactgcctac aacttaatac    1620
   ataccattct gtttggattc ttggcattga gtacaatgag aatgaagtac ctctggacgt    1680
   cacacatgtg tgtgttcgca tcattcggcc tatgtagccc tgaaatatgg gagttacttc    1740
   tgaagtcagt ccatctttat aacccaaaga ggatatgtat aatgcgatat tcagtaccga    1800
   tattaatact gctgtatcta tgctataaga atcagaaatc t                        1841
<210> 20
   <211> 2206
   <212> DNA
   <213> Homo sapiens
<400> 20
   cggttctgcc ctccttgtac ccgcggcgcg ctgcggcccg tggcgcggcc ccgttcccgc     60
   ctagccccgt cggcctcctt cccctcccgg agccgcgcgt gaggacggct gaggccgcag    120
   gagtgatttg gagaacaatg catgtaagtc tgacatcatg atgtccatcc ggcaaagaag    180
   agaaataaga gccacagaag tttctgaaga ctttccagcc caagaagaaa atgtgaagtt    240
   ggaaaataaa ttgccatctg gttgtaccag tagaagatta tggaagattt tgtcattgac    300
   aattggtgga accattgccc tttgcattgg acttcttaca tctgtctacc ttgccacgtt    360
   acatgaaaat gatttatggt tttctaatat taaggtatgg agtttctttg accattgtat    420
   cattcactca gtgggatctc cagtagtaag ccatgtggat gaatgaccaa ggcaacacag    480
   ttttgccata aagaatccaa tctctagaaa ggttggacta tagagtgaaa taacttttgt    540
   gtttattatt ttaaaataac atattagaat ctttttttaa atttttcttt attatttatt    600
   tatttttgag atggagtctc actctgtcac ccaggctgga gtgcggtggc gcaatcttgg    660
   ctcactacaa cctctgcctc gcaggttcag gtgattcttc tggcttagcc tcccaagtag    720
   ctgggactat aggtgcgtgc caccacaccc agctaatttt tgtattttta ctagagacgg    780
   ggtttcagca tattgaccag gctgatctcg aactcctgac cttgtgatct gcctgtctca    840
   gcctcccaaa gtgctgggat tacaggcgtg agccactgcg tccagccaga atctttattt    900
   ttcattttaa ttttttgaga tagggtattg ctctgtcacc caggctagaa tgcagtggtg    960
   caaacatggg tcactgcagc ctcaacctcc tgggctcaag tgagtatcct gcctaagctt   1020
   cctgtgtcac tgggacccca ggcatgcacc acctcaccaa gctaaatttg atttttttgt   1080
   agagacaggg tctcactttg ttgcccatgc tggtctcgaa ctcctgggct caagcgatcc   1140
   tactgccctg gtcttccaaa atatgagaat gagccatagc acccagccca gaatttttat   1200
   aatcaagtga gtttttcttt tttcattaac ttattccatt tatttagcag ttattctaaa   1260
   ttagtatttt tcaagttata gattgtgaaa ttagtgcagt aggtcatgag taacattttt   1320
   cttaatgaaa tcaaaaagaa agaatactat cacatctagt agggttgagg attgttttgt   1380
   gaaactttta attttatata tatatatata tgcacaaact gggtcacagt atacaaggta   1440
   cttccttttc ttttttttct tgttggctac aacaggaaaa aaaaaaaaca gaaaaggaaa   1500
   taaaaaagcc actgctttaa atcatggggt ctaaatgtgg ctccacagag ggtcctcagc   1560
   atgttcatga ctatctaata ctctgtgcaa gtggttttgc agggcatagg gcgatgggga   1620
   agccatatgt ttccagggaa aggaactgta attttaatca gattttcagg agggttagcc   1680
   gggcgtcacg cctgtaatcc cagcactttg ggaggtcgag gcgggcagat cacttgaagt 1740
   caggagttca agaccagcct ggccaacatg gtggaaccct atctctacta aaaatacaaa 1800
   aattagccgg gcatggtgac acacacctgt aatctcagct actcaggagg ctgaggcaca 1860
   agaatcactt gaactcggga ggaagaggtt gcagtgagct gagatcccac cactgcactc 1920
   cagcctgggc aacagagcaa tactctttat caaaaaaaaa aagaaaaaag ttgagggggt 1980
   ggtctgtgac tctttaaaca cgtttccttg ttttctttct ctctctcttt ttcaacattt 2040
   ctagaactcc tcttggcatt gttttcagaa ctcgtatata acttacatgt ggaaatttgc 2100
   atccaaatat accttacatt ttaatctaat atgtcatgat ctttaaccta aactgtggtg 2160
   tctaatgact agttgcttgt aaaaataaac aaacaccttc aaagcc 2206
<210> 21
   <211> 4456
   <212> DNA
   <213> Homo sapiens
<400> 21
   aagtttgcgg agcggcttct gctcgtcggc cgtgcggcga ggcagggcct gggctgcgac     60
   cccggcggcc gctcgcggtc ttgggagagc tggggcgcgt gcctgaactt cccggctgcc    120
   cctgtccttg gagacctacc tgatggggac gccaggtgtg caggggcgtg gcgcgtagga    180
   gtgatttgga gaacaatgca tgtaagtctg acatcatgat gtccatccgg caaagaagag    240
   aaataagagc cacagaagtt tctgaagact ttccagccca agaagaaaat gtgaagttgg    300
   aaaataaatt gccatctggt tgtaccagta gaagattatg gaagattttg tcattgacaa    360
   ttggtggaac cattgccctt tgcattggac ttcttacatc tgtctacctt gccacgttac    420
   atgaaaatga tttatggttt tctaatatta aggaagtgga gcgagaaatc tcattcagaa    480
   cagagtgtgg cctgtattac tcctactaca agcagatgct gcaggctcca accctcgtgc    540
   aaggttttca tggcctaata tatgataata aaactgaatc tatgaagaca attaacctcc    600
   ttcagcgaat gaatatttac caagaggttt ttctcagtat ttatatatag gttctaccca    660
   tacagaaata tttagagcca gtttattttt atatttacac cttatttggg ctccaggcga    720
   tctatgtcac agctctctac ataaccagct ggctactcag tggtacatgg ctgtcaggac    780
   tgttggcagc tttctggtat gtcacaaata gaatagatac cacaagagtt gagtttacca    840
   tcccactgag ggagaactgg gcgctgccat tctttgcaat tcagatagca gcaattacat    900
   atttcctgag accaaactta cagcctcttt ctgaaaggct gacacttctt gccattttca    960
   tatcaacttt tctctttagt ctgacatggc aatttaatca atttatgatg ctgatgcaag   1020
   cattagtgct gttcacactg gactccctgg acatgctgcc agcagtgaag gcgacatggc   1080
   tgtatggaat acagataaca agtttactcc tggtctgcat tcttcagttt tttaattcca 1140
   tgattcttgg atcactgctt atcagtttta acctttcagt attcattgca agaaaacttc 1200
   agaaaaatct gaaaactgga agcttcctta ataggcttgg gaaacttttg ttacatttat 1260
   ttatggtttt atgtttgaca ctttttctca acaacataat taagaaaatt cttaacctga 1320
   agtcagatga acacatattt aaatttctga aggcaaaatt tgggcttgga gcaacaaggg 1380
   attttgatgc aaatctctat ctgtgtgaag aagcttttgg cctcctgcct tttaatacat 1440
   ttggaaggct ttcagatact ctgctttttt atgcttacat attcgttctg tccatcacag 1500
   tgattgtagc attcgttgtt gcctttcata atctcagtga ttctacaaat caacaatccg 1560
   tgggtaaaat ggaaaaaggc acagttgacc tgaaaccaga aactgcctac aacttaatac 1620
   ataccattct gtttggattc ttggcattga gtacaatgag aatgaagtac ctctggacgt 1680
   cacacatgtg tgtgttcgca tcattcggcc tatgtagccc tgaaatatgg gagttacttc 1740
   tgaagtcagt ccatctttat aacccaaaga ggatatgtat aatgcgatat tcagtaccga 1800
   tattaatact gctgtatcta tgctataagt tctggccagg aatgatggat gaactctccg 1860
   agttgagaga attctatgat ccagatacag tggagctgat gaactggatt aactctaaca 1920
   ctccaagaaa ggctgtgttt gcgggaagca tgcagttgct ggccggagtc aagctgtgca 1980
   cgggaaggac cctaaccaac cacccgcact atgaagacag cagcctgaga gagcggacca 2040
   gagcggttta tcagatatat gccaagaggg caccagagga agtgcatgcc ctcctaaggt 2100
   ccttcggcac tgactacgta atcctggaag acagcatctg ctacgagcgg aggcaccgcc 2160
   ggggctgccg actccgggac ctgctggaca ttgccaacgg ccacatgatg gatggcccag 2220
   gagagaatga tcctgatttg aaacctgcag accaccctcg cttctgtgaa gagatcaaaa 2280
   gaaacctgcc tccctacgtg gcctacttca ccagagtgtt ccagaacaaa accttccacg 2340
   tttacaagct gtccagaaac aagtagcgca gatttctgcc cagtgtctat ttttgatacg 2400
   gagaaactgc atcatgatga aactcaatag atgacgtttc ctatgtaagt aggtagccca 2460
   aaccttcaag ctgtgatatg agtaagttct acagatgttt acacaagtgt tgccatcttt 2520
   gaaagcatct tctacaagca gaagtctttt tcgttgtgtg tctatctttc tcattaatgt 2580
   tctttagcct aaatgttaac aactttctaa gagtgaccta gaattatgtt gttggagaga 2640
   atgatgtgtg ttccatggat acctggatag gcacataaca tgttggaaga tgagcacctg 2700
   ctcaggattt gaaatacgtt taattttcag gtgacttaag acagctatga ttgaatcaac 2760
   tagagatgat gatcgactta tttaatatga tttcactggt gaagaccaat tggtagcttt 2820
   ttaaaaagca ctttagtgtc ctgttttacc ttaaaatgtt ataatatttt ccagttgtca 2880
   tgctgtcaac attaacaaaa aaaatcatgt taaggctttg tatcaaacat tttgttacac 2940
   tctgtctgaa atgtaatgtg gagtacttca gcagtatgtg tcatgtattg tgtgtgtctg   3000
   tgtgtgtgca tgtgcacaca tgtgttttaa tgctgggcac agaaaagtgt tacaagttcc   3060
   atatcgtaag tccttaaagg ggcagaaata tatgtagcca agtagaattt attacatttt   3120
   agtgttatta ttttaaaact tactgatact ctttaacctc tcctgcagta atagttttgc   3180
   tttatttctt actcatttca atttattggg tttgcaaaat tttgtaaact ttttgtgttt   3240
   ttagcctttg tattttttac agcctagaat cttgcaaagt ctgaatattt tttaaatgtt   3300
   ctatcttaac tagttcacta atacagtatt tttagcagac agcattttca gacagcattt   3360
   tcataccaag tttgacttgt ggtctccaat cttactggga aggccctggt agtgtaattc   3420
   ttttccttat taaaaggtaa ccaagtgcct ctaagtcatg cttatttgta aacaacaaag   3480
   aagagtatat gtacctgctc aaaatttttt tgataatcgc ttatataatt aatttctaat   3540
   gatgaggaca tgtaaaagtt gccagtaaga acatagtatg catttaatta aatcaagatg   3600
   gctaatggaa ttaactttct cccctgttct tgccaggtgg aaatgattta agcatttctc   3660
   cttgcagttg tattgaagta aattaccata ggcatcaaga tggctgcatc acattttcaa   3720
   atgattttat attcagttgc tacttataaa gcagcattca aaaagtcttt tacactgtca   3780
   tgttggacac aagcagactc agcttttatc aaaacttgtt taaataaaaa attgacagta   3840
   gctgggttat taaattatgc aactgaaact cctgaattat atcttttctg tatcccttaa   3900
   taagattgga gaccactgcc gtttaggata atacaataat aaaacgtttt aatcagtact   3960
   aaaactttaa ttaagccaat aatgatgcat gcctgttgta gctgacagca tgggtcagta   4020
   catccttcag cgagtgcctt actctaattg aaaccaagca cacgtaaggt acaatatgtt   4080
   agactctgtg attttgtttt caaaatcctc tgttatggct atatttaaat ttattttaaa   4140
   tattcctgta tgtattcatc taagcatttg ggcatttgga gtcttaatat acaagaaaca   4200
   cgtacttaaa tttttatgct tatcaccgca atgatggcaa acagtgattt tttttttcat   4260
   agtttaggtg tcattgttgc cagcaccttt agtgctcagt cttcagtgaa aaatataaag   4320
   tgccaaaaaa atcttgcaag acagaatcca tacttaacac tctttccaag acactgtgac   4380
   catgtacagt agctatttcc tgatgaccaa atctctcaac gaatcatgtt attaataaat   4440
   atttttagca ctcatc                                                   4456
<210> 22
   <211> 336
   <212> DNA
   <213> Homo sapiens
<400> 22
   atgatgtcca tccggcaaag aagagaaata agagccacag aagtttctga agactttcca     60
   gcccaagaag aaaatgtgaa gttggaaaat aaattgccat ctggttgtac cagtagaaga    120
   ttatggaaga ttttgtcatt gacaattggt ggaaccccct ttgcattgga cttcttacat    180
   ctgtctacct tgccacgtta catgaaaatg atttatggtt ttctaatatt aaggaagtgg    240
   agcgagaaat ctcattcaga acagagtgtg gcctgtatta ctcctactac aagcagatgc    300
   tgcaggctcc aaccctcgtg caaggtaatt acaact                              336
<210> 23
   <211> 831
   <212> PRT
   <213> Homo sapiens
<400> 23
   Met Lys Val His Met His Thr Lys Phe Cys Leu Ile Cys Leu Leu Thr
   1               5                   10                  15
   Phe Ile Phe His His Cys Asn His Cys His Glu Glu His Asp His Gly
               20                  25                  30
   Pro Glu Ala Leu His Arg Gln His Arg Gly Met Thr Glu Leu Glu Pro
           35                  40                  45
   Ser Lys Phe Ser Lys Gln Ala Ala Glu Asn Glu Lys Lys Tyr Tyr Ile
       50                  55                  60
   Glu Lys Leu Phe Glu Arg Tyr Gly Glu Asn Gly Arg Leu Ser Phe Phe
   65                  70                  75                  80
   Gly Leu Glu Lys Leu Leu Thr Asn Leu Gly Leu Gly Glu Arg Lys Val
                   85                  90                  95
   Val Glu Ile Asn His Glu Asp Leu Gly His Asp His Val Ser His Leu
               100                 105                 110
   Asp Ile Leu Ala Val Gln Glu Gly Lys His Phe His Ser His Asn His
           115                 120                 125
   Gln His Ser His Asn His Leu Asn Ser Glu Asn Gln Thr Val Thr Ser
       130                 135                 140
   Val Ser Thr Lys Arg Asn His Lys Cys Asp Pro Glu Lys Glu Thr Val
   145                 150                 155                 160
   Glu Val Ser Val Lys Ser Asp Asp Lys His Met His Asp His Asn His
                   165                 170                 175
   Arg Leu Arg His His His Arg Leu His His His Leu Asp His Asn Asn
               180                 185                 190
   Thr His His Phe His Asn Asp Ser Ile Thr Pro Ser Glu Arg Gly Glu
           195                 200                 205
   Pro Ser Asn Glu Pro Ser Thr Glu Thr Asn Lys Thr Gln Glu Gln Ser
       210                 215                 220
   Asp Val Lys Leu Pro Lys Gly Lys Arg Lys Lys Lys Gly Arg Lys Ser
   225                 230                 235                 240
   Asn Glu Asn Ser Glu Val Ile Thr Pro Gly Phe Pro Pro Asn His Asp
                   245                 250                 255
   Gln Gly Glu Gln Tyr Glu His Asn Arg Val His Lys Pro Asp Arg Val
               260                 265                 270
   His Asn Pro Gly His Ser His Val His Leu Pro Glu Arg Asn Gly His
           275                 280                 285
   Asp Pro Gly Arg Gly His Gln Asp Leu Asp Pro Asp Asn Glu Gly Glu
       290                 295                 300
   Leu Arg His Thr Arg Lys Arg Glu Ala Pro His Val Lys Asn Asn Ala
   305                 310                 315                 320
   Ile Ile Ser Leu Arg Lys Asp Leu Asn Glu Asp Asp His His His Glu
                   325                 330                 335
   Cys Leu Asn Val Thr Gln Leu Leu Lys Tyr Tyr Gly His Gly Ala Asn
               340                 345                 350
   Ser Pro Ile Ser Thr Asp Leu Phe Thr Tyr Leu Cys Pro Ala Leu Leu
           355                 360                 365
   Tyr Gln Ile Asp Ser Arg Leu Cys Ile Glu His Phe Asp Lys Leu Leu
       370                 375                 380
   Val Glu Asp Ile Asn Lys Asp Lys Asn Leu Val Pro Glu Asp Glu Ala
   385                 390                 395                 400
   Asn Ile Gly Ala Ser Ala Trp Ile Cys Gly Ile Ile Ser Ile Thr Val
                   405                 410                 415
   Ile Ser Leu Leu Ser Leu Leu Gly Val Ile Leu Val Pro Ile Ile Asn
               420                 425                 430
   Gln Gly Cys Phe Lys Phe Leu Leu Thr Phe Leu Val Ala Leu Ala Val
           435                 440                 445
   Gly Thr Met Ser Gly Asp Ala Leu Leu His Leu Leu Pro His Ser Gln
       450                 455                 460
   Gly Gly His Asp His Ser His Gln His Ala His Gly His Gly His Ser
   465                 470                 475                 480
   His Gly His Glu Ser Asn Lys Phe Leu Glu Glu Tyr Asp Ala Val Leu
                   485                 490                 495
   Lys Gly Leu Val Ala Leu Gly Gly Ile Tyr Leu Leu Phe Ile Ile Glu
               500                 505                 510
   His Cys Ile Arg Met Phe Lys His Tyr Lys Gln Gln Arg Gly Lys Gln
           515                 520                 525
   Lys Trp Phe Met Lys Gln Asn Thr Glu Glu Ser Thr Ile Gly Arg Lys
       530                 535                 540
   Leu Ser Asp His Lys Leu Asn Asn Thr Pro Asp Ser Asp Trp Leu Gln
   545                 550                 555                 560
   Leu Lys Pro Leu Ala Gly Thr Asp Asp Ser Val Val Ser Glu Asp Arg
                   565                 570                 575
   Leu Asn Glu Thr Glu Leu Thr Asp Leu Glu Gly Gln Gln Glu Ser Pro
               580                 585                 590
   Pro Lys Asn Tyr Leu Cys Ile Glu Glu Glu Lys Ile Ile Asp His Ser
           595                 600                 605
   His Ser Asp Gly Leu His Thr Ile His Glu His Asp Leu His Ala Ala
       610                 615                 620
   Ala His Asn His His Gly Glu Asn Lys Thr Val Leu Arg Lys His Asn
   625                 630                 635                 640
   His Gln Trp His His Lys His Ser His His Ser His Gly Pro Cys His
                   645                 650                 655
   Ser Gly Ser Asp Leu Lys Glu Thr Gly Ile Ala Asn Ile Ala Trp Met
               660                 665                 670
   Val Ile Met Gly Asp Gly Ile His Asn Phe Ser Asp Gly Leu Ala Ile
               675                 680                 685
   Gly Ala Ala Phe Ser Ala Gly Leu Thr Gly Gly Ile Ser Thr Ser Ile
       690                 695                 700
   Ala Val Phe Cys His Glu Leu Pro His Glu Leu Gly Asp Phe Ala Val
   705                 710                 715                 720
   Leu Leu Lys Ala Gly Met Thr Val Lys Gln Ala Ile Val Tyr Asn Leu
                   725                 730                 735
   Leu Ser Ala Met Met Ala Tyr Ile Gly Met Leu Ile Gly Thr Ala Val
               740                 745                 750
   Gly Gln Tyr Ala Asn Asn Ile Thr Leu Trp Ile Phe Ala Val Thr Ala
           755                 760                 765
   Gly Met Phe Leu Tyr Val Ala Leu Val Asp Met Leu Pro Glu Met Leu
       770                 775                 780
   His Gly Asp Gly Asp Asn Glu Glu His Gly Phe Cys Pro Val Gly Gln
   785                 790                 795                 800
   Phe Ile Leu Gln Asn Leu Gly Leu Leu Phe Gly Phe Ala Ile Met Leu
                   805                 810                 815
   Val Ile Ala Leu Tyr Glu Asp Lys Ile Val Phe Asp Ile Gln Phe
               820                 825                 830
<210> 24
   <211> 831
   <212> PRT
   <213> Homo sapiens
<400> 24
   Met Lys Val His Met His Thr Lys Phe Cys Leu Ile Cys Leu Leu Thr
   1               5                   10                  15
   Phe Ile Phe His His Cys Asn His Cys His Glu Glu His Asp His Gly
               20                  25                  30
   Pro Glu Ala Leu His Arg Gln His Arg Gly Met Thr Glu Leu Glu Pro
           35                  40                  45
   Ser Lys Phe Ser Lys Gln Ala Ala Glu Asn Glu Lys Lys Tyr Tyr Ile
       50                  55                  60
   Glu Lys Leu Phe Glu Arg Tyr Gly Glu Asn Gly Arg Leu Ser Phe Phe
   65                  70                  75                  80
   Gly Leu Glu Lys Leu Leu Thr Asn Leu Gly Leu Gly Glu Arg Lys Val
                   85                  90                  95
   Val Glu Ile Asn His Glu Asp Leu Gly His Asp His Val Ser His Leu
               100                 105                 110
   Asp Ile Leu Ala Val Gln Glu Gly Lys His Phe His Ser His Asn His
           115                 120                 125
   Gln His Ser His Asn His Leu Asn Ser Glu Asn Gln Thr Val Thr Ser
       130                 135                 140
   Val Ser Thr Lys Arg Asn His Lys Cys Asp Pro Glu Lys Glu Thr Val
   145                 150                 155                 160
   Glu Val Ser Val Lys Ser Asp Asp Lys His Met His Asp His Asn His
                   165                 170                 175
   Arg Leu Arg His His His Arg Leu His His His Leu Asp His Asn Asn
               180                 185                 190
   Thr His His Phe His Asn Asp Ser Ile Thr Pro Ser Glu Arg Gly Glu
           195                 200                 205
   Pro Ser Asn Glu Pro Ser Thr Glu Thr Asn Lys Thr Gln Glu Gln Ser
       210                 215                 220
   Asp Val Lys Leu Pro Lys Gly Lys Arg Lys Lys Lys Gly Arg Lys Ser
   225                 230                 235                 240
   Asn Glu Asn Ser Glu Val Ile Thr Pro Gly Phe Pro Pro Asn His Asp
                   245                 250                 255
   Gln Gly Glu Gln Tyr Glu His Asn Arg Val His Lys Pro Asp Arg Val
               260                 265                 270
   His Asn Pro Gly His Ser His Val His Leu Pro Glu Arg Asn Gly His
           275                 280                 285
   Asp Pro Gly Arg Gly His Gln Asp Leu Asp Pro Asp Asn Glu Gly Glu
       290                 295                 300
   Leu Arg His Thr Arg Lys Arg Glu Ala Pro His Val Lys Asn Asn Ala
   305                 310                 315                 320
   Ile Ile Ser Leu Arg Lys Asp Leu Asn Glu Asp Asp His His His Glu
                   325                 330                 335
   Cys Leu Asn Val Thr Gln Leu Leu Lys Tyr Tyr Gly His Gly Ala Asn
               340                 345                 350
   Ser Pro Ile Ser Thr Asp Leu Phe Thr Tyr Leu Cys Pro Ala Leu Leu
           355                 360                 365
   Tyr Gln Ile Asp Ser Arg Leu Cys Ile Glu His Phe Asp Lys Leu Leu
       370                 375                 380
   Val Glu Asp Ile Asn Lys Asp Lys Asn Leu Val Pro Glu Asp Glu Ala
   385                 390                 395                 400
   Asn Ile Gly Ala Ser Ala Trp Ile Cys Gly Ile Ile Ser Ile Thr Val
                   405                 410                 415
   Ile Ser Leu Leu Ser Leu Leu Gly Val Ile Leu Val Pro Ile Ile Asn
               420                 425                 430
   Gln Gly Cys Phe Lys Phe Leu Leu Thr Phe Leu Val Ala Leu Ala Val
           435                 440                 445
   Gly Thr Met Ser Gly Asp Ala Leu Leu His Leu Leu Pro His Ser Gln
       450                 455                 460
   Gly Gly His Asp His Ser His Gln His Ala His Gly His Gly His Ser
   465                 470                 475                 480
   His Gly His Glu Ser Asn Lys Phe Leu Glu Glu Tyr Asp Ala Val Leu
                   485                 490                 495
   Lys Gly Leu Val Ala Leu Gly Gly Ile Tyr Leu Leu Phe Ile Ile Glu
               500                 505                 510
   His Cys Ile Arg Met Phe Lys His Tyr Lys Gln Gln Arg Gly Lys Gln
           515                 520                 525
   Lys Trp Phe Met Lys Gln Asn Thr Glu Glu Ser Thr Ile Gly Arg Lys
       530                 535                 540
   Leu Ser Asp His Lys Leu Asn Asn Thr Pro Asp Ser Asp Trp Leu Gln
   545                 550                 555                 560
   Leu Lys Pro Leu Ala Gly Thr Asp Asp Ser Val Val Ser Glu Asp Arg
                   565                 570                 575
   Leu Asn Glu Thr Glu Leu Thr Asp Leu Glu Gly Gln Gln Glu Ser Pro
               580                 585                 590
   Pro Lys Asn Tyr Leu Cys Ile Glu Glu Glu Lys Ile Ile Asp His Ser
           595                 600                 605
   His Ser Asp Gly Leu His Thr Ile His Glu His Asp Leu His Ala Ala
       610                 615                 620
   Ala His Asn His His Gly Glu Asn Lys Thr Val Leu Arg Lys His Asn
   625                 630                 635                 640
   His Gln Trp His His Lys His Ser His His Ser His Gly Pro Cys His
                   645                 650                 655
   Ser Gly Ser Asp Leu Lys Glu Thr Gly Ile Ala Asn Ile Ala Trp Met
               660                 665                 670
   Val Ile Met Gly Asp Gly Ile His Asn Phe Ser Asp Gly Leu Ala Ile
           675                 680                 685
   Gly Ala Ala Phe Ser Ala Gly Leu Thr Gly Gly Ile Ser Thr Ser Ile
       690                 695                 700
   Ala Val Phe Cys His Glu Leu Pro His Glu Leu Gly Asp Phe Ala Val
   705                 710                 715                 720
   Leu Leu Lys Ala Gly Met Thr Val Lys Gln Ala Ile Val Tyr Asn Leu
                   725                 730                 735
   Leu Ser Ala Met Met Ala Tyr Ile Gly Met Leu Ile Gly Thr Ala Val
               740                 745                 750
   Gly Gln Tyr Ala Asn Asn Ile Thr Leu Trp Ile Phe Ala Val Thr Ala
           755                 760                 765
   Gly Met Phe Leu Tyr Val Ala Leu Val Asp Met Leu Pro Glu Met Leu
       770                 775                 780
   His Gly Asp Gly Asp Asn Glu Glu His Gly Phe Cys Pro Val Gly Gln
   785                 790                 795                 800
   Phe Ile Leu Gln Asn Leu Gly Leu Leu Phe Gly Phe Ala Ile Met Leu
                   805                 810                 815
   Val Ile Ala Leu Tyr Glu Asp Lys Ile Val Phe Asp Ile Gln Phe
   820 825 830
<210> 25
   <211> 5227
   <212> DNA
   <213> Homo sapiens
<400> 25
   cacgatttgg tgcagccggg gtttggtacc gagcggagag gagatgcaca cggcactcga     60
   gtgtgaggaa aaatagaaat gaaggtacat atgcacacaa aattttgcct catttgtttg    120
   ctgacattta tttttcatca ttgcaaccat tgccatgaag aacatgacca tggccctgaa    180
   gcgcttcaca gacagcatcg tggaatgaca gaattggagc caagcaaatt ttcaaagcaa    240
   gctgctgaaa atgaaaaaaa atactatatt gaaaaacttt ttgagcgtta tggtgaaaat    300
   ggaagattat ccttttttgg tttggagaaa cttttaacaa acttgggcct tggagagaga    360
   aaagtagttg agattaatca tgaggatctt ggccacgatc atgtttctca tttagatatt    420
   ttggcagttc aagagggaaa gcattttcac tcacataacc accagcattc ccataatcat    480
   ttaaattcag aaaatcaaac tgtgaccagt gtatccacaa aaagaaacca taaatgtgat    540
   ccagagaaag agacagttga agtgtctgta aaatctgatg ataaacatat gcatgaccat    600
   aatcaccgcc tacgtcatca ccatcgtttg catcatcatc ttgatcataa caacactcac    660
   cattttcata atgattccat tactcccagt gagcgtgggg agcctagcaa tgaaccttca    720
   acagagacca ataaaaccca ggaacaatct gatgttaaac taccgaaagg aaagaggaag    780
   aaaaaaggga ggaaaagtaa tgaaaattct gaggttatta caccaggttt tccccctaac    840
   catgatcagg gtgaacagta tgagcataat cgggtccaca aacctgatcg tgtacataac    900
   ccaggtcatt ctcatgtaca tcttccagaa cgtaatggtc atgatcctgg tcgtggacac    960
   caagatcttg atcctgataa tgaaggtgaa cttcgacata ctagaaagag agaagcacca   1020
   catgttaaaa ataatgcaat aatttctttg agaaaagatc taaatgaaga tgaccatcat   1080
   catgaatgtt tgaacgtcac tcagttatta aaatactatg gtcatggtgc caactctccc   1140
   atctcaactg atttatttac atacctttgc cctgcattgt tatatcaaat cgacagcaga   1200
   ctttgtattg agcattttga caaactttta gttgaagata taaataagga taaaaacctg   1260
   gttcctgaag atgaggcaaa tataggggca tcagcctgga tttgtggtat catttctatc   1320
   actgtcatta gcctgctttc cttgctaggc gtgatcttgg ttcctatcat taaccaagga   1380
   tgcttcaaat tccttcttac attccttgtt gcattagctg taggaacaat gagtggagac   1440
   gcccttcttc atctactgcc ccattctcag ggtggacatg atcacagtca ccaacatgca   1500
   catgggcatg gacattctca tggacatgaa tctaacaagt ttttggaaga atatgatgct 1560
   gtattgaaag gacttgttgc tctaggaggc atttacttgc tatttatcat tgaacactgc 1620
   attagaatgt ttaagcacta caaacaacaa agaggaaaac agaaatggtt tatgaaacag 1680
   aacacagaag aatcaactat tggaagaaag ctttcagatc acaagttaaa caatacacca 1740
   gattctgact ggcttcaact caagcctctt gccggaactg atgactcggt tgtttctgaa 1800
   gatcgactta atgaaactga actgacagat ttagaaggcc aacaagaatc ccctcctaaa 1860
   aattaccttt gtatagaaga ggagaaaatc atagaccatt ctcacagtga tggattacat 1920
   accattcatg agcatgatct ccatgctgct gcacataacc accacggcga gaacaaaact 1980
   gtgctgagga agcataatca ccagtggcac cacaagcatt ctcatcattc ccatggcccc 2040
   tgtcattctg gatccgatct gaaagaaaca ggaatagcta atatagcctg gatggtgatc 2100
   atgggggatg gcatccacaa cttcagtgat gggctcgcaa ttggtgcagc tttcagtgct 2160
   ggattgacag gaggaatcag tacttctata gccgtcttct gtcatgaact gccacatgaa 2220
   ttaggagatt ttgcagttct tcttaaagca ggcatgactg taaagcaagc aattgtatac 2280
   aacctcctct ctgccatgat ggcttacata ggcatgctca taggcacagc tgttggtcag 2340
   tatgccaata acatcacact ttggatcttt gcagtcactg caggcatgtt cctctatgta 2400
   gccttggtgg atatgcttcc agaaatgttg catggtgatg gtgacaatga agaacatggc 2460
   ttttgtcctg tggggcaatt catccttcag aatttaggat tgctctttgg atttgccatt 2520
   atgctggtga ttgccctcta tgaagataaa attgtgtttg acatccagtt ttgacctttc 2580
   ccagtaatca ctgttgatta cgagaatgtt accatgcagc tttgcatctg ttccttgtac 2640
   tgtatgcaca ttgctcaaag gaaagtcagt ggcttgcact acttacaagt ttcatagatt 2700
   tgagcctaac cacaagaggc tggtgcttag tactgttttc cctgcacgta ggggtctttt 2760
   aaaaatataa agcttgtgat aaagagagga gaatatggga ctccatgaac cagtgttgat 2820
   atgtttgatt aagacttttc acaaaataat catataaaac actagtctct ttattagtag 2880
   aaacttctgt ggctatgcag aaatagagat cgaaccaaaa aaaatcattt aaactttaaa 2940
   aatattttaa atggactttg gggagacatt ttttgtgtgt tttaagaatg aattgtagtg 3000
   ctctttaatt cagctacata tattcatgtg gtgataggga tcaacttgac acaactttga 3060
   aactgcataa agtagacata ggaactagag gaaagctcag gctgcattag agtatgaatt 3120
   tagcattggg aaaagccctt attcttgaat ctagagttac tatttttgta tatatttgca 3180
   tagtgtttaa acctgcagcc taaactactg aaatttgtga ttgtatgttt gtgtgagctt 3240
   cagtttaatg aaagattcat aatggttctt tgtattatta taatacttgg tgttggggtg 3300
   ttctttctgt tttgtttttt actttaattt tgttttgatt tttttttttt ttttttggcg 3360
   ggggtaggtg agggtttgga gcatgtggtc tttttaaaaa attgtaaccc tctagaaaat   3420
   atcaaagaaa tgaaccagac gtggtttaaa tagttgattt tcctatttta acagtaccaa   3480
   ctagttaatt gggaaatgta agttctgaat gttcacattg ctttaccagt ttggcactgg   3540
   aaccaagagc acatgtcgtg gctggctaca aggttgtaaa gcagaaaatc gaagtttacc   3600
   atgtctgtaa tgtgtacatg aagtgtcaat ttagaacagt tactaggata aactccatta   3660
   ttgccatggc tgtcatggta cccaagtgac ttggaagatg catttaaatt actcagctga   3720
   aatcacttga tcatcttgtg ccaagatatg ctgttggtgc ctgataggga ttagtctttt   3780
   aggtgccctg ttctcctacc ataattgtga atgatttgtg agaagtgcaa gccatgttta   3840
   tcctgaattt ttacttaata atttgtatta ctagtcatat gcatgtagct ttctgtttac   3900
   atcctatgcc acatggtctt catttatgcc aggtaaactg tatttgaact atgtgcagct   3960
   agctttgttt taatctgctt ggcaaccagt gtagctgctg taacaatcta tcttattgtt   4020
   caaatatata agagccaaac tcttttccat tccatctaaa atgttttcat ttagtactct   4080
   tctttcctcc tactctatga acttcaaaac aaaaacaaaa ctttgagagc agcacatgca   4140
   tccaggtatt tatagattat tgccagtgtc ttttctgtat gctataagca agggagctta   4200
   ggtgttattt ctttaattta tgcttgaatc tgaaaaatta tttctgactt actccatggc   4260
   ctccttataa taagtagaag ttttatatat aattaatttt cagcattggg cactgaatta   4320
   ggacagtcct catctcattg cttggccctt caagcaacct agctaaaagg tgctgatatt   4380
   ttatttagta ctgccaactt caagtgattt agatatctat ctatctagat ttctgaacca   4440
   agatatattt atagttcact tttgggtttt tatacccacg gtaggattct gcattccagc   4500
   attaaatctg cttcatttta gaacctttat aaaagcaata gctggaatat actcccagtt   4560
   ttaaaataaa tgcctgattg atttaaagca agtaggttat gctgaagtat ataaagaagt   4620
   tttatattct ctcaaaaatg gtattatctt tctttatttg ctagattctt acaaatcttt   4680
   taagagggct gtaacagttg ctgctagtat tagggttcca catcattcta atgtatagtt   4740
   tcaagtctta atagacaatc tgaattccac tacatttctt ttggctccaa cattcctttt   4800
   agcttgacca gtctaattta aaatgtgttt gttggaggtc attaacgtta cttgtacaat   4860
   gctgtcactg tgtgacatcc atatgaattt tggtatatat caatcaatca atcaatcaca   4920
   ttgcattcaa tcaatcagct gtgattgatt gattatgctt agaaatacta tagtaactag   4980
   atgcagtgtg aattttttcc attaacaaac aaacaagtca gtggcttaaa tgtgattatg   5040
   gtcctgcaag gtgattcttg ctaaaatatc taaacttttg ttttgtttta actgaatcat   5100
   tttttaactt aaaaagctgg aaaatatcaa atgctgtttt ttttttttca ttgtcaacag   5160
   tggtgtgtca ttttatgtat gttcctaatg cttatggaac tcctccaaaa taaagttact   5220
   caaagag 5227
<210> 26
   <211> 5432
   <212> DNA
   <213> Homo sapiens
<400> 26
   agttgatcac tctgaagctt tttggctaaa gcgtttgggt ttagagcttc cattactcat     60
   tcgccttgcc caaggcctca gcaaccgacg ttcgaaagcc aggagaaaag gcgaatgata    120
   aagggcgctc cacgcatgcg ttaagaagcc gccccaactc ccccgcggcg ttctttcttg    180
   gaacaaaact agcgcggagc cacggaactc cgcagtttgc gtagacttga atttcctatt    240
   cctcggacga tccatgtgga atccgaaaaa tagaaatgaa ggtacatatg cacacaaaat    300
   tttgcctcat ttgtttgctg acatttattt ttcatcattg caaccattgc catgaagaac    360
   atgaccatgg ccctgaagcg cttcacagac agcatcgtgg aatgacagaa ttggagccaa    420
   gcaaattttc aaagcaagct gctgaaaatg aaaaaaaata ctatattgaa aaactttttg    480
   agcgttatgg tgaaaatgga agattatcct tttttggttt ggagaaactt ttaacaaact    540
   tgggccttgg agagagaaaa gtagttgaga ttaatcatga ggatcttggc cacgatcatg    600
   tttctcattt agatattttg gcagttcaag agggaaagca ttttcactca cataaccacc    660
   agcattccca taatcattta aattcagaaa atcaaactgt gaccagtgta tccacaaaaa    720
   gaaaccataa atgtgatcca gagaaagaga cagttgaagt gtctgtaaaa tctgatgata    780
   aacatatgca tgaccataat caccgcctac gtcatcacca tcgtttgcat catcatcttg    840
   atcataacaa cactcaccat tttcataatg attccattac tcccagtgag cgtggggagc    900
   ctagcaatga accttcaaca gagaccaata aaacccagga acaatctgat gttaaactac    960
   cgaaaggaaa gaggaagaaa aaagggagga aaagtaatga aaattctgag gttattacac   1020
   caggttttcc ccctaaccat gatcagggtg aacagtatga gcataatcgg gtccacaaac   1080
   ctgatcgtgt acataaccca ggtcattctc atgtacatct tccagaacgt aatggtcatg   1140
   atcctggtcg tggacaccaa gatcttgatc ctgataatga aggtgaactt cgacatacta   1200
   gaaagagaga agcaccacat gttaaaaata atgcaataat ttctttgaga aaagatctaa   1260
   atgaagatga ccatcatcat gaatgtttga acgtcactca gttattaaaa tactatggtc   1320
   atggtgccaa ctctcccatc tcaactgatt tatttacata cctttgccct gcattgttat   1380
   atcaaatcga cagcagactt tgtattgagc attttgacaa acttttagtt gaagatataa   1440
   ataaggataa aaacctggtt cctgaagatg aggcaaatat aggggcatca gcctggattt   1500
   gtggtatcat ttctatcact gtcattagcc tgctttcctt gctaggcgtg atcttggttc   1560
   ctatcattaa ccaaggatgc ttcaaattcc ttcttacatt ccttgttgca ttagctgtag   1620
   gaacaatgag tggagacgcc cttcttcatc tactgcccca ttctcagggt ggacatgatc   1680
   acagtcacca acatgcacat gggcatggac attctcatgg acatgaatct aacaagtttt   1740
   tggaagaata tgatgctgta ttgaaaggac ttgttgctct aggaggcatt tacttgctat   1800
   ttatcattga acactgcatt agaatgttta agcactacaa acaacaaaga ggaaaacaga   1860
   aatggtttat gaaacagaac acagaagaat caactattgg aagaaagctt tcagatcaca   1920
   agttaaacaa tacaccagat tctgactggc ttcaactcaa gcctcttgcc ggaactgatg   1980
   actcggttgt ttctgaagat cgacttaatg aaactgaact gacagattta gaaggccaac   2040
   aagaatcccc tcctaaaaat tacctttgta tagaagagga gaaaatcata gaccattctc   2100
   acagtgatgg attacatacc attcatgagc atgatctcca tgctgctgca cataaccacc   2160
   acggcgagaa caaaactgtg ctgaggaagc ataatcacca gtggcaccac aagcattctc   2220
   atcattccca tggcccctgt cattctggat ccgatctgaa agaaacagga atagctaata   2280
   tagcctggat ggtgatcatg ggggatggca tccacaactt cagtgatggg ctcgcaattg   2340
   gtgcagcttt cagtgctgga ttgacaggag gaatcagtac ttctatagcc gtcttctgtc   2400
   atgaactgcc acatgaatta ggagattttg cagttcttct taaagcaggc atgactgtaa   2460
   agcaagcaat tgtatacaac ctcctctctg ccatgatggc ttacataggc atgctcatag   2520
   gcacagctgt tggtcagtat gccaataaca tcacactttg gatctttgca gtcactgcag   2580
   gcatgttcct ctatgtagcc ttggtggata tgcttccaga aatgttgcat ggtgatggtg   2640
   acaatgaaga acatggcttt tgtcctgtgg ggcaattcat ccttcagaat ttaggattgc   2700
   tctttggatt tgccattatg ctggtgattg ccctctatga agataaaatt gtgtttgaca   2760
   tccagttttg acctttccca gtaatcactg ttgattacga gaatgttacc atgcagcttt   2820
   gcatctgttc cttgtactgt atgcacattg ctcaaaggaa agtcagtggc ttgcactact   2880
   tacaagtttc atagatttga gcctaaccac aagaggctgg tgcttagtac tgttttccct   2940
   gcacgtaggg gtcttttaaa aatataaagc ttgtgataaa gagaggagaa tatgggactc   3000
   catgaaccag tgttgatatg tttgattaag acttttcaca aaataatcat ataaaacact   3060
   agtctcttta ttagtagaaa cttctgtggc tatgcagaaa tagagatcga accaaaaaaa   3120
   atcatttaaa ctttaaaaat attttaaatg gactttgggg agacattttt tgtgtgtttt   3180
   aagaatgaat tgtagtgctc tttaattcag ctacatatat tcatgtggtg atagggatca   3240
   acttgacaca actttgaaac tgcataaagt agacatagga actagaggaa agctcaggct   3300
   gcattagagt atgaatttag cattgggaaa agcccttatt cttgaatcta gagttactat   3360
   ttttgtatat atttgcatag tgtttaaacc tgcagcctaa actactgaaa tttgtgattg   3420
   tatgtttgtg tgagcttcag tttaatgaaa gattcataat ggttctttgt attattataa   3480
   tacttggtgt tggggtgttc tttctgtttt gttttttact ttaattttgt tttgattttt 3540
   tttttttttt tttggcgggg gtaggtgagg gtttggagca tgtggtcttt ttaaaaaatt 3600
   gtaaccctct agaaaatatc aaagaaatga accagacgtg gtttaaatag ttgattttcc 3660
   tattttaaca gtaccaacta gttaattggg aaatgtaagt tctgaatgtt cacattgctt 3720
   taccagtttg gcactggaac caagagcaca tgtcgtggct ggctacaagg ttgtaaagca 3780
   gaaaatcgaa gtttaccatg tctgtaatgt gtacatgaag tgtcaattta gaacagttac 3840
   taggataaac tccattattg ccatggctgt catggtaccc aagtgacttg gaagatgcat 3900
   ttaaattact cagctgaaat cacttgatca tcttgtgcca agatatgctg ttggtgcctg 3960
   atagggatta gtcttttagg tgccctgttc tcctaccata attgtgaatg atttgtgaga 4020
   agtgcaagcc atgtttatcc tgaattttta cttaataatt tgtattacta gtcatatgca 4080
   tgtagctttc tgtttacatc ctatgccaca tggtcttcat ttatgccagg taaactgtat 4140
   ttgaactatg tgcagctagc tttgttttaa tctgcttggc aaccagtgta gctgctgtaa 4200
   caatctatct tattgttcaa atatataaga gccaaactct tttccattcc atctaaaatg 4260
   ttttcattta gtactcttct ttcctcctac tctatgaact tcaaaacaaa aacaaaactt 4320
   tgagagcagc acatgcatcc aggtatttat agattattgc cagtgtcttt tctgtatgct 4380
   ataagcaagg gagcttaggt gttatttctt taatttatgc ttgaatctga aaaattattt 4440
   ctgacttact ccatggcctc cttataataa gtagaagttt tatatataat taattttcag 4500
   cattgggcac tgaattagga cagtcctcat ctcattgctt ggcccttcaa gcaacctagc 4560
   taaaaggtgc tgatatttta tttagtactg ccaacttcaa gtgatttaga tatctatcta 4620
   tctagatttc tgaaccaaga tatatttata gttcactttt gggtttttat acccacggta 4680
   ggattctgca ttccagcatt aaatctgctt cattttagaa cctttataaa agcaatagct 4740
   ggaatatact cccagtttta aaataaatgc ctgattgatt taaagcaagt aggttatgct 4800
   gaagtatata aagaagtttt atattctctc aaaaatggta ttatctttct ttatttgcta 4860
   gattcttaca aatcttttaa gagggctgta acagttgctg ctagtattag ggttccacat 4920
   cattctaatg tatagtttca agtcttaata gacaatctga attccactac atttcttttg 4980
   gctccaacat tccttttagc ttgaccagtc taatttaaaa tgtgtttgtt ggaggtcatt 5040
   aacgttactt gtacaatgct gtcactgtgt gacatccata tgaattttgg tatatatcaa 5100
   tcaatcaatc aatcacattg cattcaatca atcagctgtg attgattgat tatgcttaga 5160
   aatactatag taactagatg cagtgtgaat tttttccatt aacaaacaaa caagtcagtg 5220
   gcttaaatgt gattatggtc ctgcaaggtg attcttgcta aaatatctaa acttttgttt 5280
   tgttttaact gaatcatttt ttaacttaaa aagctggaaa atatcaaatg ctgttttttt 5340
   tttttcattg tcaacagtgg tgtgtcattt tatgtatgtt cctaatgctt atggaactcc 5400
   tccaaaataa agttactcaa agagagcaaa ta 5432
<210> 27
   <211> 543
   <212> PRT
   <213> Homo sapiens
<400> 27
<210> 28
   <211> 938
   <212> PRT
   <213> Homo sapiens
<400> 28
   Met Pro Ala Leu Glu His Met Asn Gln Ile Leu His Ile Leu Phe Val
   1               5                   10                  15
   Phe Leu Pro Phe Leu Trp Ala Leu Gly Thr Leu Pro Pro Pro Asp Ala
               20                  25                  30
   Leu Leu Leu Trp Ala Met Glu Gln Val Leu Glu Phe Gly Leu Gly Gly
           35                  40                  45
   Ser Ser Met Ser Thr His Leu Arg Leu Leu Val Met Phe Ile Met Ser
       50                  55                  60
   Ala Gly Thr Ala Ile Ala Ser Tyr Phe Ile Pro Ser Thr Val Gly Val
   65                  70                  75                  80
   Val Leu Phe Met Thr Gly Phe Gly Phe Leu Leu Ser Leu Asn Leu Ser
                   85                  90                  95
   Asp Met Gly His Lys Ile Gly Thr Lys Ser Lys Asp Leu Pro Ser Gly
               100                 105                 110
   Pro Glu Lys His Phe Ser Trp Lys Glu Cys Leu Phe Tyr Ile Ile Ile
           115                 120                 125
   Leu Val Leu Ala Leu Leu Glu Thr Ser Leu Leu His His Phe Ala Gly
       130                 135                 140
   Phe Ser Gln Ile Ser Lys Ser Asn Ser Gln Ala Ile Val Gly Tyr Gly
   145                 150                 155                 160
   Leu Met Ile Leu Leu Ile Ile Leu Trp Ile Leu Arg Glu Ile Gln Ser
                   165                 170                 175
   Val Tyr Ile Ile Gly Ile Phe Arg Asn Pro Phe Tyr Pro Lys Asp Val
               180                 185                 190
   Gln Thr Val Thr Val Phe Phe Glu Lys Gln Thr Arg Leu Met Lys Ile
           195                 200                 205
   Gly Ile Val Arg Arg Ile Leu Leu Thr Leu Val Ser Pro Phe Ala Met
       210                 215                 220
   Ile Ala Phe Leu Ser Leu Asp Ser Ser Leu Gln Gly Leu His Ser Val
   225                 230                 235                 240
   Ser Val Cys Ile Gly Phe Thr Arg Ala Phe Arg Met Val Trp Gln Asn
                   245                 250                 255
   Thr Glu Asn Ala Leu Leu Glu Thr Val Ile Val Ser Thr Val His Leu
               260                 265                 270
   Ile Ser Ser Thr Asp Ile Trp Trp Asn Arg Ser Leu Asp Thr Gly Leu
           275                 280                 285
   Arg Leu Leu Leu Val Gly Ile Ile Arg Asp Arg Leu Ile Gln Phe Ile
       290                 295                 300
   Ser Lys Leu Gln Phe Ala Val Thr Val Leu Leu Thr Ser Trp Thr Glu
   305                 310                 315                 320
   Lys Lys Gln Arg Arg Lys Thr Thr Ala Thr Leu Cys Ile Leu Asn Ile
                   325                 330                 335
   Val Phe Ser Pro Phe Val Leu Val Ile Ile Val Phe Ser Thr Leu Leu
               340                 345                 350
   Ser Ser Pro Leu Leu Pro Leu Phe Thr Leu Pro Val Phe Leu Val Gly
           355                 360                 365
   Phe Pro Arg Pro Ile Gln Ser Trp Pro Gly Ala Ala Gly Thr Thr Ala
       370                 375                 380
   Cys Val Cys Ala Asp Thr Val Tyr Tyr Tyr Gln Met Val Pro Arg Leu
   385                 390                 395                 400
   Thr Ala Val Leu Gln Thr Ala Met Ala Ala Gly Ser Leu Gly Leu Leu
                   405                 410                 415
   Leu Pro Gly Ser His Tyr Leu Gly Arg Phe Gln Asp Arg Leu Met Trp
               420                 425                 430
   Ile Met Ile Leu Glu Cys Gly Tyr Thr Tyr Cys Ser Ile Asn Ile Lys
           435                 440                 445
   Gly Leu Glu Leu Gln Glu Thr Ser Cys His Thr Ala Glu Ala Arg Arg
       450                 455                 460
   Val Asp Glu Val Phe Glu Asp Ala Phe Glu Gln Glu Tyr Thr Arg Val
   465                 470                 475                 480
   Cys Ser Leu Asn Glu His Phe Gly Asn Val Leu Thr Pro Cys Thr Val
                   485                 490                 495
   Leu Pro Val Lys Leu Tyr Ser Asp Ala Arg Asn Val Leu Ser Gly Ile
               500                 505                 510
   Ile Asp Ser His Glu Asn Leu Lys Glu Phe Lys Gly Asp Leu Ile Lys
           515                 520                 525
   Val Leu Val Trp Ile Leu Val Gln Tyr Cys Ser Lys Arg Pro Gly Met
       530                 535                 540
   Lys Glu Asn Val His Asn Thr Glu Asn Lys Gly Lys Ala Pro Leu Met
   545                 550                 555                 560
   Leu Pro Ala Leu Asn Thr Leu Pro Pro Pro Lys Ser Pro Glu Asp Ile
                   565                 570                 575
   Asp Ser Leu Asn Ser Glu Thr Phe Asn Asp Trp Ser Asp Asp Asn Ile
               580                 585                 590
   Phe Asp Asp Glu Pro Thr Ile Lys Lys Val Ile Glu Glu Lys His Gln
           595                 600                 605
   Leu Lys Asp Leu Pro Gly Thr Asn Leu Phe Ile Pro Gly Ser Val Glu
       610                 615                 620
   Ser Gln Arg Val Gly Asp His Ser Thr Gly Thr Val Pro Glu Asn Asp
   625                 630                 635                 640
   Leu Tyr Lys Ala Val Leu Leu Gly Tyr Pro Ala Val Asp Lys Gly Lys
                   645                 650                 655
   Gln Glu Asp Met Pro Tyr Ile Pro Leu Met Glu Phe Ser Cys Ser His
               660                 665                 670
   Ser His Leu Val Cys Leu Pro Ala Glu Trp Arg Thr Ser Cys Met Pro
           675                 680                 685
   Ser Ser Lys Met Lys Glu Met Ser Ser Leu Phe Pro Glu Asp Trp Tyr
       690                 695                 700
   Gln Phe Val Leu Arg Gln Leu Glu Cys Tyr His Ser Glu Glu Lys Ala
   705                 710                 715                 720
   Ser Asn Val Leu Glu Glu Ile Ala Lys Asp Lys Val Leu Lys Asp Phe
                   725                 730                 735
   Tyr Val His Thr Val Met Thr Cys Tyr Phe Ser Leu Phe Gly Ile Asp
               740                 745                 750
   Asn Met Ala Pro Ser Pro Gly His Ile Leu Arg Val Tyr Gly Gly Val
           755                 760                 765
   Leu Pro Trp Ser Val Ala Leu Asp Trp Leu Thr Glu Lys Pro Glu Leu
       770                 775                 780
   Phe Gln Leu Ala Leu Lys Ala Phe Arg Tyr Thr Leu Lys Leu Met Ile
   785                 790                 795                 800
   Asp Lys Ala Ser Leu Gly Pro Ile Glu Asp Phe Arg Glu Leu Ile Lys
                   805                 810                 815
   Tyr Leu Glu Glu Tyr Glu Arg Asp Trp Tyr Ile Gly Leu Val Ser Asp
               820                 825                 830
   Glu Lys Trp Lys Glu Ala Ile Leu Gln Glu Lys Pro Tyr Leu Phe Ser
           835                 840                 845
   Leu Gly Tyr Asp Ser Asn Met Gly Ile Tyr Thr Gly Arg Val Leu Ser
       850                 855                 860
   Leu Gln Glu Leu Leu Ile Gln Val Gly Lys Leu Asn Pro Glu Ala Val
   865                 870                 875                 880
   Arg Gly Gln Trp Ala Asn Leu Ser Trp Glu Leu Leu Tyr Ala Thr Asn
                   885                 890                 895
   Asp Asp Glu Glu Arg Tyr Ser Ile Gln Ala His Pro Leu Leu Leu Arg
               900                 905                 910
   Asn Leu Thr Val Gln Ala Ala Glu Pro Pro Leu Gly Tyr Pro Ile Tyr
           915                 920                 925
   Ser Ser Lys Pro Leu His Ile His Leu Tyr
       930                 935
<210> 29
   <211> 230
   <212> PRT
   <213> Homo sapiens
<400> 29
   Met Ser Pro Asp Val Pro Leu Leu Asn Asp Tyr Lys Gln Asp Phe Phe
   1               5                   10                  15
   Leu Lys Arg Phe Pro Gln Thr Val Leu Gly Gly Pro Arg Phe Lys Leu
               20                  25                  30
   Gly Tyr Cys Ala Pro Pro Tyr Ile Tyr Val Asn Gln Ile Ile Leu Phe
           35                  40                  45
   Leu Met Pro Trp Val Trp Gly Gly Val Gly Thr Leu Leu Tyr Gln Leu
       50                  55                  60
   Gly Ile Leu Lys Asp Tyr Tyr Thr Ala Ala Leu Ser Gly Gly Leu Met
   65                  70                  75                  80
   Leu Phe Thr Ala Phe Val Ile Gln Phe Thr Ser Leu Tyr Ala Lys Asn
                   85                  90                  95
   Lys Ser Thr Thr Val Glu Arg Ile Leu Thr Thr Asp Ile Leu Ala Glu
               100                 105                 110
   Glu Asp Glu His Glu Phe Thr Ser Cys Thr Gly Ala Glu Thr Val Lys
           115                 120                 125
   Phe Leu Ile Pro Gly Lys Lys Tyr Val Ala Asn Thr Val Phe His Ser
       130                 135                 140
   Ile Leu Ala Gly Leu Ala Cys Gly Leu Gly Thr Trp Tyr Leu Leu Pro
   145                 150                 155                 160
   Asn Arg Ile Thr Leu Leu Tyr Gly Ser Thr Gly Gly Thr Ala Leu Leu
                   165                 170                 175
   Phe Phe Phe Gly Trp Met Thr Leu Cys Ile Ala Glu Tyr Ser Leu Ile
               180                 185                 190
   Val Asn Thr Ala Thr Glu Thr Ala Thr Phe Gln Thr Gln Asp Thr Tyr
           195                 200                 205
   Glu Ile Ile Pro Leu Met Arg Pro Leu Tyr Ile Phe Phe Phe Val Ser
       210                 215                 220
   Val Asp Leu Ala His Arg
   225                 230
<210> 30
   <211> 1172
   <212> PRT
   <213> Homo sapiens
<400> 30
   Met Ser Pro Asp Val Pro Leu Leu Asn Asp Tyr Lys Gln Asp Phe Phe
   1               5                   10                  15
   Leu Lys Arg Phe Pro Gln Thr Val Leu Gly Gly Pro Arg Phe Lys Leu
               20                  25                  30
   Gly Tyr Cys Ala Pro Pro Tyr Ile Tyr Val Asn Gln Ile Ile Leu Phe
           35                  40                  45
   Leu Met Pro Trp Val Trp Gly Gly Val Gly Thr Leu Leu Tyr Gln Leu
       50                  55                  60
   Gly Ile Leu Lys Asp Tyr Tyr Thr Ala Ala Leu Ser Gly Gly Leu Met
   65                  70                  75                  80
   Leu Phe Thr Ala Phe Val Ile Gln Phe Thr Ser Leu Tyr Ala Lys Asn
                   85                  90                  95
   Lys Ser Thr Thr Val Glu Arg Ile Leu Thr Thr Asp Ile Leu Ala Glu
               100                 105                 110
   Glu Asp Glu His Glu Phe Thr Ser Cys Thr Gly Ala Glu Thr Val Lys
           115                 120                 125
   Phe Leu Ile Pro Gly Lys Lys Tyr Val Ala Asn Thr Val Phe His Ser
       130                 135                 140
   Ile Leu Ala Gly Leu Ala Cys Gly Leu Gly Thr Trp Tyr Leu Leu Pro
   145                 150                 155                 160
   Asn Arg Ile Thr Leu Leu Tyr Gly Ser Thr Gly Gly Thr Ala Leu Leu
                   165                 170                 175
   Phe Phe Phe Gly Trp Met Thr Leu Cys Ile Ala Glu Tyr Ser Leu Ile
               180                 185                 190
   Val Asn Thr Ala Thr Glu Thr Ala Thr Phe Gln Thr Gln Asp Thr Tyr
           195                 200                 205
   Glu Ile Ile Pro Leu Met Arg Pro Leu Tyr Ile Phe Phe Phe Val Ser
       210                 215                 220
   Val Asp Leu Ala His Arg Phe Val Val Asn Met Pro Ala Leu Glu His
   225                 230                 235                 240
   Met Asn Gln Ile Leu His Ile Leu Phe Val Phe Leu Pro Phe Leu Trp
                   245                 250                 255
   Ala Leu Gly Thr Leu Pro Pro Pro Asp Ala Leu Leu Leu Trp Ala Met
               260                 265                 270
   Glu Gln Val Leu Glu Phe Gly Leu Gly Gly Ser Ser Met Ser Thr His
           275                 280                 285
   Leu Arg Leu Leu Val Met Phe Ile Met Ser Ala Gly Thr Ala Ile Ala
       290                 295                 300
   Ser Tyr Phe Ile Pro Ser Thr Val Gly Val Val Leu Phe Met Thr Gly
   305                 310                 315                 320
   Phe Gly Phe Leu Leu Ser Leu Asn Leu Ser Asp Met Gly His Lys Ile
                   325                 330                 335
   Gly Thr Lys Ser Lys Asp Leu Pro Ser Gly Pro Glu Lys His Phe Ser
               340                 345                 350
   Trp Lys Glu Cys Leu Phe Tyr Ile Ile Ile Leu Val Leu Ala Leu Leu
           355                 360                 365
   Glu Thr Ser Leu Leu His His Phe Ala Gly Phe Ser Gln Ile Ser Lys
       370                 375                 380
   Ser Asn Ser Gln Ala Ile Val Gly Tyr Gly Leu Met Ile Leu Leu Ile
   385                 390                 395                 400
   Ile Leu Trp Ile Leu Arg Glu Ile Gln Ser Val Tyr Ile Ile Gly Ile
                   405                 410                 415
   Phe Arg Asn Pro Phe Tyr Pro Lys Asp Val Gln Thr Val Thr Val Phe
               420                 425                 430
   Phe Glu Lys Gln Thr Arg Leu Met Lys Ile Gly Ile Val Arg Arg Ile
           435                 440                 445
   Leu Leu Thr Leu Val Ser Pro Phe Ala Met Ile Ala Phe Leu Ser Leu
       450                 455                 460
   Asp Ser Ser Leu Gln Gly Leu His Ser Val Ser Val Cys Ile Gly Phe
   465                 470                 475                 480
   Thr Arg Ala Phe Arg Met Val Trp Gln Asn Thr Glu Asn Ala Leu Leu
                   485                 490                 495
   Glu Thr Val Ile Val Ser Thr Val His Leu Ile Ser Ser Thr Asp Ile
               500                 505                 510
   Trp Trp Asn Arg Ser Leu Asp Thr Gly Leu Arg Leu Leu Leu Val Gly
           515                 520                 525
   Ile Ile Arg Asp Arg Leu Ile Gln Phe Ile Ser Lys Leu Gln Phe Ala
       530                 535                 540
   Val Thr Val Leu Leu Thr Ser Trp Thr Glu Lys Lys Gln Arg Arg Lys
   545                 550                 555                 560
   Thr Thr Ala Thr Leu Cys Ile Leu Asn Ile Val Phe Ser Pro Phe Val
                   565                 570                 575
   Leu Val Ile Ile Val Phe Ser Thr Leu Leu Ser Ser Pro Leu Leu Pro
               580                 585                 590
   Leu Phe Thr Leu Pro Val Phe Leu Val Gly Phe Pro Arg Pro Ile Gln
           595                 600                 605
   Ser Trp Pro Gly Ala Ala Gly Thr Thr Ala Cys Val Cys Ala Asp Thr
       610                 615                 620
   Val Tyr Tyr Tyr Gln Met Val Pro Arg Leu Thr Ala Val Leu Gln Thr
   625                 630                 635                 640
   Ala Met Ala Ala Gly Ser Leu Gly Leu Leu Leu Pro Gly Ser His Tyr
                   645                 650                 655
   Leu Gly Arg Phe Gln Asp Arg Leu Met Trp Ile Met Ile Leu Glu Cys
               660                 665                 670
   Gly Tyr Thr Tyr Cys Ser Ile Asn Ile Lys Gly Leu Glu Leu Gln Glu
           675                 680                 685
   Thr Ser Cys His Thr Ala Glu Ala Arg Arg Val Asp Glu Val Phe Glu
       690                 695                 700
   Asp Ala Phe Glu Gln Glu Tyr Thr Arg Val Cys Ser Leu Asn Glu His
   705                 710                 715                 720
   Phe Gly Asn Val Leu Thr Pro Cys Thr Val Leu Pro Val Lys Leu Tyr
                   725                 730                 735
   Ser Asp Ala Arg Asn Val Leu Ser Gly Ile Ile Asp Ser His Glu Asn
               740                 745                 750
   Leu Lys Glu Phe Lys Gly Asp Leu Ile Lys Val Leu Val Trp Ile Leu
           755                 760                 765
   Val Gln Tyr Cys Ser Lys Arg Pro Gly Met Lys Glu Asn Val His Asn
       770                 775                 780
   Thr Glu Asn Lys Gly Lys Ala Pro Leu Met Leu Pro Ala Leu Asn Thr
   785                 790                 795                 800
   Leu Pro Pro Pro Lys Ser Pro Glu Asp Ile Asp Ser Leu Asn Ser Glu
                   805                 810                 815
   Thr Phe Asn Asp Trp Ser Asp Asp Asn Ile Phe Asp Asp Glu Pro Thr
               820                 825                 830
   Ile Lys Lys Val Ile Glu Glu Lys His Gln Leu Lys Asp Leu Pro Gly
           835                 840                 845
   Thr Asn Leu Phe Ile Pro Gly Ser Val Glu Ser Gln Arg Val Gly Asp
       850                 855                 860
   His Ser Thr Gly Thr Val Pro Glu Asn Asp Leu Tyr Lys Ala Val Leu
   865                 870                 875                 880
   Leu Gly Tyr Pro Ala Val Asp Lys Gly Lys Gln Glu Asp Met Pro Tyr
                   885                 890                 895
   Ile Pro Leu Met Glu Phe Ser Cys Ser His Ser His Leu Val Cys Leu
               900                 905                 910
   Pro Ala Glu Trp Arg Thr Ser Cys Met Pro Ser Ser Lys Met Lys Glu
           915                 920                 925
   Met Ser Ser Leu Phe Pro Glu Asp Trp Tyr Gln Phe Val Leu Arg Gln
       930                 935                 940
   Leu Glu Cys Tyr His Ser Glu Glu Lys Ala Ser Asn Val Leu Glu Glu
   945                 950                 955                 960
   Ile Ala Lys Asp Lys Val Leu Lys Asp Phe Tyr Val His Thr Val Met
                   965                 970                 975
   Thr Cys Tyr Phe Ser Leu Phe Gly Ile Asp Asn Met Ala Pro Ser Pro
               980                 985                 990
   Gly His Ile Leu Arg Val Tyr Gly  Gly Val Leu Pro Trp  Ser Val Ala
           995                 1000                1005
   Leu Asp  Trp Leu Thr Glu Lys  Pro Glu Leu Phe Gln  Leu Ala Leu
       1010                1015                1020
   Lys Ala  Phe Arg Tyr Thr Leu  Lys Leu Met Ile Asp  Lys Ala Ser
       1025                1030                1035
   Leu Gly  Pro Ile Glu Asp Phe  Arg Glu Leu Ile Lys  Tyr Leu Glu
       1040                1045                1050
   Glu Tyr  Glu Arg Asp Trp Tyr  Ile Gly Leu Val Ser  Asp Glu Lys
       1055                1060                1065
   Trp Lys  Glu Ala Ile Leu Gln  Glu Lys Pro Tyr Leu  Phe Ser Leu
       1070                1075                1080
   Gly Tyr  Asp Ser Asn Met Gly  Ile Tyr Thr Gly Arg  Val Leu Ser
       1085                1090                1095
   Leu Gln  Glu Leu Leu Ile Gln  Val Gly Lys Leu Asn  Pro Glu Ala
       1100                1105                1110
   Val Arg  Gly Gln Trp Ala Asn  Leu Ser Trp Glu Leu  Leu Tyr Ala
       1115                 1120                 1125
   Thr Asn  Asp Asp Glu Glu Arg  Tyr Ser Ile Gln Ala  His Pro Leu
       1130                 1135                 1140
   Leu Leu  Arg Asn Leu Thr Val  Gln Ala Ala Glu Pro  Pro Leu Gly
       1145                 1150                 1155
   Tyr Pro  Ile Tyr Ser Ser Lys  Pro Leu His Ile His  Leu Tyr
       1160                 1165                 1170
<210> 31
   <211> 873
   <212> PRT
   <213> Homo sapiens
<400> 31
   Met Pro Ala Leu Glu His Met Asn Gln Ile Leu His Ile Leu Phe Val
   1               5                   10                  15
   Phe Leu Pro Phe Leu Trp Ala Leu Gly Thr Leu Pro Pro Pro Asp Ala
               20                  25                  30
   Leu Leu Leu Trp Ala Met Glu Gln Val Leu Glu Phe Gly Leu Gly Gly
           35                  40                  45
   Ser Ser Met Ser Thr His Leu Arg Leu Leu Val Met Phe Ile Met Ser
       50                  55                  60
   Ala Gly Thr Ala Ile Ala Ser Tyr Phe Ile Pro Ser Thr Val Gly Val
   65                  70                  75                  80
   Val Leu Phe Met Thr Gly Phe Gly Phe Leu Leu Ser Leu Asn Leu Ser
                   85                  90                  95
   Asp Met Gly His Lys Ile Gly Thr Lys Ser Lys Asp Leu Pro Ser Gly
               100                 105                 110
   Pro Glu Lys His Phe Ser Trp Lys Glu Cys Leu Phe Tyr Ile Ile Ile
           115                 120                 125
   Leu Val Leu Ala Leu Leu Glu Thr Ser Leu Leu His His Phe Ala Gly
       130                 135                 140
   Phe Ser Gln Ile Ser Lys Ser Asn Ser Gln Ala Ile Val Gly Tyr Gly
   145                 150                 155                 160
   Leu Met Ile Leu Leu Ile Ile Leu Trp Ile Leu Arg Glu Ile Gln Ser
                   165                 170                 175
   Val Tyr Ile Ile Gly Ile Phe Arg Asn Pro Phe Tyr Pro Lys Asp Val
               180                 185                 190
   Gln Thr Val Thr Val Phe Phe Glu Lys Gln Thr Arg Leu Met Lys Ile
           195                 200                 205
   Gly Ile Val Arg Arg Ile Leu Leu Thr Leu Val Ser Pro Phe Ala Met
       210                 215                 220
   Ile Ala Phe Leu Ser Leu Asp Ser Ser Leu Gln Gly Leu His Ser Val
   225                 230                 235                 240
   Ser Val Cys Ile Gly Phe Thr Arg Ala Phe Arg Met Val Trp Gln Asn
                   245                 250                 255
   Thr Glu Asn Ala Leu Leu Glu Thr Val Ile Val Ser Thr Val His Leu
               260                 265                 270
   Ile Ser Ser Thr Asp Ile Trp Trp Asn Arg Ser Leu Asp Thr Gly Leu
           275                 280                 285
   Arg Leu Leu Leu Val Gly Ile Ile Arg Asp Arg Leu Ile Gln Phe Ile
       290                 295                 300
   Ser Lys Leu Gln Phe Ala Val Thr Val Leu Leu Thr Ser Trp Thr Glu
   305                 310                 315                 320
   Lys Lys Gln Arg Arg Lys Thr Thr Ala Thr Leu Cys Ile Leu Asn Ile
                   325                 330                 335
   Val Phe Ser Pro Phe Val Leu Val Ile Ile Val Phe Ser Thr Leu Leu
               340                 345                 350
   Ser Ser Pro Leu Leu Pro Leu Phe Thr Leu Pro Val Phe Leu Val Gly
           355                 360                 365
   Phe Pro Arg Pro Ile Gln Ser Trp Pro Gly Ala Ala Gly Thr Thr Ala
       370                 375                 380
   Cys Val Cys Ala Asp Thr Val Tyr Tyr Tyr Gln Met Val Pro Arg Leu
   385                 390                 395                 400
   Thr Ala Val Leu Gln Thr Ala Met Ala Ala Gly Ser Leu Gly Leu Leu
                   405                 410                 415
   Leu Pro Gly Ser His Tyr Leu Gly Arg Phe Gln Asp Arg Leu Met Trp
               420                 425                 430
   Ile Met Ile Leu Glu Cys Gly Tyr Thr Tyr Cys Ser Ile Asn Ile Lys
           435                 440                 445
   Gly Leu Glu Leu Gln Glu Thr Ser Cys His Thr Ala Glu Ala Arg Arg
       450                 455                 460
   Val Asp Glu Val Phe Glu Asp Ala Phe Glu Gln Glu Tyr Thr Arg Val
   465                 470                 475                 480
   Cys Ser Leu Asn Glu His Phe Gly Asn Val Leu Thr Pro Cys Thr Val
                   485                 490                 495
   Leu Pro Val Lys Leu Tyr Ser Asp Ala Arg Asn Val Leu Ser Gly Ile
               500                 505                 510
   Ile Asp Ser His Glu Asn Leu Lys Glu Phe Lys Gly Asp Leu Ile Lys
           515                 520                 525
   Val Leu Val Trp Ile Leu Val Gln Tyr Cys Ser Lys Arg Pro Gly Met
       530                 535                 540
   Lys Glu Asn Val His Asn Thr Glu Asn Lys Gly Lys Ala Pro Leu Met
   545                 550                 555                 560
   Leu Pro Ala Leu Asn Thr Leu Pro Pro Pro Lys Ser Pro Glu Asp Ile
                   565                 570                 575
   Asp Ser Leu Asn Ser Glu Thr Phe Asn Asp Trp Ser Asp Asp Asn Ile
               580                 585                 590
   Phe Asp Asp Glu Pro Thr Ile Lys Lys Val Ile Glu Glu Lys His Gln
           595                 600                 605
   Leu Lys Asp Leu Pro Gly Thr Asn Leu Phe Ile Pro Gly Ser Val Glu
       610                 615                 620
   Ser Gln Arg Val Gly Asp His Ser Thr Gly Thr Val Pro Glu Asn Asp
   625                 630                 635                 640
   Leu Tyr Lys Ala Val Leu Leu Gly Tyr Pro Ala Val Asp Lys Gly Lys
                   645                 650                 655
   Gln Glu Asp Met Pro Tyr Ile Pro Leu Met Glu Phe Ser Cys Ser His
               660                 665                 670
   Ser His Leu Val Cys Leu Pro Ala Glu Trp Arg Thr Ser Cys Met Pro
           675                 680                 685
   Ser Ser Lys Met Lys Glu Met Ser Ser Leu Phe Pro Glu Asp Trp Tyr
       690                 695                 700
   Gln Phe Val Leu Arg Gln Leu Glu Cys Tyr His Ser Glu Glu Lys Ala
   705                 710                 715                 720
   Ser Asn Val Leu Glu Glu Ile Ala Lys Asp Lys Val Leu Lys Asp Phe
                   725                 730                 735
   Tyr Val His Thr Val Met Thr Cys Tyr Phe Ser Leu Phe Gly Ile Asp
               740                 745                 750
   Asn Met Ala Pro Ser Pro Gly His Ile Leu Arg Val Tyr Gly Gly Val
           755                 760                 765
   Leu Pro Trp Ser Val Ala Leu Asp Trp Leu Thr Glu Lys Pro Glu Leu
       770                 775                 780
   Phe Gln Leu Ala Leu Lys Ala Phe Arg Tyr Thr Leu Lys Leu Met Ile
   785                 790                 795                 800
   Asp Lys Ala Ser Leu Gly Pro Ile Glu Asp Phe Arg Glu Leu Ile Lys
                   805                 810                 815
   Tyr Leu Glu Glu Tyr Glu Arg Asp Trp Tyr Ile Gly Leu Val Ser Asp
               820                 825                 830
   Glu Lys Trp Lys Glu Ala Ile Leu Gln Glu Lys Pro Tyr Leu Phe Ser
           835                 840                 845
   Leu Gly Tyr Asp Ser Asn Met Pro Gly Pro Ala Leu Glu Ile Ser Arg
       850                 855                 860
   Val Asn Arg Asn Leu Trp Ser Gln Ile
   865                 870
<210> 32
   <211> 3927
   <212> DNA
   <213> Homo sapiens
<400> 32
   aacgacgctc ttgcgtaaag gcccggccca agggaacgtt cagggcgtct cggctttccc     60
   cgctgctgct tctgctaggc ccagtgcgag accagagcac gagcgactcc cgtcgtcccc    120
   ggccaggcag atgttggcct agtcctggcg cgaacgaagc gcgctatttc cctgcttcct    180
   ctaggccaag cctgctttac ggcagggccc gcctcgggag cgagcacaga ccggggcagc    240
   gaggccagcc aggcgccgac gaggtccccg aacgcgcacg cgctccgttc agctccgggt    300
   ggcggccgcc ggagtagacg ttagccatgg aaaccgagag ctggcccggg cggggccgcg    360
   gtgagctcgt tattcggccg ccgcagcttt tctgcctccg cattcgggca ctaaccaacc    420
   tcccggcggg agcgcccagc ccgagtttac ctgcaaaaat gcggtccctg ggatgccttc    480
   gcgtcttctc ttccctcggg tgacttgagg tttgtggtaa atatgccagc tctagaacac    540
   atgaatcaga ttttacacat cttgtttgta tttttaccct ttctgtgggc acttgggact    600
   ctgcccccac ccgatgcact tctcttatgg gcaatggagc aggttttaga gttcggcctt    660
   ggaggctcat ctatgtcaac ccacttacgg ttattagtaa tgttcatcat gtctgctgga    720
   acagctatag catcatattt cattccaagc actgttggtg tggttctttt catgactgga    780
   tttggtttct tgctgagtct gaacttaagt gatatgggtc acaaaattgg aaccaaatct    840
   aaggatttac ccagtggtcc ggaaaaacat ttttcatgga aggaatgcct tttctacatc    900
   attatattag tcttggctct tttagaaact agcttgcttc atcactttgc tggcttctca    960
   cagatttcta aaagcaattc ccaggctatt gtgggctatg gtttgatgat attacttata   1020
   atactgtgga tacttagaga aattcaaagc gtatatatca ttggaatttt ccgaaatccc   1080
   ttttatccga aggatgtgca aactgtgact gtattctttg agaagcaaac taggctcatg   1140
   aagattggta ttgtcagacg gattttgcta actttagtat caccttttgc catgatagca   1200
   tttctttcat tggacagttc cttacaaggg ctccactcag tgtctgtctg tattggattc   1260
   acaagagcct ttagaatggt atggcagaat acagaaaatg ctttattgga gacagtcatt   1320
   gtatcaacag tacacttgat ctccagtaca gacatatggt ggaacagaag cctggataca   1380
   ggactcagac tcttactggt tggtatcata cgtgatcgtt tgattcagtt catctctaaa   1440
   ttgcagtttg ccgtgactgt gcttttgaca tcatggacag agaaaaaaca acgtcgaaaa   1500
   acaactgcca ctttatgtat actcaacatt gtcttttctc cattcgtgtt ggtcatcata   1560
   gttttttcta cactactctc ttctccctta ctccctcttt tcacccttcc tgtgttcttg   1620
   gtggggtttc cccgacctat tcagagttgg ccaggagcag caggcaccac agcctgtgtg   1680
   tgtgcagata cagtgtacta ctaccaaatg gtgcccaggt tgactgctgt actgcagact   1740
   gcaatggcag ctggaagttt aggtctcctc ctacctggat ctcattactt gggccgtttt   1800
   caggatcgtt taatgtggat aatgattctg gaatgtggct atacttactg ctctattaac 1860
   attaaggggt tagaattgca ggaaacatcc tgtcatactg cagaagctcg cagagttgat 1920
   gaagtttttg aagatgcttt tgagcaagaa tacacaagag tatgttccct taatgaacac 1980
   tttggaaatg tcttgacacc ctgtactgtt ttgcctgtga aattgtattc tgatgccagg 2040
   aatgttctat caggcataat tgattctcat gaaaacttaa aagaatttaa aggtgacctc 2100
   attaaagtac ttgtgtggat acttgttcaa tactgctcca aaaggcctgg catgaaagag 2160
   aatgttcaca acactgaaaa taaagggaaa gcacctctaa tgttgcctgc tttgaacact 2220
   ttgccacctc ccaaatcccc agaagacata gacagtttaa attcagaaac ttttaatgac 2280
   tggtctgatg ataatatttt tgatgatgag ccaactatca aaaaagtaat agaagaaaaa 2340
   catcagttga aagatttgcc aggtacaaat ttgtttattc caggatcagt agaatcacag 2400
   agggttggtg atcattctac aggcactgtt cctgaaaacg atctttacaa agcagttcta 2460
   ttaggatacc ctgctgttga caaaggaaaa caagaggaca tgccatatat tcctctcatg 2520
   gagttcagtt gttcacattc tcacttagta tgcttacccg cagagtggag gactagctgt 2580
   atgcccagtt ccaaaatgaa ggagatgagc tcgttatttc cagaagactg gtaccaattt 2640
   gttctaaggc agttggaatg ttatcattca gaagagaagg cctcaaatgt actggaagaa 2700
   attgccaagg acaaagtttt aaaagacttt tatgttcata cagtaatgac ttgttatttt 2760
   agtttatttg gaatagacaa tatggctcct agtcctggtc atatattgag agtttacggt 2820
   ggtgttttgc cttggtctgt tgctttggac tggctcacag aaaagccaga actgtttcaa 2880
   ctagcactga aagcattcag gtatactctg aaactaatga ttgataaagc aagtttaggt 2940
   ccaatagaag actttagaga actgattaag taccttgaag aatatgaacg tgactggtac 3000
   attggtttgg tatctgatga aaagtggaag gaagcaattt tacaagaaaa gccatacttg 3060
   ttttctctgg ggtatgattc taatatggga atttacactg ggagagtgct tagccttcaa 3120
   gaattattga tccaagtggg aaagttaaat cctgaagctg ttagaggtca gtgggccaat 3180
   ctttcatggg aattacttta tgccacaaac gatgatgaag aacgttatag tatacaagct 3240
   catccactac ttttaagaaa tcttacggta caagcagcag aacctcccct gggatatccg 3300
   atttattctt caaaacctct ccacatacat ttgtattaga gctcattttg actgtaatgt 3360
   catcaaatgc aatgttttta ttttttcatc ctaaaaaagt aactgtgatt cttgtaactt 3420
   gaggacttct ccacaccccc attcagatgc ctgagaacag ctaagctccg taaagttggt 3480
   tctcttagcc atcttaatgg ttctaaaaaa cagcaaaaac atctttatgt ctaagataaa 3540
   agaactattt ggccaatatt tgtgccctct ggactttagt aggctttggt aaatgtgaga 3600
   aaacttttgt agaattatca tataatgaat tttgtaatgc tttcttaaat gtgttatagg 3660
   tgaattgcca tacaaagtta acagctatgt aatttttaca tacttaagag ataaacatat 3720
   cagtgttcta agtagtgata atggatcctg ttgaaggtta acataatgtg tatatatttg 3780
   tttgaaatat aatttatagt attttcaaat gtgctgattt attttgacat ctaatatctg 3840
   aatgtttttg tatcaagtag tttgttttca tagacttcaa ttcataaact ttaaaaaact 3900
   tttaataaaa tattttcctt ccttttc 3927
<210> 33
   <211> 3932
   <212> DNA
   <213> Homo sapiens
<400> 33
   aacgacgctc ttgcgtaaag gcccggccca agggaacgtt cagggcgtct cggctttccc     60
   cgctgctgct tctgctaggc ccagtgcgag accagagcac gagcgactcc cgtcgtcccc    120
   ggccaggcag atgttggcct agtcctggcg cgaacgaagc gcgctatttc cctgcttcct    180
   ctaggccaag cctgctttac ggcagggccc gcctcgggag cgagcacaga ccggggcagc    240
   gaggccagcc aggcgccgac gaggtccccg aacgcgcacg cgctccgttc agctccgggt    300
   ggcggccgcc ggagtagacg ttagccatgg aaaccgagag ctggcccggg cggggccgcg    360
   gtgagctcgt tattcggccg ccgcagcttt tctgcctccg cattcgggca ctaaccaacc    420
   tcccggcggg agcgcccagc ccgagtttac ctgcaaaaat gcggtccctg ggatgccttc    480
   gcgtcttctc ttccctcggg tgacttgagg tttgtggtaa atatgccagc tctagaacac    540
   atgaatcaga ttttacacat cttgtttgta tttttaccct ttctgtgggc acttgggact    600
   ctgcccccac ccgatgcact tctcttatgg gcaatggagc aggttttaga gttcggcctt    660
   ggaggctcat ctatgtcaac ccacttacgg ttattagtaa tgttcatcat gtctgctgga    720
   acagctatag catcatattt cattccaagc actgttggtg tggttctttt catgactgga    780
   tttggtttct tgctgagtct gaacttaagt gatatgggtc acaaaattgg aaccaaatct    840
   aaggatttac ccagtggtcc ggaaaaacat ttttcatgga aggaatgcct tttctacatc    900
   attatattag tcttggctct tttagaaact agcttgcttc atcactttgc tggcttctca    960
   cagatttcta aaagcaattc ccaggctatt gtgggctatg gtttgatgat attacttata   1020
   atactgtgga tacttagaga aattcaaagc gtatatatca ttggaatttt ccgaaatccc   1080
   ttttatccga aggatgtgca aactgtgact gtattctttg agaagcaaac taggctcatg   1140
   aagattggta ttgtcagacg gattttgcta actttagtat caccttttgc catgatagca   1200
   tttctttcat tggacagttc cttacaaggg ctccactcag tgtctgtctg tattggattc   1260
   acaagagcct ttagaatggt atggcagaat acagaaaatg ctttattgga gacagtcatt   1320
   gtatcaacag tacacttgat ctccagtaca gacatatggt ggaacagaag cctggataca 1380
   ggactcagac tcttactggt tggtatcata cgtgatcgtt tgattcagtt catctctaaa 1440
   ttgcagtttg ccgtgactgt gcttttgaca tcatggacag agaaaaaaca acgtcgaaaa 1500
   acaactgcca ctttatgtat actcaacatt gtcttttctc cattcgtgtt ggtcatcata 1560
   gttttttcta cactactctc ttctccctta ctccctcttt tcacccttcc tgtgttcttg 1620
   gtggggtttc cccgacctat tcagagttgg ccaggagcag caggcaccac agcctgtgtg 1680
   tgtgcagata cagtgtacta ctaccaaatg gtgcccaggt tgactgctgt actgcagact 1740
   gcaatggcag ctggaagttt aggtctcctc ctacctggat ctcattactt gggccgtttt 1800
   caggatcgtt taatgtggat aatgattctg gaatgtggct atacttactg ctctattaac 1860
   attaaggggt tagaattgca ggaaacatcc tgtcatactg cagaagctcg cagagttgat 1920
   gaagtttttg aagatgcttt tgagcaagaa tacacaagag tatgttccct taatgaacac 1980
   tttggaaatg tcttgacacc ctgtactgtt ttgcctgtga aattgtattc tgatgccagg 2040
   aatgttctat caggcataat tgattctcat gaaaacttaa aagaatttaa aggtgacctc 2100
   attaaagtac ttgtgtggat acttgttcaa tactgctcca aaaggcctgg catgaaagag 2160
   aatgttcaca acactgaaaa taaagggaaa gcacctctaa tgttgcctgc tttgaacact 2220
   ttgccacctc ccaaatcccc agaagacata gacagtttaa attcagaaac ttttaatgac 2280
   tggtctgatg ataatatttt tgatgatgag ccaactatca aaaaagtaat agaagaaaaa 2340
   catcagttga aagatttgcc aggtacaaat ttgtttattc caggatcagt agaatcacag 2400
   agggttggtg atcattctac aggcactgtt cctgaaaacg atctttacaa agcagttcta 2460
   ttaggatacc ctgctgttga caaaggaaaa caagaggaca tgccatatat tcctctcatg 2520
   gagttcagtt gttcacattc tcacttagta tgcttacccg cagagtggag gactagctgt 2580
   atgcccagtt ccaaaatgaa ggagatgagc tcgttatttc cagaagactg gtaccaattt 2640
   gttctaaggc agttggaatg ttatcattca gaagagaagg cctcaaatgt actggaagaa 2700
   attgccaagg acaaagtttt aaaagacttt tatgttcata cagtaatgac ttgttatttt 2760
   agtttatttg gaatagacaa tatggctcct agtcctggtc atatattgag agtttacggt 2820
   ggtgttttgc cttggtctgt tgctttggac tggctcacag aaaagccaga actgtttcaa 2880
   ctagcactga aagcattcag gtatactctg aaactaatga ttgataaagc aagtttaggt 2940
   ccaatagaag actttagaga actgattaag taccttgaag aatatgaacg tgactggtac 3000
   attggtttgg tatctgatga aaagtggaag gaagcaattt tacaagaaaa gccatacttg 3060
   ttttctctgg ggtatgattc taatatggga atttacactg ggagagtgct tagccttcaa 3120
   gaattattga tccaagtggg aaagttaaat cctgaagctg ttagaggtca gtgggccaat 3180
   ctttcatggg aattacttta tgccacaaac gatgatgaag aacgttatag tatacaagct   3240
   catccactac ttttaagaaa tcttacggta caagcagcag aacctcccct gggatatccg   3300
   atttattctt caaaacctct ccacatacat ttgtattaga gctcattttg actgtaatgt   3360
   catcaaatgc aatgttttta ttttttcatc ctaaaaaagt aactgtgatt cttgtaactt    3420
   gaggacttct ccacaccccc attcagatgc ctgagaacag ctaagctccg taaagttggt   3480
   tctcttagcc atcttaatgg ttctaaaaaa cagcaaaaac atctttatgt ctaagataaa   3540
   agaactattt ggccaatatt tgtgccctct ggactttagt aggctttggt aaatgtgaga   3600
   aaacttttgt agaattatca tataatgaat tttgtaatgc tttcttaaat gtgttatagg   3660
   tgaattgcca tacaaagtta acagctatgt aatttttaca tacttaagag ataaacatat   3720
   cagtgttcta agtagtgata atggatcctg ttgaaggtta acataatgtg tatatatttg   3780
   tttgaaatat aatttatagt attttcaaat gtgctgattt attttgacat ctaatatctg   3840
   aatgtttttg tatcaagtag tttgttttca tagacttcaa ttcataaact ttaaaaaact   3900
   tttaataaaa tattttcctt ccttttcaaa ta                                 3932
<210> 34
   <211> 4073
   <212> DNA
   <213> Homo sapiens
<400> 34
   tcttgcgtaa aggcccggcc caagggaacg ttcagggcgt ctcggctttc cccgctgctg     60
   cttctgctag gcccagtgcg agaccagagc acgagcgact cccgtcgtcc ccggccaggc    120
   agatgttggc ctagtcctgg cgcgaacgaa gcgcgctatt tccctgcttc ctctaggcca    180
   agcctgcttt acggcagggc ccgcctcggg agcgagcaca gaccggggca gcgaggccag    240
   ccaggcgccg acgaggtccc cgaacgcgca cgcgctccgt tcagctccgg gtggcggccg    300
   ccggagtaga cgttagccat ggaaaccgag agctggcccg ggcggggccg cggtgagctc    360
   gttattcggc cgccgcagct tttctgcctc cgcattcggg cactaaccaa cctcccggcg    420
   ggagcgccca gcccgagttt acctgcaaaa atgcggtccc tgggatgcct tcgcgtcttc    480
   tcttccctcg ggtgacttga gaaactgctg tgttacagaa aagcatgtga ctttcagaat    540
   aatcccgagt gaggatgagt ccagatgtgc ctctactgaa tgattacaag caggacttct    600
   ttctgaagcg ctttccacag actgttcttg gaggccctcg attcaaatta ggctattgtg    660
   cccctcctta catatatgtt aatcaaatta ttctttttct aatgccatgg gtttggggtg    720
   gagtcggaac acttttatac cagttaggca tcctgaaaga ctattataca gcagcacttt    780
   caggtggatt aatgcttttt actgcatttg tcatccagtt cacaagttta tacgccaaaa    840
   acaaatcaac aacagtagaa agaatactaa ccacggatat cttagcagag gaggatgagc    900
   atgaatttac cagttgtact ggtgctgaga ctgtcaaatt tctcattcct ggcaagaaat    960
   atgtagccaa tacagttttt cattctattc ttgctggatt agcgtgtggt cttggaacat   1020
   ggtatctgct cccaaataga ataaccttgc tgtatggcag tacaggaggc actgctctac   1080
   tattcttctt tggatggatg acactatgta tagcagaata ttctttaatt gtaaacacag   1140
   ctacagagac tgcgactttc caaacacagg atacttatga aattattcct cttatgagac   1200
   ctctttatat tttttctttt gtttctgtgg atctggcaca caggtaaaaa cctaccaaat   1260
   actttgtaac taactttgtt tttaagtata cagagtaaga gagctttcct tttagtgtta   1320
   caaaaaaatg aatccatgga ttaaaaatca tcaaaccatt gggtgacagg ttattttgat   1380
   aattattctt ttaggattaa tctctgtaaa acatactaaa gcaatagtta aaacttatta   1440
   aagagttttt ttaaaaaacc ctttttgaga taaggaactt ttcaattttg tgtttcactt   1500
   taaataagga gctttgagtt tttaagatag cctggctaaa acctgtgtaa ggagatggaa   1560
   ctttcctgtg gggggaaaga agaaattaaa atttatacat ataaatatta tatacagatt   1620
   gaatgaattt aagacaaata caaaatttat ttctaatttt atgatagcaa caatagtaga   1680
   agtaatattg attttttaaa aaccaacttg ttacagaaga aaagtagaaa atagtttttt   1740
   taacagacat aattgttcac aaaattgttt gataacccct tttacttgcc ttttcaagtt   1800
   tgacttttct ttctccgtct ccgtagattg cagctttctt ttcttaggtt agtgtccagg   1860
   tagaaatgtt cagcatgtta tggactgaat atttgtgtct ctccagaatt catatattac   1920
   agtcttaaac cccaatgtga tggtattttg agatgaggcc tttgggaggt aattaggtca   1980
   tgaaggtggg tccttggtat gatgggacta gtccccttat gagaagaggt accagagagc   2040
   ttgatttgtc cctctctgtg ccatctaagg acacagcaaa aatgtggcca cctgcaagtc   2100
   aaagaagaga gctctcatca aaacctgacc atactggcag cctgatcatg gtctttcggc   2160
   cttcagaagt gtgagaaagt aaattgatgt tgtttaagcc actcagctta tggcattttt   2220
   ttatggcagc ccatgctgat taagattttg ctaccaagaa gtgggatgcc tttgtaccaa   2280
   atacctaaaa atgtggaaat ggctttgtaa ctgttggtaa tgggtagagg ctgaaagagg   2340
   tttttttgtt tgtttctttg ttttgttttg tttgagatgg agtcttcact ctcttgccca   2400
   ggctggagtg cagtggcaca atcttggccc actgcaacgt ccgcctcctg gcttcaagtg   2460
   atactcctcc ctgagcctcc tgagtagctg gaattacaga catgcgccac catgcccggc   2520
   taatttttat atttttaata gagacagggt ttcgccatgt tggccaggct ggtcccaaag   2580
   tcctgacctc aagtgatcca cccgcctcgg cctcccaaag ttctgggatt acaggcttga   2640
   gccatcacac ccagccaagg gttttgatgt gcatgctaga aatatggaca ttaagggtga   2700
   ttctgatgaa gtctgaagtc ccctgaaccc agaggaaagg cagtccttgt tacaaagcgt   2760
   caaagaactt agctgaactg tgtcctagtg ttttgtggag ccatgaagtt ggatacctac 2820
   gtaaggagag ttcaaagcag tgttgaaagg agcagttggc ttctcctgtt tctagtaaaa 2880
   tgtgaaagga gagagatgga ttgaagaagg gattgttaag caagagaagg aaccagaact 2940
   tgaagatttg gaaaattttc agcctggatt atccatattg taaaacatga gaaagcatat 3000
   tctgaagaga acaccaaaag tgtggggctg gactgtccct cagtaaagag cttttggcat 3060
   tatgtgagta gaaacactgc cagtttgaat tgaagtggtt ggagacagga agtaatgaag 3120
   gccgacagtt gaacttcttg gatttgacag gatgtaatga tagaactgtt cagctacaaa 3180
   agtgcagtat tcttcaagaa aaggggaaaa ttatgccaaa ggtgatttaa gggtcttgag 3240
   ggctaccacc tgtttcaaca agtcagctag cctctaccca aagcctcggg agcagaactg 3300
   aacttcagag ccacagaagc aggaccctca cctagagcac tggcggtgac ctgccacccc 3360
   agtggcctgg tgggcagagt atggaaccaa acagaattat tctcaagctc taagatctaa 3420
   tggaatttgc cttgctaggt tttgacttcc ttgggatcat cacccttttt ttcctgtttc 3480
   tcctattgga gtggggatgt ctcttctata cctgccctac ccttagattt tggaagcacg 3540
   taactcatct ggtttcacag attcacagct ggagaggaat tttgcctcag gatggattgt 3600
   acctaggtct catccatatc tgatttagat gagactttga attttagcca tcagagttaa 3660
   tgctagaatg agtgaagact ttgggggata tggggatgga atgaatgtct tttgcatttg 3720
   aatttggaaa ggacatgagt tttgaggggc cagggatgga atgttataga ctaattgtgt 3780
   tccctcaaaa atgtttatgt tgaagcccta acccccaatg tgttggtatt tggagatggg 3840
   cctctgggag gtagtttatg aaggtgagac cctagtctga taggattagt gcccttagga 3900
   gagatgccaa agagcttgat gtctctcttt ttgctacaaa aagacacagc aaaaaggcag 3960
   ccatgtgtaa gccaggaaga gagtcttcac cagaacctga ctatactggc agcctgatct 4020
   tgtacttgta gcccccagaa ctgttagaaa ataaatttct gttgtttaag cca 4073
<210> 35
   <211> 4666
   <212> DNA
   <213> Homo sapiens
<400> 35
   tcttgcgtaa aggcccggcc caagggaacg ttcagggcgt ctcggctttc cccgctgctg      60
   cttctgctag gcccagtgcg agaccagagc acgagcgact cccgtcgtcc ccggccaggc     120
   agatgttggc ctagtcctgg cgcgaacgaa gcgcgctatt tccctgcttc ctctaggcca     180
   agcctgcttt acggcagggc ccgcctcggg agcgagcaca gaccggggca gcgaggccag     240
   ccaggcgccg acgaggtccc cgaacgcgca cgcgctccgt tcagctccgg gtggcggccg     300
   ccggagtaga cgttagccat ggaaaccgag agctggcccg ggcggggccg cggtgagctc     360
   gttattcggc cgccgcagct tttctgcctc cgcattcggg cactaaccaa cctcccggcg     420
   ggagcgccca gcccgagttt acctgcaaaa atgcggtccc tgggatgcct tcgcgtcttc     480
   tcttccctcg ggtgacttga gaaactgctg tgttacagaa aagcatgtga ctttcagaat     540
   aatcccgagt gaggatgagt ccagatgtgc ctctactgaa tgattacaag caggacttct     600
   ttctgaagcg ctttccacag actgttcttg gaggccctcg attcaaatta ggctattgtg     660
   cccctcctta catatatgtt aatcaaatta ttctttttct aatgccatgg gtttggggtg     720
   gagtcggaac acttttatac cagttaggca tcctgaaaga ctattataca gcagcacttt     780
   caggtggatt aatgcttttt actgcatttg tcatccagtt cacaagttta tacgccaaaa     840
   acaaatcaac aacagtagaa agaatactaa ccacggatat cttagcagag gaggatgagc     900
   atgaatttac cagttgtact ggtgctgaga ctgtcaaatt tctcattcct ggcaagaaat     960
   atgtagccaa tacagttttt cattctattc ttgctggatt agcgtgtggt cttggaacat    1020
   ggtatctgct cccaaataga ataaccttgc tgtatggcag tacaggaggc actgctctac    1080
   tattcttctt tggatggatg acactatgta tagcagaata ttctttaatt gtaaacacag    1140
   ctacagagac tgcgactttc caaacacagg atacttatga aattattcct cttatgagac    1200
   ctctttatat ttttttcttt gtttctgtgg atctggcaca caggtttgtg gtaaatatgc    1260
   cagctctaga acacatgaat cagattttac acatcttgtt tgtattttta ccctttctgt    1320
   gggcacttgg gactctgccc ccacccgatg cacttctctt atgggcaatg gagcaggttt    1380
   tagagttcgg ccttggaggc tcatctatgt caacccactt acggttatta gtaatgttca    1440
   tcatgtctgc tggaacagct atagcatcat atttcattcc aagcactgtt ggtgtggttc    1500
   ttttcatgac tggatttggt ttcttgctga gtctgaactt aagtgatatg ggtcacaaaa    1560
   ttggaaccaa atctaaggat ttacccagtg gtccggaaaa acatttttca tggaaggaat    1620
   gccttttcta catcattata ttagtcttgg ctcttttaga aactagcttg cttcatcact    1680
   ttgctggctt ctcacagatt tctaaaagca attcccaggc tattgtgggc tatggtttga    1740
   tgatattact tataatactg tggatactta gagaaattca aagcgtatat atcattggaa    1800
   ttttccgaaa tcccttttat ccgaaggatg tgcaaactgt gactgtattc tttgagaagc    1860
   aaactaggct catgaagatt ggtattgtca gacggatttt gctaacttta gtatcacctt    1920
   ttgccatgat agcatttctt tcattggaca gttccttaca agggctccac tcagtgtctg    1980
   tctgtattgg attcacaaga gcctttagaa tggtatggca gaatacagaa aatgctttat    2040
   tggagacagt cattgtatca acagtacact tgatctccag tacagacata tggtggaaca    2100
   gaagcctgga tacaggactc agactcttac tggttggtat catacgtgat cgtttgattc    2160
   agttcatctc taaattgcag tttgccgtga ctgtgctttt gacatcatgg acagagaaaa 2220
   aacaacgtcg aaaaacaact gccactttat gtatactcaa cattgtcttt tctccattcg 2280
   tgttggtcat catagttttt tctacactac tctcttctcc cttactccct cttttcaccc 2340
   ttcctgtgtt cttggtgggg tttccccgac ctattcagag ttggccagga gcagcaggca 2400
   ccacagcctg tgtgtgtgca gatacagtgt actactacca aatggtgccc aggttgactg 2460
   ctgtactgca gactgcaatg gcagctggaa gtttaggtct cctcctacct ggatctcatt 2520
   acttgggccg ttttcaggat cgtttaatgt ggataatgat tctggaatgt ggctatactt 2580
   actgctctat taacattaag gggttagaat tgcaggaaac atcctgtcat actgcagaag 2640
   ctcgcagagt tgatgaagtt tttgaagatg cttttgagca agaatacaca agagtatgtt 2700
   cccttaatga acactttgga aatgtcttga caccctgtac tgttttgcct gtgaaattgt 2760
   attctgatgc caggaatgtt ctatcaggca taattgattc tcatgaaaac ttaaaagaat 2820
   ttaaaggtga cctcattaaa gtacttgtgt ggatacttgt tcaatactgc tccaaaaggc 2880
   ctggcatgaa agagaatgtt cacaacactg aaaataaagg gaaagcacct ctaatgttgc 2940
   ctgctttgaa cactttgcca cctcccaaat ccccagaaga catagacagt ttaaattcag 3000
   aaacttttaa tgactggtct gatgataata tttttgatga tgagccaact atcaaaaaag 3060
   taatagaaga aaaacatcag ttgaaagatt tgccaggtac aaatttgttt attccaggat 3120
   cagtagaatc acagagggtt ggtgatcatt ctacaggcac tgttcctgaa aacgatcttt 3180
   acaaagcagt tctattagga taccctgctg ttgacaaagg aaaacaagag gacatgccat 3240
   atattcctct catggagttc agttgttcac attctcactt agtatgctta cccgcagagt 3300
   ggaggactag ctgtatgccc agttccaaaa tgaaggagat gagctcgtta tttccagaag 3360
   actggtacca atttgttcta aggcagttgg aatgttatca ttcagaagag aaggcctcaa 3420
   atgtactgga agaaattgcc aaggacaaag ttttaaaaga cttttatgtt catacagtaa 3480
   tgacttgtta ttttagttta tttggaatag acaatatggc tcctagtcct ggtcatatat 3540
   tgagagttta cggtggtgtt ttgccttggt ctgttgcttt ggactggctc acagaaaagc 3600
   cagaactgtt tcaactagca ctgaaagcat tcaggtatac tctgaaacta atgattgata 3660
   aagcaagttt aggtccaata gaagacttta gagaactgat taagtacctt gaagaatatg 3720
   aacgtgactg gtacattggt ttggtatctg atgaaaagtg gaaggaagca attttacaag 3780
   aaaagccata cttgttttct ctggggtatg attctaatat gggaatttac actgggagag 3840
   tgcttagcct tcaagaatta ttgatccaag tgggaaagtt aaatcctgaa gctgttagag 3900
   gtcagtgggc caatctttca tgggaattac tttatgccac aaacgatgat gaagaacgtt 3960
   atagtataca agctcatcca ctacttttaa gaaatcttac ggtacaagca gcagaacctc 4020
   ccctgggata tccgatttat tcttcaaaac ctctccacat acatttgtat tagagctcat   4080
   tttgactgta atgtcatcaa atgcaatgtt tttatttttt catcctaaaa aagtaactgt   4140
   gattcttgta acttgaggac ttctccacac ccccattcag atgcctgaga acagctaagc   4200
   tccgtaaagt tggttctctt agccatctta atggttctaa aaaacagcaa aaacatcttt   4260
   atgtctaaga taaaagaact atttggccaa tatttgtgcc ctctggactt tagtaggctt   4320
   tggtaaatgt gagaaaactt ttgtagaatt atcatataat gaattttgta atgctttctt   4380
   aaatgtgtta taggtgaatt gccatacaaa gttaacagct atgtaatttt tacatactta   4440
   agagataaac atatcagtgt tctaagtagt gataatggat cctgttgaag gttaacataa   4500
   tgtgtatata tttgtttgaa atataattta tagtattttc aaatgtgctg atttattttg   4560
   acatctaata tctgaatgtt tttgtatcaa gtagtttgtt ttcatagact tcaattcata   4620
   aactttaaaa aacttttaat aaaatatttt ccttcctttt caaata                  4666
<210> 36
   <211> 3513
   <212> DNA
   <213> Homo sapiens
<400> 36
   aaaggcccgg cccaagggaa cgttcagggc gtctcggctt tccccgctgc tgcttctgct     60
   aggcccagtg cgagaccaga gcacgagcga ctcccgtcgt ccccggccag gcagatgttg    120
   gcctagtcct ggcgcgaacg aagcgcgcta tttccctgct tcctctaggc caagcctgct    180
   ttacggcagg gcccgcctcg ggagcgagca cagaccgggg cagcgaggcc agccaggcgc    240
   cgacgaggtc cccgaacgcg cacgcgctcc gttcagctcc gggtggcggc cgccggagta    300
   gacgttagcc atggaaaccg agagctggcc cgggcggggc cgcggtgagc tcgttattcg    360
   gccgccgcag cttttctgcc tccgcattcg ggcactaacc aacctcccgg cgggagcgcc    420
   cagcccgagt ttacctgcaa aaatgcggtc cctgggatgc cttcgcgtct tctcttccct    480
   cgggtgactt gaggtttgtg gtaaatatgc cagctctaga acacatgaat cagattttac    540
   acatcttgtt tgtattttta ccctttctgt gggcacttgg gactctgccc ccacccgatg    600
   cacttctctt atgggcaatg gagcaggttt tagagttcgg ccttggaggc tcatctatgt    660
   caacccactt acggttatta gtaatgttca tcatgtctgc tggaacagct atagcatcat    720
   atttcattcc aagcactgtt ggtgtggttc ttttcatgac tggatttggt ttcttgctga    780
   gtctgaactt aagtgatatg ggtcacaaaa ttggaaccaa atctaaggat ttacccagtg    840
   gtccggaaaa acatttttca tggaaggaat gccttttcta catcattata ttagtcttgg    900
   ctcttttaga aactagcttg cttcatcact ttgctggctt ctcacagatt tctaaaagca    960
   attcccaggc tattgtgggc tatggtttga tgatattact tataatactg tggatactta   1020
   gagaaattca aagcgtatat atcattggaa ttttccgaaa tcccttttat ccgaaggatg   1080
   tgcaaactgt gactgtattc tttgagaagc aaactaggct catgaagatt ggtattgtca   1140
   gacggatttt gctaacttta gtatcacctt ttgccatgat agcatttctt tcattggaca   1200
   gttccttaca agggctccac tcagtgtctg tctgtattgg attcacaaga gcctttagaa   1260
   tggtatggca gaatacagaa aatgctttat tggagacagt cattgtatca acagtacact   1320
   tgatctccag tacagacata tggtggaaca gaagcctgga tacaggactc agactcttac   1380
   tggttggtat catacgtgat cgtttgattc agttcatctc taaattgcag tttgccgtga   1440
   ctgtgctttt gacatcatgg acagagaaaa aacaacgtcg aaaaacaact gccactttat   1500
   gtatactcaa cattgtcttt tctccattcg tgttggtcat catagttttt tctacactac   1560
   tctcttctcc cttactccct cttttcaccc ttcctgtgtt cttggtgggg tttccccgac   1620
   ctattcagag ttggccagga gcagcaggca ccacagcctg tgtgtgtgca gatacagtgt   1680
   actactacca aatggtgccc aggttgactg ctgtactgca gactgcaatg gcagctggaa   1740
   gtttaggtct cctcctacct ggatctcatt acttgggccg ttttcaggat cgtttaatgt   1800
   ggataatgat tctggaatgt ggctatactt actgctctat taacattaag gggttagaat    1860
   tgcaggaaac atcctgtcat actgcagaag ctcgcagagt tgatgaagtt tttgaagatg   1920
   cttttgagca agaatacaca agagtatgtt cccttaatga acactttgga aatgtcttga   1980
   caccctgtac tgttttgcct gtgaaattgt attctgatgc caggaatgtt ctatcaggca   2040
   taattgattc tcatgaaaac ttaaaagaat ttaaaggtga cctcattaaa gtacttgtgt   2100
   ggatacttgt tcaatactgc tccaaaaggc ctggcatgaa agagaatgtt cacaacactg   2160
   aaaataaagg gaaagcacct ctaatgttgc ctgctttgaa cactttgcca cctcccaaat   2220
   ccccagaaga catagacagt ttaaattcag aaacttttaa tgactggtct gatgataata   2280
   tttttgatga tgagccaact atcaaaaaag taatagaaga aaaacatcag ttgaaagatt   2340
   tgccaggtac aaatttgttt attccaggat cagtagaatc acagagggtt ggtgatcatt   2400
   ctacaggcac tgttcctgaa aacgatcttt acaaagcagt tctattagga taccctgctg   2460
   ttgacaaagg aaaacaagag gacatgccat atattcctct catggagttc agttgttcac   2520
   attctcactt agtatgctta cccgcagagt ggaggactag ctgtatgccc agttccaaaa   2580
   tgaaggagat gagctcgtta tttccagaag actggtacca atttgttcta aggcagttgg   2640
   aatgttatca ttcagaagag aaggcctcaa atgtactgga agaaattgcc aaggacaaag   2700
   ttttaaaaga cttttatgtt catacagtaa tgacttgtta ttttagttta tttggaatag   2760
   acaatatggc tcctagtcct ggtcatatat tgagagttta cggtggtgtt ttgccttggt   2820
   ctgttgcttt ggactggctc acagaaaagc cagaactgtt tcaactagca ctgaaagcat   2880
   tcaggtatac tctgaaacta atgattgata aagcaagttt aggtccaata gaagacttta   2940
   gagaactgat taagtacctt gaagaatatg aacgtgactg gtacattggt ttggtatctg   3000
   atgaaaagtg gaaggaagca attttacaag aaaagccata cttgttttct ctggggtatg   3060
   attctaatat gccaggacca gccttggaga taagcagagt gaacagaaat ttatggtctc   3120
   agatttaaga aaaacaaaat tctttcttgc ttcttaaatc atactccatc ccattggctt   3180
   gcaaacatgc tgacactcct cataatttca ctcttcataa accaaagcat aaggtcagag   3240
   gagaacttga catattagaa cacttaggca ttgaaagtgg ttagtctaac taacccattg   3300
   aagttttgga gaacctggga ctcaaatttt ggaagatgtg acagatgata tgttaacata   3360
   cattgcaccg aggctgaagt gggaggattg cttgagactg cctggaaggc agaagttgca   3420
   gtgagccgag actgatggtg tcactgcact ccagcctggg caacagagca agaccctgtc   3480
   ttaaaaaaac aaaacaaaca aacaaaagaa acc                                3513
<210> 37
   <211> 406
   <212> PRT
   <213> Homo sapiens
<400> 37
   Met Asp Cys Arg Thr Lys Ala Asn Pro Asp Arg Thr Phe Asp Leu Val
   1               5                   10                  15
   Leu Lys Val Lys Cys His Ala Ser Glu Asn Glu Asp Pro Val Val Leu
               20                  25                  30
   Trp Lys Phe Pro Glu Asp Phe Gly Asp Gln Glu Ile Leu Gln Ser Val
           35                  40                  45
   Pro Lys Phe Cys Phe Pro Phe Asp Val Glu Arg Val Ser Gln Asn Gln
       50                  55                  60
   Val Gly Gln His Phe Thr Phe Val Leu Thr Asp Ile Glu Ser Lys Gln
   65                  70                  75                  80
   Arg Phe Gly Phe Cys Arg Leu Thr Ser Gly Gly Thr Ile Cys Leu Cys
                   85                  90                  95
   Ile Leu Ser Tyr Leu Pro Trp Phe Glu Val Tyr Tyr Lys Leu Leu Asn
               100                 105                 110
   Thr Leu Ala Asp Tyr Leu Ala Lys Glu Leu Glu Asn Asp Leu Asn Glu
           115                 120                 125
   Thr Leu Arg Ser Leu Tyr Asn His Pro Val Pro Lys Ala Asn Thr Pro
       130                 135                 140
   Val Asn Leu Ser Val His Ser Tyr Phe Ile Ala Pro Asp Val Thr Gly
   145                 150                 155                 160
   Leu Pro Thr Ile Pro Glu Ser Arg Asn Leu Thr Glu Tyr Phe Val Ala
                   165                 170                 175
   Val Asp Val Asn Asn Met Leu Gln Leu Tyr Ala Ser Met Leu His Glu
               180                 185                 190
   Arg Arg Ile Val Ile Ile Ser Ser Lys Leu Ser Thr Leu Thr Ala Cys
           195                 200                 205
   Ile His Gly Ser Ala Ala Leu Leu Tyr Pro Met Tyr Trp Gln His Ile
       210                 215                 220
   Tyr Ile Pro Val Leu Pro Pro His Leu Leu Asp Tyr Cys Cys Ala Pro
   225                 230                 235                 240
   Met Pro Tyr Leu Ile Gly Ile His Ser Ser Leu Ile Glu Arg Val Lys
                   245                 250                 255
   Asn Lys Ser Leu Glu Asp Val Val Met Leu Asn Val Asp Thr Asn Thr
               260                 265                 270
   Leu Glu Ser Pro Phe Ser Asp Leu Asn Asn Leu Pro Ser Asp Val Val
           275                 280                 285
   Ser Ala Leu Lys Asn Lys Leu Lys Lys Gln Ser Thr Ala Thr Gly Asp
       290                 295                 300
   Gly Val Ala Arg Ala Phe Leu Arg Ala Gln Ala Ala Leu Phe Gly Ser
   305                 310                 315                 320
   Tyr Arg Asp Ala Leu Arg Tyr Lys Pro Gly Glu Pro Ile Thr Phe Cys
                   325                 330                 335
   Glu Glu Ser Phe Val Lys His Arg Ser Ser Val Met Lys Gln Phe Leu
               340                 345                 350
   Glu Thr Ala Ile Asn Leu Gln Leu Phe Lys Gln Phe Ile Asp Gly Arg
           355                 360                 365
   Leu Ala Lys Leu Asn Ala Gly Arg Gly Phe Ser Asp Val Phe Glu Glu
       370                 375                 380
   Glu Ile Thr Ser Gly Gly Phe Cys Gly Gly Lys Asp Lys Leu Gln Tyr
   385                 390                 395                 400
   Asp Tyr Pro Phe Ser Gln
                   405
<210> 38
   <211> 426
   <212> PRT
   <213> Homo sapiens
<400> 38
   Met Asp Cys Arg Thr Lys Ala Asn Pro Asp Arg Thr Phe Asp Leu Val
   1               5                   10                  15
   Leu Lys Val Lys Cys His Ala Ser Glu Asn Glu Asp Pro Val Val Leu
               20                  25                  30
   Trp Lys Phe Pro Glu Asp Phe Gly Asp Gln Glu Ile Leu Gln Ser Val
           35                  40                  45
   Pro Lys Phe Cys Phe Pro Phe Asp Val Glu Arg Val Ser Gln Asn Gln
       50                  55                  60
   Val Gly Gln His Phe Thr Phe Val Leu Thr Asp Ile Glu Ser Lys Gln
   65                  70                  75                  80
   Arg Phe Gly Phe Cys Arg Leu Thr Ser Gly Gly Thr Ile Cys Leu Cys
                   85                  90                  95
   Ile Leu Ser Tyr Leu Pro Trp Phe Glu Val Tyr Tyr Lys Leu Leu Asn
               100                 105                 110
   Thr Leu Ala Asp Tyr Leu Ala Lys Glu Leu Glu Asn Asp Leu Asn Glu
           115                 120                 125
   Thr Leu Arg Ser Leu Tyr Asn His Pro Val Pro Lys Ala Asn Thr Pro
       130                 135                 140
   Val Asn Leu Ser Val Asn Gln Glu Ile Phe Ile Ala Cys Glu Gln Val
   145                 150                 155                 160
   Leu Lys Asp Gln Pro Ala Leu Val Pro His Ser Tyr Phe Ile Ala Pro
                   165                 170                 175
   Asp Val Thr Gly Leu Pro Thr Ile Pro Glu Ser Arg Asn Leu Thr Glu
               180                 185                 190
   Tyr Phe Val Ala Val Asp Val Asn Asn Met Leu Gln Leu Tyr Ala Ser
           195                 200                 205
   Met Leu His Glu Arg Arg Ile Val Ile Ile Ser Ser Lys Leu Ser Thr
       210                 215                 220
   Leu Thr Ala Cys Ile His Gly Ser Ala Ala Leu Leu Tyr Pro Met Tyr
   225                 230                 235                 240
   Trp Gln His Ile Tyr Ile Pro Val Leu Pro Pro His Leu Leu Asp Tyr
                   245                 250                 255
   Cys Cys Ala Pro Met Pro Tyr Leu Ile Gly Ile His Ser Ser Leu Ile
               260                 265                 270
   Glu Arg Val Lys Asn Lys Ser Leu Glu Asp Val Val Met Leu Asn Val
           275                 280                 285
   Asp Thr Asn Thr Leu Glu Ser Pro Phe Ser Asp Leu Asn Asn Leu Pro
       290                 295                 300
   Ser Asp Val Val Ser Ala Leu Lys Asn Lys Leu Lys Lys Gln Ser Thr
   305                 310                 315                 320
   Ala Thr Gly Asp Gly Val Ala Arg Ala Phe Leu Arg Ala Gln Ala Ala
                   325                 330                 335
   Leu Phe Gly Ser Tyr Arg Asp Ala Leu Arg Tyr Lys Pro Gly Glu Pro
               340                 345                 350
   Ile Thr Phe Cys Glu Glu Ser Phe Val Lys His Arg Ser Ser Val Met
           355                 360                 365
   Lys Gln Phe Leu Glu Thr Ala Ile Asn Leu Gln Leu Phe Lys Gln Phe
       370                 375                 380
   Ile Asp Gly Arg Leu Ala Lys Leu Asn Ala Gly Arg Gly Phe Ser Asp
   385                 390                 395                 400
   Val Phe Glu Glu Glu Ile Thr Ser Gly Gly Phe Cys Gly Gly Lys Asp
                   405                 410                 415
   Lys Leu Gln Tyr Asp Tyr Pro Phe Ser Gln
               420                 425
<210> 39
   <211> 426
   <212> PRT
   <213> Homo sapiens
<400> 39
   Ile Glu Thr Lys Thr Arg Ala Asn Pro Asp Arg Thr Phe Asp Leu Val
   1               5                   10                  15
   Leu Lys Val Lys Cys His Ala Ser Glu Asn Glu Asp Pro Val Val Leu
               20                  25                  30
   Trp Lys Phe Pro Glu Asp Phe Gly Asp Gln Glu Ile Leu Gln Ser Val
           35                  40                  45
   Pro Lys Phe Cys Phe Pro Phe Asp Val Glu Arg Val Ser Gln Asn Gln
       50                  55                  60
   Val Gly Gln His Phe Thr Phe Val Leu Thr Asp Ile Glu Ser Lys Gln
   65                  70                  75                  80
   Arg Phe Gly Phe Cys Arg Leu Thr Ser Gly Gly Thr Ile Cys Leu Cys
                   85                  90                  95
   Ile Leu Ser Tyr Leu Pro Trp Phe Glu Val Tyr Tyr Lys Leu Leu Asn
               100                 105                 110
   Thr Leu Ala Asp Tyr Leu Ala Lys Glu Leu Glu Asn Asp Leu Asn Glu
           115                 120                 125
   Thr Leu Arg Ser Leu Tyr Asn His Pro Val Pro Lys Ala Asn Thr Pro
       130                 135                 140
   Val Asn Leu Ser Val Asn Gln Glu Ile Phe Ile Ala Cys Glu Gln Val
   145                 150                 155                 160
   Leu Lys Asp Gln Pro Ala Leu Val Pro His Ser Tyr Phe Ile Ala Pro
                   165                 170                 175
   Asp Val Thr Gly Leu Pro Thr Ile Pro Glu Ser Arg Asn Leu Thr Glu
               180                 185                 190
   Tyr Phe Val Ala Val Asp Val Asn Asn Met Leu Gln Leu Tyr Ala Ser
           195                 200                 205
   Met Leu His Glu Arg Arg Ile Val Ile Ile Ser Ser Lys Leu Ser Thr
       210                 215                 220
   Leu Thr Ala Cys Ile His Gly Ser Ala Ala Leu Leu Tyr Pro Met Tyr
   225                 230                 235                 240
   Trp Gln His Ile Tyr Ile Pro Val Leu Pro Pro His Leu Leu Asp Tyr
                   245                 250                 255
   Cys Cys Ala Pro Met Pro Tyr Leu Ile Gly Ile His Ser Ser Leu Ile
               260                 265                 270
   Glu Arg Val Lys Asn Lys Ser Leu Glu Asp Val Val Met Leu Asn Val
           275                 280                 285
   Asp Thr Asn Thr Leu Glu Ser Pro Phe Ser Asp Leu Asn Asn Leu Pro
       290                 295                 300
   Ser Asp Val Val Ser Ala Leu Lys Asn Lys Leu Lys Lys Gln Ser Thr
   305                 310                 315                 320
   Ala Thr Gly Asp Gly Val Ala Arg Ala Phe Leu Arg Ala Gln Ala Ala
                   325                 330                 335
   Leu Phe Gly Ser Tyr Arg Asp Ala Leu Arg Tyr Lys Pro Gly Glu Pro
               340                 345                 350
   Ile Thr Phe Cys Glu Glu Ser Phe Val Lys His Arg Ser Ser Val Met
           355                 360                 365
   Lys Gln Phe Leu Glu Thr Ala Ile Asn Leu Gln Leu Phe Lys Gln Phe
       370                 375                 380
   Ile Asp Gly Arg Leu Ala Lys Leu Asn Ala Gly Arg Gly Phe Ser Asp
   385                 390                 395                 400
   Val Phe Glu Glu Glu Ile Thr Ser Gly Gly Phe Cys Gly Gly Lys Asp
                   405                 410                 415
   Lys Leu Gln Tyr Asp Tyr Pro Phe Ser Gln
               420                 425
<210> 40
   <211> 396
   <212> PRT
   <213> Homo sapiens
<400> 40
   Met Ala Ala Ala Pro Arg Glu Glu Lys Arg Trp Pro Gln Pro Val Phe
   1               5                   10                  15
   Ser Asn Pro Val Val Leu Trp Lys Phe Pro Glu Asp Phe Gly Asp Gln
               20                  25                  30
   Glu Ile Leu Gln Ser Val Pro Lys Phe Cys Phe Pro Phe Asp Val Glu
           35                  40                  45
   Arg Val Ser Gln Asn Gln Val Gly Gln His Phe Thr Phe Val Leu Thr
       50                  55                  60
   Asp Ile Glu Ser Lys Gln Arg Phe Gly Phe Cys Arg Leu Thr Ser Gly
   65                  70                  75                  80
   Gly Thr Ile Cys Leu Cys Ile Leu Ser Tyr Leu Pro Trp Phe Glu Val
                   85                  90                  95
   Tyr Tyr Lys Leu Leu Asn Thr Leu Ala Asp Tyr Leu Ala Lys Glu Leu
               100                 105                 110
   Glu Asn Asp Leu Asn Glu Thr Leu Arg Ser Leu Tyr Asn His Pro Val
           115                 120                 125
   Pro Lys Ala Asn Thr Pro Val Asn Leu Ser Val His Ser Tyr Phe Ile
       130                 135                 140
   Ala Pro Asp Val Thr Gly Leu Pro Thr Ile Pro Glu Ser Arg Asn Leu
   145                 150                 155                 160
   Thr Glu Tyr Phe Val Ala Val Asp Val Asn Asn Met Leu Gln Leu Tyr
                   165                 170                 175
   Ala Ser Met Leu His Glu Arg Arg Ile Val Ile Ile Ser Ser Lys Leu
               180                 185                 190
   Ser Thr Leu Thr Ala Cys Ile His Gly Ser Ala Ala Leu Leu Tyr Pro
           195                 200                 205
   Met Tyr Trp Gln His Ile Tyr Ile Pro Val Leu Pro Pro His Leu Leu
       210                 215                 220
   Asp Tyr Cys Cys Ala Pro Met Pro Tyr Leu Ile Gly Ile His Ser Ser
   225                 230                 235                 240
   Leu Ile Glu Arg Val Lys Asn Lys Ser Leu Glu Asp Val Val Met Leu
                   245                 250                 255
   Asn Val Asp Thr Asn Thr Leu Glu Ser Pro Phe Ser Asp Leu Asn Asn
               260                 265                 270
   Leu Pro Ser Asp Val Val Ser Ala Leu Lys Asn Lys Leu Lys Lys Gln
           275                 280                 285
   Ser Thr Ala Thr Gly Asp Gly Val Ala Arg Ala Phe Leu Arg Ala Gln
       290                 295                 300
   Ala Ala Leu Phe Gly Ser Tyr Arg Asp Ala Leu Arg Tyr Lys Pro Gly
   305                 310                 315                 320
   Glu Pro Ile Thr Phe Cys Glu Glu Ser Phe Val Lys His Arg Ser Ser
                   325                 330                 335
   Val Met Lys Gln Phe Leu Glu Thr Ala Ile Asn Leu Gln Leu Phe Lys
               340                 345                 350
   Gln Phe Ile Asp Gly Arg Leu Ala Lys Leu Asn Ala Gly Arg Gly Phe
           355                 360                 365
   Ser Asp Val Phe Glu Glu Glu Ile Thr Ser Gly Gly Phe Cys Gly Gly
       370                 375                 380
   Lys Asp Lys Leu Gln Tyr Asp Tyr Pro Phe Ser Gln
   385                 390                 395
<210> 41
   <211> 2117
   <212> DNA
   <213> Homo sapiens
<400> 41
   gccgggggcg cagccgacat gggcccgccg ccacggctgc tgtgagcagc ctctttccct      60
   gtgtggccgc cggcgtgggc ggggacggcg cgaccctcgc gcggccgggc tgcgggcttc     120
   caggccagcg cgcgggggcc ggacggacag ccccacaccg acatgtaacc atggactgca     180
   ggaccaaggc aaatccagac agaacctttg acttggtgtt gaaagtgaaa tgtcatgcct     240
   ctgaaaatga agatcctgtg gtattgtgga aattcccaga ggactttgga gaccaggaaa     300
   tactacagag tgtgccaaag ttctgttttc cctttgacgt tgaaagggtg tctcagaatc     360
   aagttggaca gcactttacc tttgtactga cagacattga aagtaaacag agatttggat     420
   tctgcagact gacgtcagga ggcacaattt gtttatgcat ccttagttac cttccctggt     480
   ttgaagtgta ttacaagctt ctaaatactc ttgcagatta cttggctaag gaactggaaa     540
   atgatttgaa tgaaactctc agatcactgt ataaccaccc agtaccaaag gcaaatactc    600
   ctgtaaattt gagtgtgcat tcctacttca ttgcccctga tgtaactgga ctcccaacaa    660
   tacccgagag tagaaatctt acagaatatt ttgttgccgt ggatgtgaac aacatgctgc    720
   agctgtatgc cagtatgctg catgaaaggc gcatcgtgat tatctcgagc aaattaagca    780
   ctttaactgc ctgtatccat ggatcagctg ctcttctata cccaatgtat tggcaacaca    840
   tatacatccc agtgcttcct ccacacctgc tggactactg ctgtgcccca atgccatacc    900
   tgattggaat acactccagc ctcatagaga gagtgaaaaa caaatcattg gaagatgttg    960
   ttatgttaaa tgttgataca aacacattag aatcaccatt tagtgacttg aacaacctac   1020
   caagtgatgt ggtctcggcc ttgaaaaata aactgaagaa gcagtctaca gctacgggtg   1080
   atggagtagc tagggccttt cttagagcac aggctgcttt gtttggatcc tacagagatg   1140
   cactgagata caaacctggt gagcccatca ctttctgtga ggagagtttt gtaaagcacc   1200
   gctcaagcgt gatgaaacag ttcctggaaa ctgccattaa cctccagctt tttaagcagt   1260
   ttatcgatgg tcgactggca aaactaaatg caggaagggg tttctctgat gtatttgaag   1320
   aagagatcac ttcaggtggc ttttgtggag gtaaagacaa gttacaatat gattatccat   1380
   tttctcaata acaattttct tggtctttgc acttgtgtct gataaaacct atttcataaa   1440
   caactaatga tttcctccta aatatgtaat gtcttaaata catttttcat cttataaaag   1500
   ctatggaatt agcttatttt gcctgatacc tgttactcaa ggcattaagt tggcctcctg   1560
   aattggcagc tgttggcctc gataatctct taatattgct ggaaattagt aatacagaaa   1620
   tccaatcaac tcatatcttc ctgtctttcc ttctgaatag tagtattctc tgctagaaaa   1680
   ctactagtga tggttattac tgagtatgaa tttaagaact gaggttatga ttggtaatac   1740
   aatccaaaaa gaagggtctg aacaccaaaa ttctttatac atatttaagt aactgtatta   1800
   ttattataca gatgtcttta cctttttgac tttatagatc actgcagcat taagaaagtt   1860
   tccagtttac cattccataa gtacaattaa tccttctagt gtaaatgttc aaatactgtt   1920
   ataattatct aggcaattaa taatttacaa actgatattt ttgcacgatt gtagtggtgt   1980
   atagtcttga cttgcagagc attttgcttg agtccttgaa atgtcgtgtt cattcattat   2040
   ttgctgagtg cttacaatgt attaggcact gttctaaata ttaagtgtac taaataaaca   2100
   aaaatccttg tattctg                                                  2117
<210> 42
   <211> 2177
   <212> DNA
   <213> Homo sapiens
<400> 42
   gccgggggcg cagccgacat gggcccgccg ccacggctgc tgtgagcagc ctctttccct 60
   gtgtggccgc cggcgtgggc ggggacggcg cgaccctcgc gcggccgggc tgcgggcttc 120
   caggccagcg cgcgggggcc ggacggacag ccccacaccg acatgtaacc atggactgca 180
   ggaccaaggc aaatccagac agaacctttg acttggtgtt gaaagtgaaa tgtcatgcct 240
   ctgaaaatga agatcctgtg gtattgtgga aattcccaga ggactttgga gaccaggaaa 300
   tactacagag tgtgccaaag ttctgttttc cctttgacgt tgaaagggtg tctcagaatc 360
   aagttggaca gcactttacc tttgtactga cagacattga aagtaaacag agatttggat 420
   tctgcagact gacgtcagga ggcacaattt gtttatgcat ccttagttac cttccctggt 480
   ttgaagtgta ttacaagctt ctaaatactc ttgcagatta cttggctaag gaactggaaa 540
   atgatttgaa tgaaactctc agatcactgt ataaccaccc agtaccaaag gcaaatactc 600
   ctgtaaattt gagtgtgaac caagagatat ttattgcctg tgagcaagtt ctgaaagatc 660
   agcctgctct agtaccgcat tcctacttca ttgcccctga tgtaactgga ctcccaacaa 720
   tacccgagag tagaaatctt acagaatatt ttgttgccgt ggatgtgaac aacatgctgc 780
   agctgtatgc cagtatgctg catgaaaggc gcatcgtgat tatctcgagc aaattaagca 840
   ctttaactgc ctgtatccat ggatcagctg ctcttctata cccaatgtat tggcaacaca 900
   tatacatccc agtgcttcct ccacacctgc tggactactg ctgtgcccca atgccatacc 960
   tgattggaat acactccagc ctcatagaga gagtgaaaaa caaatcattg gaagatgttg 1020
   ttatgttaaa tgttgataca aacacattag aatcaccatt tagtgacttg aacaacctac 1080
   caagtgatgt ggtctcggcc ttgaaaaata aactgaagaa gcagtctaca gctacgggtg 1140
   atggagtagc tagggccttt cttagagcac aggctgcttt gtttggatcc tacagagatg 1200
   cactgagata caaacctggt gagcccatca ctttctgtga ggagagtttt gtaaagcacc 1260
   gctcaagcgt gatgaaacag ttcctggaaa ctgccattaa cctccagctt tttaagcagt 1320
   ttatcgatgg tcgactggca aaactaaatg caggaagggg tttctctgat gtatttgaag 1380
   aagagatcac ttcaggtggc ttttgtggag gtaaagacaa gttacaatat gattatccat 1440
   tttctcaata acaattttct tggtctttgc acttgtgtct gataaaacct atttcataaa 1500
   caactaatga tttcctccta aatatgtaat gtcttaaata catttttcat cttataaaag 1560
   ctatggaatt agcttatttt gcctgatacc tgttactcaa ggcattaagt tggcctcctg 1620
   aattggcagc tgttggcctc gataatctct taatattgct ggaaattagt aatacagaaa 1680
   tccaatcaac tcatatcttc ctgtctttcc ttctgaatag tagtattctc tgctagaaaa 1740
   ctactagtga tggttattac tgagtatgaa tttaagaact gaggttatga ttggtaatac 1800
   aatccaaaaa gaagggtctg aacaccaaaa ttctttatac atatttaagt aactgtatta 1860
   ttattataca gatgtcttta cctttttgac tttatagatc actgcagcat taagaaagtt 1920
   tccagtttac cattccataa gtacaattaa tccttctagt gtaaatgttc aaatactgtt 1980
   ataattatct aggcaattaa taatttacaa actgatattt ttgcacgatt gtagtggtgt 2040
   atagtcttga cttgcagagc attttgcttg agtccttgaa atgtcgtgtt cattcattat 2100
   ttgctgagtg cttacaatgt attaggcact gttctaaata ttaagtgtac taaataaaca 2160
   aaaatccttg tattctg 2177
<210> 43
   <211> 2007
   <212> DNA
   <213> Homo sapiens
<400> 43
   attgagacaa aaacaagggc aaatccagac agaacctttg acttggtgtt gaaagtgaaa     60
   tgtcatgcct ctgaaaatga agatcctgtg gtattgtgga aattcccaga ggactttgga    120
   gaccaggaaa tactacagag tgtgccaaag ttctgttttc cctttgacgt tgaaagggtg    180
   tctcagaatc aagttggaca gcactttacc tttgtactga cagacattga aagtaaacag    240
   agatttggat tctgcagact gacgtcagga ggcacaattt gtttatgcat ccttagttac    300
   cttccctggt ttgaagtgta ttacaagctt ctaaatactc ttgcagatta cttggctaag    360
   gaactggaaa atgatttgaa tgaaactctc agatcactgt ataaccaccc agtaccaaag    420
   gcaaatactc ctgtaaattt gagtgtgaac caagagatat ttattgcctg tgagcaagtt    480
   ctgaaagatc agcctgctct agtaccgcat tcctacttca ttgcccctga tgtaactgga    540
   ctcccaacaa tacccgagag tagaaatctt acagaatatt ttgttgccgt ggatgtgaac    600
   aacatgctgc agctgtatgc cagtatgctg catgaaaggc gcatcgtgat tatctcgagc    660
   aaattaagca ctttaactgc ctgtatccat ggatcagctg ctcttctata cccaatgtat    720
   tggcaacaca tatacatccc agtgcttcct ccacacctgc tggactactg ctgtgcccca    780
   atgccatacc tgattggaat acactccagc ctcatagaga gagtgaaaaa caaatcattg    840
   gaagatgttg ttatgttaaa tgttgataca aacacattag aatcaccatt tagtgacttg    900
   aacaacctac caagtgatgt ggtctcggcc ttgaaaaata aactgaagaa gcagtctaca    960
   gctacgggtg atggagtagc tagggccttt cttagagcac aggctgcttt gtttggatcc   1020
   tacagagatg cactgagata caaacctggt gagcccatca ctttctgtga ggagagtttt   1080
   gtaaagcacc gctcaagcgt gatgaaacag ttcctggaaa ctgccattaa cctccagctt   1140
   tttaagcagt ttatcgatgg tcgactggca aaactaaatg caggaagggg tttctctgat   1200
   gtatttgaag aagagatcac ttcaggtggc ttttgtggag gtaaagacaa gttacaatat   1260
   gattatccat tttctcaata acaattttct tggtctttgc acttgtgtct gataaaacct   1320
   atttcataaa caactaatga tttcctccta aatatgtaat gtcttaaata catttttcat    1380
   cttataaaag ctatggaatt agcttatttt gcctgatacc tgttactcaa ggcattaagt    1440
   tggcctcctg aattggcagc tgttggcctc gataatctct taatattgct ggaaattagt    1500
   aatacagaaa tccaatcaac tcatatcttc ctgtctttcc ttctgaatag tagtattctc    1560
   tgctagaaaa ctactagtga tggttattac tgagtatgaa tttaagaact gaggttatga    1620
   ttggtaatac aatccaaaaa gaagggtctg aacaccaaaa ttctttatac atatttaagt    1680
   aactgtatta ttattataca gatgtcttta cctttttgac tttatagatc actgcagcat    1740
   taagaaagtt tccagtttac cattccataa gtacaattaa tccttctagt gtaaatgttc    1800
   aaatactgtt ataattatct aggcaattaa taatttacaa actgatattt ttgcacgatt    1860
   gtagtggtgt atagtcttga cttgcagagc attttgcttg agtccttgaa atgtcgtgtt    1920
   cattcattat ttgctgagtg cttacaatgt attaggcact gttctaaata ttaagtgtac    1980
   taaataaaca aaaatccttg tattctg                                        2007
<210> 44
   <211> 2197
   <212> DNA
   <213> Homo sapiens
<400> 44
   gcgggggccg gacggacagc cccacaccga catgtaacca tggactgcag gaccaaggca      60
   aatccagaca gaacctttga cttggtgttg aaagtgaaat gtcatgcctc tgaaaatgaa     120
   gaggacagtc cagcttatct gccgaggatt ccccctggaa aagtacgccg attcgcattt     180
   tgcattaaga aactggaaaa ctttcctgtc ggtcctggcg tagcgcctcc cgtgtccggg     240
   gtagaccttg taccggctga aaccgcatag ctcgaccttc atggcggcag ctccacggga     300
   ggagaaaaga tggccccaac ctgtattttc gaatcctgtg gtattgtgga aattcccaga     360
   ggactttgga gaccaggaaa tactacagag tgtgccaaag ttctgttttc cctttgacgt     420
   tgaaagggtg tctcagaatc aagttggaca gcactttacc tttgtactga cagacattga     480
   aagtaaacag agatttggat tctgcagact gacgtcagga ggcacaattt gtttatgcat     540
   ccttagttac cttccctggt ttgaagtgta ttacaagctt ctaaatactc ttgcagatta     600
   cttggctaag gaactggaaa atgatttgaa tgaaactctc agatcactgt ataaccaccc     660
   agtaccaaag gcaaatactc ctgtaaattt gagtgtgcat tcctacttca ttgcccctga     720
   tgtaactgga ctcccaacaa tacccgagag tagaaatctt acagaatatt ttgttgccgt     780
   ggatgtgaac aacatgctgc agctgtatgc cagtatgctg catgaaaggc gcatcgtgat     840
   tatctcgagc aaattaagca ctttaactgc ctgtatccat ggatcagctg ctcttctata     900
   cccaatgtat tggcaacaca tatacatccc agtgcttcct ccacacctgc tggactactg     960
   ctgtgcccca atgccatacc tgattggaat acactccagc ctcatagaga gagtgaaaaa   1020
   caaatcattg gaagatgttg ttatgttaaa tgttgataca aacacattag aatcaccatt   1080
   tagtgacttg aacaacctac caagtgatgt ggtctcggcc ttgaaaaata aactgaagaa   1140
   gcagtctaca gctacgggtg atggagtagc tagggccttt cttagagcac aggctgcttt   1200
   gtttggatcc tacagagatg cactgagata caaacctggt gagcccatca ctttctgtga   1260
   ggagagtttt gtaaagcacc gctcaagcgt gatgaaacag ttcctggaaa ctgccattaa   1320
   cctccagctt tttaagcagt ttatcgatgg tcgactggca aaactaaatg caggaagggg   1380
   tttctctgat gtatttgaag aagagatcac ttcaggtggc ttttgtggag gtaaagacaa   1440
   gttacaatat gattatccat tttctcaata acaattttct tggtctttgc acttgtgtct   1500
   gataaaacct atttcataaa caactaatga tttcctccta aatatgtaat gtcttaaata   1560
   catttttcat cttataaaag ctatggaatt agcttatttt gcctgatacc tgttactcaa   1620
   ggcattaagt tggcctcctg aattggcagc tgttggcctc gataatctct taatattgct   1680
   ggaaattagt aatacagaaa tccaatcaac tcatatcttc ctgtctttcc ttctgaatag   1740
   tagtattctc tgctagaaaa ctactagtga tggttattac tgagtatgaa tttaagaact   1800
   gaggttatga ttggtaatac aatccaaaaa gaagggtctg aacaccaaaa ttctttatac   1860
   atatttaagt aactgtatta ttattataca gatgtcttta cctttttgac tttatagatc   1920
   actgcagcat taagaaagtt tccagtttac cattccataa gtacaattaa tccttctagt   1980
   gtaaatgttc aaatactgtt ataattatct aggcaattaa taatttacaa actgatattt   2040
   ttgcacgatt gtagtggtgt atagtcttga cttgcagagc attttgcttg agtccttgaa   2100
   atgtcgtgtt cattcattat ttgctgagtg cttacaatgt attaggcact gttctaaata   2160
   ttaagtgtac taaataaaca aaaatccttg tattctg                            2197
<210> 45
   <211> 423
   <212> PRT
   <213> Homo sapiens
<400> 45
   Met Gly Gly Pro Arg Ala Trp Ala Leu Leu Cys Leu Gly Leu Leu Leu
   1               5                   10                  15
   Pro Gly Gly Gly Ala Ala Trp Ser Ile Gly Ala Ala Pro Phe Ser Gly
               20                  25                  30
   Arg Arg Asn Trp Cys Ser Tyr Val Val Thr Arg Thr Ile Ser Cys His
           35                  40                  45
   Val Gln Asn Gly Thr Tyr Leu Gln Arg Val Leu Gln Asn Cys Pro Trp
       50                  55                  60
   Pro Met Ser Cys Pro Gly Ser Arg Thr Val Val Arg Pro Thr Tyr Lys
   65                  70                  75                  80
   Val Met Tyr Lys Ile Val Thr Ala Pro Ser Ser Ala Ser Leu Glu Pro
                   85                  90                  95
   Met Trp Ser Gly Ser Thr Met Arg Arg Met Ala Leu Arg Pro Thr Ala
               100                 105                 110
   Phe Ser Gly Cys Leu Asn Cys Ser Lys Val Ser Glu Leu Thr Glu Arg
           115                 120                 125
   Leu Lys Val Leu Glu Ala Lys Met Thr Met Leu Thr Val Ile Glu Gln
       130                 135                 140
   Pro Val Pro Pro Thr Pro Ala Thr Pro Glu Asp Pro Ala Pro Leu Trp
   145                 150                 155                 160
   Gly Pro Pro Pro Ala Gln Gly Ser Pro Gly Asp Gly Gly Leu Gln Asp
                   165                 170                 175
   Gln Val Gly Ala Trp Gly Leu Pro Gly Pro Thr Gly Pro Lys Gly Asp
               180                 185                 190
   Ala Gly Ser Arg Gly Pro Met Gly Met Arg Gly Pro Pro Gly Pro Gln
           195                 200                 205
   Gly Pro Pro Gly Ser Pro Gly Arg Ala Gly Ala Val Gly Thr Pro Gly
       210                 215                 220
   Glu Arg Gly Pro Pro Gly Pro Pro Gly Pro Pro Gly Pro Pro Gly Pro
   225                 230                 235                 240
   Pro Ala Pro Val Gly Pro Pro His Ala Arg Ile Ser Gln His Gly Asp
                   245                 250                 255
   Pro Leu Leu Ser Asn Thr Phe Thr Glu Thr Asn Asn His Trp Pro Gln
               260                 265                 270
   Gly Pro Thr Gly Pro Pro Gly Pro Pro Gly Pro Met Gly Pro Pro Gly
           275                 280                 285
   Pro Pro Gly Pro Thr Gly Val Pro Gly Ser Pro Gly His Ile Gly Pro
       290                 295                 300
   Pro Gly Pro Thr Gly Pro Lys Gly Ile Ser Gly His Pro Gly Glu Lys
   305                 310                 315                 320
   Gly Glu Arg Gly Leu Arg Gly Glu Pro Gly Pro Gln Gly Ser Ala Gly
                   325                 330                 335
   Gln Arg Gly Glu Pro Gly Pro Lys Gly Asp Pro Gly Glu Lys Ser His
               340                 345                 350
   Trp Gly Glu Gly Leu His Gln Leu Arg Glu Ala Leu Lys Ile Leu Ala
           355                 360                 365
   Glu Arg Val Leu Ile Leu Glu Thr Met Ile Gly Leu Tyr Glu Pro Glu
       370                 375                 380
   Leu Gly Ser Gly Ala Gly Pro Ala Gly Thr Gly Thr Pro Ser Leu Leu
   385                 390                 395                 400
   Arg Gly Lys Arg Gly Gly His Ala Thr Asn Tyr Arg Ile Val Ala Pro
                   405                 410                 415
   Arg Ser Arg Asp Glu Arg Gly
               420
<210> 46
   <211> 212
   <212> PRT
   <213> Homo sapiens
<400> 46
   Met Gly Gly Pro Arg Ala Trp Ala Leu Leu Cys Leu Gly Leu Leu Leu
   1               5                   10                  15
   Pro Gly Gly Gly Ala Ala Trp Ser Ile Gly Ala Ala Pro Phe Ser Gly
               20                  25                  30
   Arg Arg Asn Trp Cys Ser Tyr Val Val Thr Arg Thr Ile Ser Cys His
           35                  40                  45
   Val Gln Asn Gly Thr Tyr Leu Gln Arg Val Leu Gln Asn Cys Pro Trp
       50                  55                  60
   Pro Met Ser Cys Pro Gly Ser Ser Tyr Arg Thr Val Val Arg Pro Thr
   65                  70                  75                  80
   Tyr Lys Val Met Tyr Lys Ile Val Thr Ala Arg Glu Trp Arg Cys Cys
                   85                  90                  95
   Pro Gly His Ser Gly Val Ser Cys Glu Glu Val Ala Ala Ser Ser Ala
               100                 105                 110
   Ser Leu Glu Pro Met Trp Ser Gly Ser Thr Met Arg Arg Met Ala Leu
           115                 120                 125
   Arg Pro Thr Ala Phe Ser Gly Cys Leu Asn Cys Ser Lys Val Ser Glu
       130                 135                 140
   Leu Thr Glu Arg Leu Lys Val Leu Glu Ala Lys Met Thr Met Leu Thr
   145                 150                 155                 160
   Val Ile Glu Gln Pro Val Pro Pro Thr Pro Ala Thr Pro Glu Asp Pro
                   165                 170                 175
   Ala Pro Leu Trp Gly Pro Pro Pro Ala Gln Gly Ser Pro Gly Asp Gly
               180                 185                 190
   Gly Leu Gln Gly Asp Pro Leu Leu Ser Asn Thr Phe Thr Glu Thr Asn
           195                 200                 205
   Asn His Trp Pro
       210
<210> 47
   <211> 175
   <212> PRT
   <213> Homo sapiens
<400> 47
   Met Gly Gly Pro Arg Ala Trp Ala Leu Leu Cys Leu Gly Leu Leu Leu
   1               5                   10                  15
   Pro Gly Gly Gly Ala Ala Trp Ser Ile Gly Ala Ala Pro Phe Ser Gly
               20                  25                  30
   Arg Arg Asn Trp Cys Ser Tyr Val Val Thr Arg Thr Ile Ser Cys His
           35                  40                  45
   Val Gln Asn Gly Thr Tyr Leu Gln Arg Val Leu Gln Asn Cys Pro Trp
       50                  55                  60
   Pro Met Ser Cys Pro Gly Ser Ser Tyr Arg Thr Val Val Arg Pro Thr
   65                  70                  75                  80
   Tyr Lys Val Met Tyr Lys Ile Val Thr Ala Arg Glu Trp Arg Cys Cys
                   85                  90                  95
   Pro Gly His Ser Gly Val Ser Cys Glu Glu Gly Cys Leu Asn Cys Ser
               100                 105                 110
   Lys Val Ser Glu Leu Thr Glu Arg Leu Lys Val Leu Glu Ala Lys Met
           115                 120                 125
   Thr Met Leu Thr Val Ile Glu Gln Pro Val Pro Pro Thr Pro Ala Thr
       130                 135                 140
   Pro Glu Asp Pro Ala Pro Leu Trp Gly Pro Pro Pro Ala Gln Gly Ser
   145                 150                 155                 160
   Pro Gly Asp Gly Gly Leu Gln Asp Gln Val Gly Ala Trp Gly Leu
                   165                 170                 175
<210> 48
   <211> 34
   <212> PRT
   <213> Homo sapiens
<400> 48
   Met Gly Gly Pro Arg Ala Trp Ala Leu Leu Cys Leu Gly Leu Leu Leu
   1               5                   10                  15
   Pro Gly Gly Gly Ala Ala Trp Ser Ile Gly Ala Ala Pro Phe Ser Gly
               20                  25                  30
   Arg Arg
<210> 49
   <211> 445
   <212> PRT
   <213> Homo sapiens
<400> 49
   Met Gly Gly Pro Arg Ala Trp Ala Leu Leu Cys Leu Gly Leu Leu Leu
   1               5                   10                  15
   Pro Gly Gly Gly Ala Ala Trp Ser Ile Gly Ala Ala Pro Phe Ser Gly
               20                  25                  30
   Arg Arg Asn Trp Cys Ser Tyr Val Val Thr Arg Thr Ile Ser Cys His
           35                  40                  45
   Val Gln Asn Gly Thr Tyr Leu Gln Arg Val Leu Gln Asn Cys Pro Trp
       50                  55                  60
   Pro Met Ser Cys Pro Gly Ser Ser Tyr Arg Thr Val Val Arg Pro Thr
   65                  70                  75                  80
   Tyr Lys Val Met Tyr Lys Ile Val Thr Ala Arg Glu Trp Arg Cys Cys
                   85                  90                  95
   Pro Gly His Ser Gly Val Ser Cys Glu Glu Val Ala Ala Ser Ser Ala
               100                 105                 110
   Ser Leu Glu Pro Met Trp Ser Gly Ser Thr Met Arg Arg Met Ala Leu
           115                 120                 125
   Arg Pro Thr Ala Phe Ser Gly Cys Leu Asn Cys Ser Lys Val Ser Glu
       130                 135                 140
   Leu Thr Glu Arg Leu Lys Val Leu Glu Ala Lys Met Thr Met Leu Thr
   145                 150                 155                 160
   Val Ile Glu Gln Pro Val Pro Pro Thr Pro Ala Thr Pro Glu Asp Pro
                   165                 170                 175
   Ala Pro Leu Trp Gly Pro Pro Pro Ala Gln Gly Ser Pro Gly Asp Gly
               180                 185                 190
   Gly Leu Gln Asp Gln Val Gly Ala Trp Gly Leu Pro Gly Pro Thr Gly
           195                 200                 205
   Pro Lys Gly Asp Ala Gly Ser Arg Gly Pro Met Gly Met Arg Gly Pro
       210                 215                 220
   Pro Gly Pro Gln Gly Pro Pro Gly Ser Pro Gly Arg Ala Gly Ala Val
   225                 230                 235                 240
   Gly Thr Pro Gly Glu Arg Gly Pro Pro Gly Pro Pro Gly Pro Pro Gly
                   245                 250                 255
   Pro Pro Gly Pro Pro Ala Pro Val Gly Pro Pro His Ala Arg Ile Ser
               260                 265                 270
   Gln His Gly Asp Pro Leu Leu Ser Asn Thr Phe Thr Glu Thr Asn Asn
           275                 280                 285
   His Trp Pro Gln Gly Pro Thr Gly Pro Pro Gly Pro Pro Gly Pro Met
       290                 295                 300
   Gly Pro Pro Gly Pro Pro Gly Pro Thr Gly Val Pro Gly Ser Pro Gly
   305                 310                 315                 320
   His Ile Gly Pro Pro Gly Pro Thr Gly Pro Lys Gly Ile Ser Gly His
                   325                 330                 335
   Pro Gly Glu Lys Gly Glu Arg Gly Leu Arg Gly Glu Pro Gly Pro Gln
               340                 345                 350
   Gly Ser Ala Gly Gln Arg Gly Glu Pro Gly Pro Lys Gly Asp Pro Gly
           355                 360                 365
   Glu Lys Ser His Trp Ala Pro Ser Leu Gln Ser Phe Leu Gln Gln Gln
       370                 375                 380
   Ala Gln Leu Glu Leu Leu Ala Arg Arg Val Thr Leu Leu Glu Ala Ile
   385                 390                 395                 400
   Ile Trp Pro Glu Pro Glu Leu Gly Ser Gly Ala Gly Pro Ala Gly Thr
                   405                 410                 415
   Gly Thr Pro Ser Leu Leu Arg Gly Lys Arg Gly Gly His Ala Thr Asn
               420                 425                 430
   Tyr Arg Ile Val Ala Pro Arg Ser Arg Asp Glu Arg Gly
           435                 440                 445
<210> 50
   <211> 443
   <212> PRT
   <213> Homo sapiens
<400> 50
   Met Gly Gly Pro Arg Ala Trp Ala Leu Leu Cys Leu Gly Leu Leu Leu
   1               5                   10                  15
   Pro Gly Gly Gly Ala Ala Trp Ser Ile Gly Ala Ala Pro Phe Ser Gly
               20                  25                  30
   Arg Arg Asn Trp Cys Ser Tyr Val Val Thr Arg Thr Ile Ser Cys His
           35                  40                  45
   Val Gln Asn Gly Thr Tyr Leu Gln Arg Val Leu Gln Asn Cys Pro Trp
       50                  55                  60
   Pro Met Ser Cys Pro Gly Ser Ser Tyr Arg Thr Val Val Arg Pro Thr
   65                  70                  75                  80
   Tyr Lys Val Met Tyr Lys Ile Val Thr Ala Arg Glu Trp Arg Cys Cys
                   85                  90                  95
   Pro Gly His Ser Gly Val Ser Cys Glu Glu Val Ala Ala Ser Ser Ala
               100                 105                 110
   Ser Leu Glu Pro Met Trp Ser Gly Ser Thr Met Arg Arg Met Ala Leu
           115                 120                 125
   Arg Pro Thr Ala Phe Ser Gly Cys Leu Asn Cys Ser Lys Val Ser Glu
       130                 135                 140
   Leu Thr Glu Arg Leu Lys Val Leu Glu Ala Lys Met Thr Met Leu Thr
   145                 150                 155                 160
   Val Ile Glu Gln Pro Val Pro Pro Thr Pro Ala Thr Pro Glu Asp Pro
                   165                 170                 175
   Ala Pro Leu Trp Gly Pro Pro Pro Ala Gln Gly Ser Pro Gly Asp Gly
               180                 185                 190
   Gly Leu Gln Asp Gln Val Gly Ala Trp Gly Leu Pro Gly Pro Thr Gly
           195                 200                 205
   Pro Lys Gly Asp Ala Gly Ser Arg Gly Pro Met Gly Met Arg Gly Pro
       210                 215                 220
   Pro Gly Pro Gln Gly Pro Pro Gly Ser Pro Gly Arg Ala Gly Ala Val
   225                 230                 235                 240
   Gly Thr Pro Gly Glu Arg Gly Pro Pro Gly Pro Pro Gly Pro Pro Gly
                   245                 250                 255
   Pro Pro Gly Pro Pro Ala Pro Val Gly Pro Pro His Ala Arg Ile Ser
               260                 265                 270
   Gln His Gly Asp Pro Leu Leu Ser Asn Thr Phe Thr Glu Thr Asn Asn
           275                 280                 285
   His Trp Pro Gln Gly Pro Thr Gly Pro Pro Gly Pro Pro Gly Pro Met
       290                 295                 300
   Gly Pro Pro Gly Pro Pro Gly Pro Thr Gly Val Pro Gly Ser Pro Gly
   305                 310                 315                 320
   His Ile Gly Pro Pro Gly Pro Thr Gly Pro Lys Gly Ile Ser Gly His
                   325                 330                 335
   Pro Gly Glu Lys Gly Glu Arg Gly Leu Arg Gly Glu Pro Gly Pro Gln
               340                 345                 350
   Gly Ser Ala Gly Gln Arg Gly Glu Pro Gly Pro Lys Gly Asp Pro Gly
           355                 360                 365
   Glu Lys Ser His Trp Gly Glu Gly Leu His Gln Leu Arg Glu Ala Leu
       370                 375                 380
   Lys Ile Leu Ala Glu Arg Val Leu Ile Leu Glu Thr Met Ile Gly Leu
   385                 390                 395                 400
   Tyr Glu Pro Glu Leu Gly Ser Gly Ala Gly Pro Ala Gly Thr Gly Thr
                   405                 410                 415
   Pro Ser Leu Leu Arg Gly Lys Arg Gly Gly His Ala Thr Asn Tyr Arg
               420                 425                 430
   Ile Val Ala Pro Arg Ser Arg Asp Glu Arg Gly
           435                 440
<210> 51
   <211> 441
   <212> PRT
   <213> Homo sapiens
<400> 51
   Met Gly Gly Pro Arg Ala Trp Ala Leu Leu Cys Leu Gly Leu Leu Leu
   1               5                   10                  15
   Pro Gly Gly Gly Ala Ala Trp Ser Ile Gly Ala Ala Pro Phe Ser Gly
               20                  25                  30
   Arg Arg Asn Trp Cys Ser Tyr Val Val Thr Arg Thr Ile Ser Cys His
           35                  40                  45
   Val Gln Asn Gly Thr Tyr Leu Gln Arg Val Leu Gln Asn Cys Pro Trp
       50                  55                  60
   Pro Met Ser Cys Pro Gly Ser Ser Tyr Arg Thr Val Val Arg Pro Thr
   65                  70                  75                  80
   Tyr Lys Val Met Tyr Lys Ile Val Thr Ala Arg Glu Trp Arg Cys Cys
   85 90 95
   Pro Gly His Ser Gly Val Ser Cys Glu Glu Ala Ser Ser Ala Ser Leu
   100 105 110
   Glu Pro Met Trp Ser Gly Ser Thr Met Arg Arg Met Ala Leu Arg Pro
   115 120 125
   Thr Ala Phe Ser Gly Cys Leu Asn Cys Ser Lys Val Ser Glu Leu Thr
   130 135 140
   Glu Arg Leu Lys Val Leu Glu Ala Lys Met Thr Met Leu Thr Val Ile
   145 150 155 160
   Glu Gln Pro Val Pro Pro Thr Pro Ala Thr Pro Glu Asp Pro Ala Pro
   165 170 175
   Leu Trp Gly Pro Pro Pro Ala Gln Gly Ser Pro Gly Asp Gly Gly Leu
   180 185 190
   Gln Asp Gln Val Gly Ala Trp Gly Leu Pro Gly Pro Thr Gly Pro Lys
   195 200 205
   Gly Asp Ala Gly Ser Arg Gly Pro Met Gly Met Arg Gly Pro Pro Gly
   210 215 220
   Pro Gln Gly Pro Pro Gly Ser Pro Gly Arg Ala Gly Ala Val Gly Thr
   225 230 235 240
   Pro Gly Glu Arg Gly Pro Pro Gly Pro Pro Gly Pro Pro Gly Pro Pro
   245 250 255
   Gly Pro Pro Ala Pro Val Gly Pro Pro His Ala Arg Ile Ser Gln His
   260 265 270
   Gly Asp Pro Leu Leu Ser Asn Thr Phe Thr Glu Thr Asn Asn His Trp
   275 280 285
   Pro Gln Gly Pro Thr Gly Pro Pro Gly Pro Pro Gly Pro Met Gly Pro
   290 295 300
   Pro Gly Pro Pro Gly Pro Thr Gly Val Pro Gly Ser Pro Gly His Ile
   305 310 315 320
   Gly Pro Pro Gly Pro Thr Gly Pro Lys Gly Ile Ser Gly His Pro Gly
   325 330 335
   Glu Lys Gly Glu Arg Gly Leu Arg Gly Glu Pro Gly Pro Gln Gly Ser
               340                 345                 350
   Ala Gly Gln Arg Gly Glu Pro Gly Pro Lys Gly Asp Pro Gly Glu Lys
           355                 360                 365
   Ser His Trp Gly Glu Gly Leu His Gln Leu Arg Glu Ala Leu Lys Ile
       370                 375                 380
   Leu Ala Glu Arg Val Leu Ile Leu Glu Thr Met Ile Gly Leu Tyr Glu
   385                 390                 395                 400
   Pro Glu Leu Gly Ser Gly Ala Gly Pro Ala Gly Thr Gly Thr Pro Ser
                   405                 410                 415
   Leu Leu Arg Gly Lys Arg Gly Gly His Ala Thr Asn Tyr Arg Ile Val
               420                 425                 430
   Ala Pro Arg Ser Arg Asp Glu Arg Gly
           435                 440
<210> 52
   <211> 439
   <212> PRT
   <213> Homo sapiens
<400> 52
   Met Gly Gly Pro Arg Ala Trp Ala Leu Leu Cys Leu Gly Leu Leu Leu
   1               5                   10                  15
   Pro Gly Gly Gly Ala Ala Trp Ser Ile Gly Ala Ala Pro Phe Ser Gly
               20                  25                  30
   Arg Arg Asn Trp Cys Ser Tyr Val Val Thr Arg Thr Ile Ser Cys His
           35                  40                  45
   Val Gln Asn Gly Thr Tyr Leu Gln Arg Val Leu Gln Asn Cys Pro Trp
       50                  55                  60
   Pro Met Ser Cys Pro Gly Ser Arg Thr Val Val Arg Pro Thr Tyr Lys
   65                  70                  75                  80
   Val Met Tyr Lys Ile Val Thr Ala Arg Glu Trp Arg Cys Cys Pro Gly
                   85                  90                  95
   His Ser Gly Val Ser Cys Glu Glu Ala Ser Ser Ala Ser Leu Glu Pro
               100                 105                 110
   Met Trp Ser Gly Ser Thr Met Arg Arg Met Ala Leu Arg Pro Thr Ala
           115                 120                 125
   Phe Ser Gly Cys Leu Asn Cys Ser Lys Val Ser Glu Leu Thr Glu Arg
       130                 135                 140
   Leu Lys Val Leu Glu Ala Lys Met Thr Met Leu Thr Val Ile Glu Gln
   145                 150                 155                 160
   Pro Val Pro Pro Thr Pro Ala Thr Pro Glu Asp Pro Ala Pro Leu Trp
                   165                 170                 175
   Gly Pro Pro Pro Ala Gln Gly Ser Pro Gly Asp Gly Gly Leu Gln Asp
               180                 185                 190
   Gln Val Gly Ala Trp Gly Leu Pro Gly Pro Thr Gly Pro Lys Gly Asp
           195                 200                 205
   Ala Gly Ser Arg Gly Pro Met Gly Met Arg Gly Pro Pro Gly Pro Gln
       210                 215                 220
   Gly Pro Pro Gly Ser Pro Gly Arg Ala Gly Ala Val Gly Thr Pro Gly
   225                 230                 235                 240
   Glu Arg Gly Pro Pro Gly Pro Pro Gly Pro Pro Gly Pro Pro Gly Pro
                   245                 250                 255
   Pro Ala Pro Val Gly Pro Pro His Ala Arg Ile Ser Gln His Gly Asp
               260                 265                 270
   Pro Leu Leu Ser Asn Thr Phe Thr Glu Thr Asn Asn His Trp Pro Gln
           275                 280                 285
   Gly Pro Thr Gly Pro Pro Gly Pro Pro Gly Pro Met Gly Pro Pro Gly
       290                 295                 300
   Pro Pro Gly Pro Thr Gly Val Pro Gly Ser Pro Gly His Ile Gly Pro
   305                 310                 315                 320
   Pro Gly Pro Thr Gly Pro Lys Gly Ile Ser Gly His Pro Gly Glu Lys
                   325                 330                 335
   Gly Glu Arg Gly Leu Arg Gly Glu Pro Gly Pro Gln Gly Ser Ala Gly
               340                 345                 350
   Gln Arg Gly Glu Pro Gly Pro Lys Gly Asp Pro Gly Glu Lys Ser His
           355                 360                 365
   Trp Gly Glu Gly Leu His Gln Leu Arg Glu Ala Leu Lys Ile Leu Ala
       370                 375                 380
   Glu Arg Val Leu Ile Leu Glu Thr Met Ile Gly Leu Tyr Glu Pro Glu
   385                 390                 395                 400
   Leu Gly Ser Gly Ala Gly Pro Ala Gly Thr Gly Thr Pro Ser Leu Leu
                   405                 410                 415
   Arg Gly Lys Arg Gly Gly His Ala Thr Asn Tyr Arg Ile Val Ala Pro
               420                 425                 430
   Arg Ser Arg Asp Glu Arg Gly
           435
<210> 53
   <211> 422
   <212> PRT
   <213> Homo sapiens
<400> 53
   Met Gly Gly Pro Arg Ala Trp Ala Leu Leu Cys Leu Gly Leu Leu Leu
   1               5                   10                  15
   Pro Gly Gly Gly Ala Ala Trp Ser Ile Gly Ala Ala Pro Phe Ser Gly
               20                  25                  30
   Arg Arg Asn Trp Cys Ser Tyr Val Val Thr Arg Thr Ile Ser Cys His
           35                  40                  45
   Val Gln Asn Gly Thr Tyr Leu Gln Arg Val Leu Gln Asn Cys Pro Trp
       50                  55                  60
   Pro Met Ser Cys Pro Gly Ser Ser Tyr Arg Thr Val Val Arg Pro Thr
   65                  70                  75                  80
   Tyr Lys Val Met Tyr Lys Ile Val Thr Ala Arg Glu Trp Arg Cys Cys
                   85                  90                  95
   Pro Gly His Ser Gly Val Ser Cys Glu Glu Val Ala Ala Ser Ser Ala
               100                 105                 110
   Ser Leu Glu Pro Met Trp Ser Gly Ser Thr Met Arg Arg Met Ala Leu
           115                 120                 125
   Arg Pro Thr Ala Phe Ser Gly Cys Leu Asn Cys Ser Lys Val Ser Glu
       130                 135                 140
   Leu Thr Glu Arg Leu Lys Val Leu Glu Ala Lys Met Thr Met Leu Thr
   145                 150                 155                 160
   Val Ile Glu Gln Pro Val Pro Pro Thr Pro Ala Thr Pro Glu Asp Pro
                   165                 170                 175
   Ala Pro Leu Trp Gly Pro Pro Pro Ala Gln Gly Ser Pro Gly Asp Gly
               180                 185                 190
   Gly Leu Gln Asp Gln Val Gly Ala Trp Gly Leu Pro Gly Pro Thr Gly
           195                 200                 205
   Pro Lys Gly Asp Ala Gly Ser Arg Gly Pro Met Gly Met Arg Gly Pro
       210                 215                 220
   Pro Gly Pro Gln Gly Pro Pro Gly Ser Pro Gly Arg Ala Gly Ala Val
   225                 230                 235                 240
   Gly Thr Pro Gly Glu Arg Gly Pro Pro Gly Pro Pro Gly Pro Pro Gly
                   245                 250                 255
   Pro Pro Gly Pro Pro Ala Pro Val Gly Pro Pro His Ala Arg Ile Ser
               260                 265                 270
   Gln His Gly Asp Pro Leu Leu Ser Asn Thr Phe Thr Glu Thr Asn Asn
           275                 280                 285
   His Trp Pro Gln Gly Pro Thr Gly Pro Pro Gly Pro Pro Gly Pro Met
       290                 295                 300
   Gly Pro Pro Gly Pro Pro Gly Pro Thr Gly Val Pro Gly Ser Pro Gly
   305                 310                 315                 320
   His Ile Gly Leu Arg Gly Glu Pro Gly Pro Gln Gly Ser Ala Gly Gln
                   325                 330                 335
   Arg Gly Glu Pro Gly Pro Lys Gly Asp Pro Gly Glu Lys Ser His Trp
               340                 345                 350
   Gly Glu Gly Leu His Gln Leu Arg Glu Ala Leu Lys Ile Leu Ala Glu
           355                 360                 365
   Arg Val Leu Ile Leu Glu Thr Met Ile Gly Leu Tyr Glu Pro Glu Leu
       370                 375                 380
   Gly Ser Gly Ala Gly Pro Ala Gly Thr Gly Thr Pro Ser Leu Leu Arg
   385                 390                 395                 400
   Gly Lys Arg Gly Gly His Ala Thr Asn Tyr Arg Ile Val Ala Pro Arg
                   405                 410                 415
   Ser Arg Asp Glu Arg Gly
               420
<210> 54
   <211> 212
   <212> PRT
   <213> Homo sapiens
<400> 54
   Met Gly Gly Pro Arg Ala Trp Ala Leu Leu Cys Leu Gly Leu Leu Leu
   1               5                   10                  15
   Pro Gly Gly Gly Ala Ala Trp Ser Ile Gly Ala Ala Pro Phe Ser Gly
               20                  25                  30
   Arg Arg Asn Trp Cys Ser Tyr Val Val Thr Arg Thr Ile Ser Cys His
           35                  40                  45
   Val Gln Asn Gly Thr Tyr Leu Gln Arg Val Leu Gln Asn Cys Pro Trp
       50                  55                  60
   Pro Met Ser Cys Pro Gly Ser Ser Tyr Arg Thr Val Val Arg Pro Thr
   65                  70                  75                  80
   Tyr Lys Val Met Tyr Lys Ile Val Thr Ala Arg Glu Trp Arg Cys Cys
                   85                  90                  95
   Pro Gly His Ser Gly Val Ser Cys Glu Glu Val Ala Ala Ser Ser Ala
               100                 105                 110
   Ser Leu Glu Pro Met Trp Ser Gly Ser Thr Met Arg Arg Met Ala Leu
           115                 120                 125
   Arg Pro Thr Ala Phe Ser Gly Cys Leu Asn Cys Ser Lys Val Ser Glu
       130                 135                 140
   Leu Thr Glu Arg Leu Lys Val Leu Glu Ala Lys Met Thr Met Leu Thr
   145                 150                 155                 160
   Val Ile Glu Gln Pro Val Pro Pro Thr Pro Ala Thr Pro Glu Asp Pro
                   165                 170                 175
   Ala Pro Leu Trp Gly Pro Pro Pro Ala Gln Gly Ser Pro Gly Asp Gly
               180                 185                 190
   Gly Leu Gln Gly Asp Pro Leu Leu Ser Asn Thr Phe Thr Glu Thr Asn
           195                 200                 205
   Asn His Trp Pro
       210
<210> 55
   <211> 175
   <212> PRT
   <213> Homo sapiens
<400> 55
   Met Gly Gly Pro Arg Ala Trp Ala Leu Leu Cys Leu Gly Leu Leu Leu
   1               5                   10                  15
   Pro Gly Gly Gly Ala Ala Trp Ser Ile Gly Ala Ala Pro Phe Ser Gly
               20                  25                  30
   Arg Arg Asn Trp Cys Ser Tyr Val Val Thr Arg Thr Ile Ser Cys His
           35                  40                  45
   Val Gln Asn Gly Thr Tyr Leu Gln Arg Val Leu Gln Asn Cys Pro Trp
       50                  55                  60
   Pro Met Ser Cys Pro Gly Ser Ser Tyr Arg Thr Val Val Arg Pro Thr
   65                  70                  75                  80
   Tyr Lys Val Met Tyr Lys Ile Val Thr Ala Arg Glu Trp Arg Cys Cys
                   85                  90                  95
   Pro Gly His Ser Gly Val Ser Cys Glu Glu Gly Cys Leu Asn Cys Ser
               100                 105                 110
   Lys Val Ser Glu Leu Thr Glu Arg Leu Lys Val Leu Glu Ala Lys Met
           115                 120                 125
   Thr Met Leu Thr Val Ile Glu Gln Pro Val Pro Pro Thr Pro Ala Thr
       130                 135                 140
   Pro Glu Asp Pro Ala Pro Leu Trp Gly Pro Pro Pro Ala Gln Gly Ser
   145                 150                 155                 160
   Pro Gly Asp Gly Gly Leu Gln Asp Gln Val Gly Ala Trp Gly Leu
                   165                 170                 175
<210> 56
   <211> 236
   <212> PRT
   <213> Homo sapiens
<220>
   <221> misc_feature
   <222> (1) .. (1)
   <223> Xaa can be any naturally occurring amino acid
<400> 56
   Xaa Met Thr Met Leu Thr Val Ile Glu Gln Pro Val Pro Pro Thr Pro
   1               5                   10                  15
   Ala Thr Pro Glu Asp Pro Ala Pro Leu Trp Gly Pro Pro Pro Ala Gln
               20                  25                  30
   Gly Ser Pro Gly Asp Gly Gly Leu Gln Gly Leu Pro Gly Ala Ile Glu
           35                  40                  45
   Ser Val Arg Val Pro Leu Leu Pro Arg Asn Asp Gln Val Gly Ala Trp
       50                  55                  60
   Gly Leu Pro Gly Pro Thr Gly Pro Lys Gly Asp Ala Gly Ser Arg Gly
   65                  70                  75                  80
   Pro Met Gly Met Arg Gly Pro Pro Gly Pro Gln Gly Pro Pro Gly Ser
                   85                  90                  95
   Pro Gly Arg Ala Gly Ala Val Gly Thr Pro Gly Glu Arg Gly Pro Pro
               100                 105                 110
   Gly Pro Pro Gly Pro Pro Gly Pro Pro Gly Pro Pro Ala Pro Val Gly
           115                 120                 125
   Pro Pro His Ala Arg Ile Ser Gln His Gly Asp Pro Leu Leu Ser Asn
       130                 135                 140
   Thr Phe Thr Glu Thr Asn Asn His Trp Pro Gln Gly Pro Thr Gly Pro
   145                 150                 155                 160
   Pro Gly Pro Pro Gly Pro Met Gly Pro Pro Gly Pro Pro Gly Pro Thr
                   165                 170                 175
   Gly Val Pro Gly Ser Pro Gly His Ile Gly Pro Pro Gly Pro Thr Gly
               180                 185                 190
   Pro Lys Gly Ile Ser Gly His Pro Gly Glu Lys Gly Glu Arg Gly Leu
           195                 200                 205
   Arg Gly Glu Pro Gly Pro Gln Gly Ser Ala Gly Gln Arg Gly Glu Pro
       210                 215                 220
   Gly Pro Lys Gly Asp Pro Gly Glu Lys Ser His Trp
   225                 230                 235
<210> 57
   <211> 305
   <212> PRT
   <213> Homo sapiens
<400> 57
   Met Thr Met Leu Thr Val Ile Glu Gln Pro Val Pro Pro Thr Pro Ala
   1               5                   10                  15
   Thr Pro Glu Asp Pro Ala Pro Leu Trp Gly Pro Pro Pro Ala Gln Gly
               20                  25                  30
   Ser Pro Gly Asp Gly Gly Leu Gln Gly Leu Pro Gly Ala Ile Glu Ser
           35                  40                  45
   Val Arg Val Pro Leu Leu Pro Arg Asn Asp Gln Val Gly Ala Trp Gly
       50                  55                  60
   Leu Pro Gly Pro Thr Gly Pro Lys Gly Asp Ala Gly Ser Arg Gly Pro
   65                  70                  75                  80
   Met Gly Met Arg Gly Pro Pro Gly Pro Gln Gly Pro Pro Gly Ser Pro
                   85                  90                  95
   Gly Arg Ala Gly Ala Val Gly Thr Pro Gly Glu Arg Gly Pro Pro Gly
               100                 105                 110
   Pro Pro Gly Pro Pro Gly Pro Pro Gly Pro Pro Ala Pro Val Gly Pro
           115                 120                 125
   Pro His Ala Arg Ile Ser Gln His Gly Asp Pro Leu Leu Ser Asn Thr
       130                 135                 140
   Phe Thr Glu Thr Asn Asn His Trp Pro Gln Gly Pro Thr Gly Pro Pro
   145                 150                 155                 160
   Gly Pro Pro Gly Pro Met Gly Pro Pro Gly Pro Pro Gly Pro Thr Gly
                   165                 170                 175
   Val Pro Gly Ser Pro Gly His Ile Gly Pro Pro Gly Pro Thr Gly Pro
               180                 185                 190
   Lys Gly Ile Ser Gly His Pro Gly Glu Lys Gly Glu Arg Gly Leu Arg
           195                 200                 205
   Gly Glu Pro Gly Pro Gln Gly Ser Ala Gly Gln Arg Gly Glu Pro Gly
       210                 215                 220
   Pro Lys Gly Asp Pro Gly Glu Lys Ser His Trp Gly Glu Gly Leu His
   225                 230                 235                 240
   Gln Leu Arg Glu Ala Leu Lys Ile Leu Ala Glu Arg Val Leu Ile Leu
                   245                 250                 255
   Glu Thr Met Ile Gly Leu Tyr Glu Pro Glu Leu Gly Ser Gly Ala Gly
               260                 265                 270
   Pro Ala Gly Thr Gly Thr Pro Ser Leu Leu Arg Gly Lys Arg Gly Gly
           275                 280                 285
   His Ala Thr Asn Tyr Arg Ile Val Ala Pro Arg Ser Arg Asp Glu Arg
       290                 295                 300
   Gly
   305
<210> 58
   <211> 226
   <212> PRT
   <213> Homo sapiens
<400> 58
<210> 59
   <211> 2031
   <212> DNA
   <213> Homo sapiens
<400> 59
   gggctccgcg cgtccggggc ggctggcggc gcgggcaggc aggcggggag gacaggctgg     60
   gggcggcgac cgcgaggggc cgcgcgcgga gggcgcctgg tgcagcatgg gcggcccgcg    120
   ggcttgggcg ctgctctgcc tcgggctcct gctcccggga ggcggcgctg cgtggagcat    180
   cggggcagct ccgttctccg gacgcaggaa ctggtgctcc tatgtggtga cccgcaccat    240
   ctcatgccat gtgcagaatg gcacctacct tcagcgagtg ctgcagaact gcccctggcc    300
   catgagctgt ccggggagca gaactgtggt gagacccaca tacaaggtga tgtacaagat     360
   agtgaccgcc ccttcctctg cctccttgga gcccatgtgg tcgggcagta ccatgcggcg     420
   gatggcgctt cggcccacag ccttctcagg ttgtctcaac tgcagcaaag tgtcagagct     480
   gacagagcgg ctgaaggtgc tggaggccaa gatgaccatg ctgactgtca tagagcagcc     540
   agtacctcca acaccagcta cccctgagga ccctgccccg ctctggggtc cccctcctgc     600
   ccagggcagc cccggagatg gaggcctcca ggaccaagtc ggtgcttggg ggcttcccgg     660
   gcccaccggc cccaagggag atgccggcag tcggggccca atggggatga gaggcccacc     720
   aggtccacag ggccccccag ggagccctgg ccgggctgga gctgtgggca cccctggaga     780
   gaggggacct cctgggccac cagggcctcc tggcccccct gggcccccag cccctgttgg     840
   gccaccccat gcccggatct cccagcatgg agacccattg ctgtccaaca ccttcactga     900
   gaccaacaac cactggcccc agggacccac tgggcctcca ggccctccag ggcccatggg     960
   tccccctggg cctcctggcc ccacaggtgt ccctgggagt cctggtcaca taggaccccc    1020
   aggccccact ggacccaaag gaatctctgg ccacccagga gagaagggcg agagaggact    1080
   gcgtggggag cctggccccc aaggctctgc tgggcagcgg ggggaacctg gccctaaggg    1140
   agaccctggt gagaagagcc actgggggga ggggttgcac cagctacgcg aggctttgaa    1200
   gattttagct gagagggttt taatcttgga aacaatgatt gggctctatg aaccagagct    1260
   ggggtctggg gcgggccctg ccggcacagg cacccccagc ctccttcggg gcaagagggg    1320
   cggacatgca accaactacc ggatcgtggc ccccaggagc cgggacgaga gaggctgagg   1380
   gtggtggcgg cccctgaggc agaccaggcc aggcttcccc tcctacctgg actcggccag    1440
   ctgcctccag ggaccgcccg tccatattta ttaatgtcct cagggtccct tctgccatct    1500
   aggccttagg ggtaagcagg tctcagtcct ggcaccatgc acatgtctga ggctgagcaa    1560
   gggctgagag gagaggcttg ggcctcagtt tccctctgtg aagtgggggg aggcaggcct    1620
   tcaaggaggg atagaggtac aaggcttcgt ctcatctgct gtctgagcat ccaggcccaa    1680
   aggcactgag ggagtcagga gctggggctc ggcacatgca gagatgacag ggcagggggc    1740
   agtcttcctc cccctccccg accaaacctc ggggagccct cctgtgcccc tccctccttg    1800
   ttgtccagtg ctgggctccc caccccgagg tcaggctgcc caatcctctg actggatcac    1860
   cgggggcttc ttgcctcagt tcttccctct gagcccccag gccctcccgc atctcaggtt    1920
   ggggatgggg acatggagag gaaggggccg cctactcctg caaatgcttg tgacagatgc    1980
   caggaggtag atgtgtgctg gccaataaag gcccctacct gattccccgc a             2031
<210> 60
   <211> 769
   <212> DNA
   <213> Homo sapiens
<400> 60
   cgccctccgg ccgcggagct ggaaaccggg ctccgcgcgt ccggggcggc tggcggcgcg     60
   ggcaggcagg cggggaggac aggctggggg cggcgaccgc gaggggccgc gcgcggaggg    120
   cgcctggtgc agcatgggcg gcccgcgggc ttgggcgctg ctctgcctcg ggctcctgct    180
   cccgggaggc ggcgctgcgt ggagcatcgg ggcagctccg ttctccggac gcaggaactg    240
   gtgctcctat gtggtgaccc gcaccatctc atgccatgtg cagaatggca cctaccttca    300
   gcgagtgctg cagaactgcc cctggcccat gagctgtccg gggagcagct acagaactgt    360
   ggtgagaccc acatacaagg tgatgtacaa gatagtgacc gcccgtgagt ggaggtgctg    420
   ccctgggcac tcaggagtga gctgcgagga agttgcagct tcctctgcct ccttggagcc    480
   catgtggtcg ggcagtacca tgcggcggat ggcgcttcgg cccacagcct tctcaggttg    540
   tctcaactgc agcaaagtgt cagagctgac agagcggctg aaggtgctgg aggccaagat    600
   gaccatgctg actgtcatag agcagccagt acctccaaca ccagctaccc ctgaggaccc    660
   tgccccgctc tggggtcccc ctcctgccca gggcagcccc ggagatggag gcctccaggg    720
   agacccattg ctgtccaaca ccttcactga gaccaacaac cactggccc                769
<210> 61
   <211> 641
   <212> DNA
   <213> Homo sapiens
<400> 61
   gctggaaacc gggctccgcg cgtccggggc ggctggcggc gcgggcaggc aggcggggag 60
   gacaggctgg gggcggcgac cgcgaggggc cgcgcgcgga gggcgcctgg tgcagcatgg 120
   gcggcccgcg ggcttgggcg ctgctctgcc tcgggctcct gctcccggga ggcggcgctg 180
   cgtggagcat cggggcagct ccgttctccg gacgcaggaa ctggtgctcc tatgtggtga 240
   cccgcaccat ctcatgccat gtgcagaatg gcacctacct tcagcgagtg ctgcagaact 300
   gcccctggcc catgagctgt ccggggagca gctacagaac tgtggtgaga cccacataca 360
   aggtgatgta caagatagtg accgcccgtg agtggaggtg ctgccctggg cactcaggag 420
   tgagctgcga ggaaggttgt ctcaactgca gcaaagtgtc agagctgaca gagcggctga 480
   aggtgctgga ggccaagatg accatgctga ctgtcataga gcagccagta cctccaacac 540
   cagctacccc tgaggaccct gccccgctct ggggtccccc tcctgcccag ggcagccccg 600
   gagatggagg cctccaggac caagtcggtg cttgggggct t 641
<210> 62
   <211> 482
   <212> DNA
   <213> Homo sapiens
<400> 62
   cggcgcgggc aggcaggcgg ggaggacagg ctgggggcgg cgaccgcgag gggccgcgcg 60
   cggagggcgc ctggtgcagc atgggcggcc cgcgggcttg ggcgctgctc tgcctcgggc 120
   tcctgctccc gggaggcggc gctgcgtgga gcatcggggc agctccgttc tccggacgca 180
   gatgaccatg ctgactgtca tagagcagcc agtacctcca acaccagcta cccctgagga 240
   ccctgccccg ctctggggtc cccctcctgc ccagggcagc cccggagatg gaggcctcca 300
   ggaccaagtc ggtgcttggg ggcttcccgg gcccaccggc cccaagggag atgccggcag 360
   tcggggccca atggggatga gaggcccacc aggtccacag ggccccccag ggagccctgg 420
   ccgggctgga gctgtgggca cccctggaga gaggggacct cctgggccac cagggcctcc 480
   tg 482
<210> 63
   <211> 2066
   <212> DNA
   <213> Homo sapiens
<400> 63
   cgggcaggca ggcggggagg acaggctggg ggcggcgacc gcgaggggcc gcgcgcggag      60
   ggcgcctggt gcagcatggg cggcccgcgg gcttgggcgc tgctctgcct cgggctcctg     120
   ctcccgggag gcggcgctgc gtggagcatc ggggcagctc cgttctccgg acgcaggaac     180
   tggtgctcct atgtggtgac ccgcaccatc tcatgccatg tgcagaatgg cacctacctt     240
   cagcgagtgc tgcagaactg cccctggccc atgagctgtc cggggagcag ctacagaact     300
   gtggtgagac ccacatacaa ggtgatgtac aagatagtga ccgcccgtga gtggaggtgc     360
   tgccctgggc actcaggagt gagctgcgag gaagttgcag cttcctctgc ctccttggag     420
   cccatgtggt cgggcagtac catgcggcgg atggcgcttc ggcccacagc cttctcaggt     480
   tgtctcaact gcagcaaagt gtcagagctg acagagcggc tgaaggtgct ggaggccaag     540
   atgaccatgc tgactgtcat agagcagcca gtacctccaa caccagctac ccctgaggac     600
   cctgccccgc tctggggtcc ccctcctgcc cagggcagcc ccggagatgg aggcctccag     660
   gaccaagtcg gtgcttgggg gcttcccggg cccaccggcc ccaagggaga tgccggcagt     720
   cggggcccaa tggggatgag aggcccacca ggtccacagg gccccccagg gagccctggc     780
   cgggctggag ctgtgggcac ccctggagag aggggacctc ctgggccacc agggcctcct     840
   ggcccccctg ggcccccagc ccctgttggg ccaccccatg cccggatctc ccagcatgga     900
   gacccattgc tgtccaacac cttcactgag accaacaacc actggcccca gggacccact     960
   gggcctccag gccctccagg gcccatgggt ccccctgggc ctcctggccc cacaggtgtc    1020
   cctgggagtc ctggtcacat aggaccccca ggccccactg gacccaaagg aatctctggc 1080
   cacccaggag agaagggcga gagaggactg cgtggggagc ctggccccca aggctctgct 1140
   gggcagcggg gggaacctgg ccctaaggga gaccctggtg agaagagcca ctgggctcct 1200
   agcttacaga gcttcctgca gcagcaggct cagctggagc tcctggccag acgggtcacc 1260
   ctcctggaag ccatcatctg gccagaacca gagctggggt ctggggcggg ccctgccggc 1320
   acaggcaccc ccagcctcct tcggggcaag aggggcggac atgcaaccaa ctaccggatc 1380
   gtggccccca ggagccggga cgagagaggc tgagggtggt ggcggcccct gaggcagacc 1440
   aggccaggct tcccctccta cctggactcg gccagctgcc tccagggacc gcccgtccat 1500
   atttattaat gtcctcaggg tcccttctgc catctaggcc ttaggggtaa gcaggtctca 1560
   gtcctggcac catgcacatg tctgaggctg agcaagggct gagaggagag gcttgggcct 1620
   cagtttccct ctgtgaagtg gggggaggca ggccttcaag gagggataga ggtacaaggc 1680
   ttcgtctcat ctgctgtctg agcatccagg cccaaaggca ctgagggagt caggagctgg 1740
   ggctcggcac atgcagagat gacagggcag ggggcagtct tcctccccct ccccgaccaa 1800
   acctcgggga gccctcctgt gcccctccct ccttgttgtc cagtgctggg ctccccaccc 1860
   cgaggtcagg ctgcccaatc ctctgactgg atcaccgggg gcttcttgcc tcagttcttc 1920
   cctctgagcc cccaggccct cccgcatctc aggttgggga tggggacatg gagaggaagg 1980
   ggccgcctac tcctgcaaat gcttgtgaca gatgccagga ggtagatgtg tgctggccaa 2040
   taaaggcccc tacctgattc cccgca 2066
<210> 64
   <211> 2060
   <212> DNA
   <213> Homo sapiens
<400> 64
   cgggcaggca ggcggggagg acaggctggg ggcggcgacc gcgaggggcc gcgcgcggag      60
   ggcgcctggt gcagcatggg cggcccgcgg gcttgggcgc tgctctgcct cgggctcctg     120
   ctcccgggag gcggcgctgc gtggagcatc ggggcagctc cgttctccgg acgcaggaac     180
   tggtgctcct atgtggtgac ccgcaccatc tcatgccatg tgcagaatgg cacctacctt     240
   cagcgagtgc tgcagaactg cccctggccc atgagctgtc cggggagcag ctacagaact     300
   gtggtgagac ccacatacaa ggtgatgtac aagatagtga ccgcccgtga gtggaggtgc     360
   tgccctgggc actcaggagt gagctgcgag gaagttgcag cttcctctgc ctccttggag     420
   cccatgtggt cgggcagtac catgcggcgg atggcgcttc ggcccacagc cttctcaggt     480
   tgtctcaact gcagcaaagt gtcagagctg acagagcggc tgaaggtgct ggaggccaag     540
   atgaccatgc tgactgtcat agagcagcca gtacctccaa caccagctac ccctgaggac     600
   cctgccccgc tctggggtcc ccctcctgcc cagggcagcc ccggagatgg aggcctccag     660
   gaccaagtcg gtgcttgggg gcttcccggg cccaccggcc ccaagggaga tgccggcagt     720
   cggggcccaa tggggatgag aggcccacca ggtccacagg gccccccagg gagccctggc     780
   cgggctggag ctgtgggcac ccctggagag aggggacctc ctgggccacc agggcctcct     840
   ggcccccctg ggcccccagc ccctgttggg ccaccccatg cccggatctc ccagcatgga     900
   gacccattgc tgtccaacac cttcactgag accaacaacc actggcccca gggacccact     960
   gggcctccag gccctccagg gcccatgggt ccccctgggc ctcctggccc cacaggtgtc    1020
   cctgggagtc ctggtcacat aggaccccca ggccccactg gacccaaagg aatctctggc    1080
   cacccaggag agaagggcga gagaggactg cgtggggagc ctggccccca aggctctgct    1140
   gggcagcggg gggaacctgg ccctaaggga gaccctggtg agaagagcca ctggggggag    1200
   gggttgcacc agctacgcga ggctttgaag attttagctg agagggtttt aatcttggaa    1260
   acaatgattg ggctctatga accagagctg gggtctgggg cgggccctgc cggcacaggc    1320
   acccccagcc tccttcgggg caagaggggc ggacatgcaa ccaactaccg gatcgtggcc    1380
   cccaggagcc gggacgagag aggctgaggg tggtggcggc ccctgaggca gaccaggcca    1440
   ggcttcccct cctacctgga ctcggccagc tgcctccagg gaccgcccgt ccatatttat    1500
   taatgtcctc agggtccctt ctgccatcta ggccttaggg gtaagcaggt ctcagtcctg    1560
   gcaccatgca catgtctgag gctgagcaag ggctgagagg agaggcttgg gcctcagttt    1620
   ccctctgtga agtgggggga ggcaggcctt caaggaggga tagaggtaca aggcttcgtc    1680
   tcatctgctg tctgagcatc caggcccaaa ggcactgagg gagtcaggag ctggggctcg    1740
   gcacatgcag agatgacagg gcagggggca gtcttcctcc ccctccccga ccaaacctcg    1800
   gggagccctc ctgtgcccct ccctccttgt tgtccagtgc tgggctcccc accccgaggt    1860
   caggctgccc aatcctctga ctggatcacc gggggcttct tgcctcagtt cttccctctg    1920
   agcccccagg ccctcccgca tctcaggttg gggatgggga catggagagg aaggggccgc    1980
   ctactcctgc aaatgcttgt gacagatgcc aggaggtaga tgtgtgctgg ccaataaagg    2040
   cccctacctg attccccgca                                                2060
<210> 65
   <211> 2054
   <212> DNA
   <213> Homo sapiens
<400> 65
   cgggcaggca ggcggggagg acaggctggg ggcggcgacc gcgaggggcc gcgcgcggag     60
   ggcgcctggt gcagcatggg cggcccgcgg gcttgggcgc tgctctgcct cgggctcctg    120
   ctcccgggag gcggcgctgc gtggagcatc ggggcagctc cgttctccgg acgcaggaac 180
   tggtgctcct atgtggtgac ccgcaccatc tcatgccatg tgcagaatgg cacctacctt 240
   cagcgagtgc tgcagaactg cccctggccc atgagctgtc cggggagcag ctacagaact 300
   gtggtgagac ccacatacaa ggtgatgtac aagatagtga ccgcccgtga gtggaggtgc 360
   tgccctgggc actcaggagt gagctgcgag gaagcttcct ctgcctcctt ggagcccatg 420
   tggtcgggca gtaccatgcg gcggatggcg cttcggccca cagccttctc aggttgtctc 480
   aactgcagca aagtgtcaga gctgacagag cggctgaagg tgctggaggc caagatgacc 540
   atgctgactg tcatagagca gccagtacct ccaacaccag ctacccctga ggaccctgcc 600
   ccgctctggg gtccccctcc tgcccagggc agccccggag atggaggcct ccaggaccaa 660
   gtcggtgctt gggggcttcc cgggcccacc ggccccaagg gagatgccgg cagtcggggc 720
   ccaatgggga tgagaggccc accaggtcca cagggccccc cagggagccc tggccgggct 780
   ggagctgtgg gcacccctgg agagagggga cctcctgggc caccagggcc tcctggcccc 840
   cctgggcccc cagcccctgt tgggccaccc catgcccgga tctcccagca tggagaccca 900
   ttgctgtcca acaccttcac tgagaccaac aaccactggc cccagggacc cactgggcct 960
   ccaggccctc cagggcccat gggtccccct gggcctcctg gccccacagg tgtccctggg 1020
   agtcctggtc acataggacc cccaggcccc actggaccca aaggaatctc tggccaccca 1080
   ggagagaagg gcgagagagg actgcgtggg gagcctggcc cccaaggctc tgctgggcag 1140
   cggggggaac ctggccctaa gggagaccct ggtgagaaga gccactgggg ggaggggttg 1200
   caccagctac gcgaggcttt gaagatttta gctgagaggg ttttaatctt ggaaacaatg 1260
   attgggctct atgaaccaga gctggggtct ggggcgggcc ctgccggcac aggcaccccc 1320
   agcctccttc ggggcaagag gggcggacat gcaaccaact accggatcgt ggcccccagg 1380
   agccgggacg agagaggctg agggtggtgg cggcccctga ggcagaccag gccaggcttc 1440
   ccctcctacc tggactcggc cagctgcctc cagggaccgc ccgtccatat ttattaatgt 1500
   cctcagggtc ccttctgcca tctaggcctt aggggtaagc aggtctcagt cctggcacca 1560
   tgcacatgtc tgaggctgag caagggctga gaggagaggc ttgggcctca gtttccctct 1620
   gtgaagtggg gggaggcagg ccttcaagga gggatagagg tacaaggctt cgtctcatct 1680
   gctgtctgag catccaggcc caaaggcact gagggagtca ggagctgggg ctcggcacat 1740
   gcagagatga cagggcaggg ggcagtcttc ctccccctcc ccgaccaaac ctcggggagc 1800
   cctcctgtgc ccctccctcc ttgttgtcca gtgctgggct ccccaccccg aggtcaggct 1860
   gcccaatcct ctgactggat caccgggggc ttcttgcctc agttcttccc tctgagcccc 1920
   caggccctcc cgcatctcag gttggggatg gggacatgga gaggaagggg ccgcctactc 1980
   ctgcaaatgc ttgtgacaga tgccaggagg tagatgtgtg ctggccaata aaggccccta 2040
   cctgattccc cgca 2054
<210> 66
   <211> 2048
   <212> DNA
   <213> Homo sapiens
<400> 66
   cgggcaggca ggcggggagg acaggctggg ggcggcgacc gcgaggggcc gcgcgcggag      60
   ggcgcctggt gcagcatggg cggcccgcgg gcttgggcgc tgctctgcct cgggctcctg     120
   ctcccgggag gcggcgctgc gtggagcatc ggggcagctc cgttctccgg acgcaggaac     180
   tggtgctcct atgtggtgac ccgcaccatc tcatgccatg tgcagaatgg cacctacctt     240
   cagcgagtgc tgcagaactg cccctggccc atgagctgtc cggggagcag aactgtggtg     300
   agacccacat acaaggtgat gtacaagata gtgaccgccc gtgagtggag gtgctgccct     360
   gggcactcag gagtgagctg cgaggaagct tcctctgcct ccttggagcc catgtggtcg     420
   ggcagtacca tgcggcggat ggcgcttcgg cccacagcct tctcaggttg tctcaactgc     480
   agcaaagtgt cagagctgac agagcggctg aaggtgctgg aggccaagat gaccatgctg     540
   actgtcatag agcagccagt acctccaaca ccagctaccc ctgaggaccc tgccccgctc     600
   tggggtcccc ctcctgccca gggcagcccc ggagatggag gcctccagga ccaagtcggt     660
   gcttgggggc ttcccgggcc caccggcccc aagggagatg ccggcagtcg gggcccaatg     720
   gggatgagag gcccaccagg tccacagggc cccccaggga gccctggccg ggctggagct     780
   gtgggcaccc ctggagagag gggacctcct gggccaccag ggcctcctgg cccccctggg     840
   cccccagccc ctgttgggcc accccatgcc cggatctccc agcatggaga cccattgctg     900
   tccaacacct tcactgagac caacaaccac tggccccagg gacccactgg gcctccaggc     960
   cctccagggc ccatgggtcc ccctgggcct cctggcccca caggtgtccc tgggagtcct    1020
   ggtcacatag gacccccagg ccccactgga cccaaaggaa tctctggcca cccaggagag    1080
   aagggcgaga gaggactgcg tggggagcct ggcccccaag gctctgctgg gcagcggggg    1140
   gaacctggcc ctaagggaga ccctggtgag aagagccact gggggagggg gttgcaccag    1200
   ctacgcgagg ctttgaagat tttagctgag agggttttaa tcttggaaac aatgattggg    1260
   ctctatgaac cagagctggg gtctggggcg ggccctgccg gcacaggcac ccccagcctc    1320
   cttcggggca agaggggcgg acatgcaacc aactaccgga tcgtggcccc caggagccgg    1380
   gacgagagag gctgagggtg gtggcggccc ctgaggcaga ccaggccagg cttcccctcc    1440
   tacctggact cggccagctg cctccaggga ccgcccgtcc atatttatta atgtcctcag    1500
   ggtcccttct gccatctagg ccttaggggt aagcaggtct cagtcctggc accatgcaca    1560
   tgtctgaggc tgagcaaggg ctgagaggag aggcttgggc ctcagtttcc ctctgtgaag   1620
   tggggggagg caggccttca aggagggata gaggtacaag gcttcgtctc atctgctgtc   1680
   tgagcatcca ggcccaaagg cactgaggga gtcaggagct ggggctcggc acatgcagag   1740
   atgacagggc agggggcagt cttcctcccc ctccccgacc aaacctcggg gagccctcct   1800
   gtgcccctcc ctccttgttg tccagtgctg ggctccccac cccgaggtca ggctgcccaa   1860
   tcctctgact ggatcaccgg gggcttcttg cctcagttct tccctctgag cccccaggcc   1920
   ctcccgcatc tcaggttggg gatggggaca tggagaggaa ggggccgcct actcctgcaa   1980
   atgcttgtga cagatgccag gaggtagatg tgtgctggcc aataaaggcc cctacctgat   2040
   tccccgca                                                            2048
<210> 67
   <211> 1997
   <212> DNA
   <213> Homo sapiens
<400> 67
   cgggcaggca ggcggggagg acaggctggg ggcggcgacc gcgaggggcc gcgcgcggag     60
   ggcgcctggt gcagcatggg cggcccgcgg gcttgggcgc tgctctgcct cgggctcctg    120
   ctcccgggag gcggcgctgc gtggagcatc ggggcagctc cgttctccgg acgcaggaac    180
   tggtgctcct atgtggtgac ccgcaccatc tcatgccatg tgcagaatgg cacctacctt    240
   cagcgagtgc tgcagaactg cccctggccc atgagctgtc cggggagcag ctacagaact    300
   gtggtgagac ccacatacaa ggtgatgtac aagatagtga ccgcccgtga gtggaggtgc    360
   tgccctgggc actcaggagt gagctgcgag gaagttgcag cttcctctgc ctccttggag    420
   cccatgtggt cgggcagtac catgcggcgg atggcgcttc ggcccacagc cttctcaggt    480
   tgtctcaact gcagcaaagt gtcagagctg acagagcggc tgaaggtgct ggaggccaag    540
   atgaccatgc tgactgtcat agagcagcca gtacctccaa caccagctac ccctgaggac    600
   cctgccccgc tctggggtcc ccctcctgcc cagggcagcc ccggagatgg aggcctccag    660
   gaccaagtcg gtgcttgggg gcttcccggg cccaccggcc ccaagggaga tgccggcagt    720
   cggggcccaa tggggatgag aggcccacca ggtccacagg gccccccagg gagccctggc    780
   cgggctggag ctgtgggcac ccctggagag aggggacctc ctgggccacc agggcctcct    840
   ggcccccctg ggcccccagc ccctgttggg ccaccccatg cccggatctc ccagcatgga    900
   gacccattgc tgtccaacac cttcactgag accaacaacc actggcccca gggacccact    960
   gggcctccag gccctccagg gcccatgggt ccccctgggc ctcctggccc cacaggtgtc   1020
   cctgggagtc ctggtcacat aggactgcgt ggggagcctg gcccccaagg ctctgctggg   1080
   cagcgggggg aacctggccc taagggagac cctggtgaga agagccactg gggggagggg 1140
   ttgcaccagc tacgcgaggc tttgaagatt ttagctgaga gggttttaat cttggaaaca 1200
   atgattgggc tctatgaacc agagctgggg tctggggcgg gccctgccgg cacaggcacc 1260
   cccagcctcc ttcggggcaa gaggggcgga catgcaacca actaccggat cgtggccccc 1320
   aggagccggg acgagagagg ctgagggtgg tggcggcccc tgaggcagac caggccaggc 1380
   ttcccctcct acctggactc ggccagctgc ctccagggac cgcccgtcca tatttattaa 1440
   tgtcctcagg gtcccttctg ccatctaggc cttaggggta agcaggtctc agtcctggca 1500
   ccatgcacat gtctgaggct gagcaagggc tgagaggaga ggcttgggcc tcagtttccc 1560
   tctgtgaagt ggggggaggc aggccttcaa ggagggatag aggtacaagg cttcgtctca 1620
   tctgctgtct gagcatccag gcccaaaggc actgagggag tcaggagctg gggctcggca 1680
   catgcagaga tgacagggca gggggcagtc ttcctccccc tccccgacca aacctcgggg 1740
   agccctcctg tgcccctccc tccttgttgt ccagtgctgg gctccccacc ccgaggtcag 1800
   gctgcccaat cctctgactg gatcaccggg ggcttcttgc ctcagttctt ccctctgagc 1860
   ccccaggccc tcccgcatct caggttgggg atggggacat ggagaggaag gggccgccta 1920
   ctcctgcaaa tgcttgtgac agatgccagg aggtagatgt gtgctggcca ataaaggccc 1980
   ctacctgatt ccccgca 1997
<210> 68
   <211> 1823
   <212> DNA
   <213> Homo sapiens
<400> 68
   cgggcaggca ggcggggagg acaggctggg ggcggcgacc gcgaggggcc gcgcgcggag      60
   ggcgcctggt gcagcatggg cggcccgcgg gcttgggcgc tgctctgcct cgggctcctg     120
   ctcccgggag gcggcgctgc gtggagcatc ggggcagctc cgttctccgg acgcaggaac     180
   tggtgctcct atgtggtgac ccgcaccatc tcatgccatg tgcagaatgg cacctacctt     240
   cagcgagtgc tgcagaactg cccctggccc atgagctgtc cggggagcag ctacagaact     300
   gtggtgagac ccacatacaa ggtgatgtac aagatagtga ccgcccgtga gtggaggtgc     360
   tgccctgggc actcaggagt gagctgcgag gaagttgcag cttcctctgc ctccttggag     420
   cccatgtggt cgggcagtac catgcggcgg atggcgcttc ggcccacagc cttctcaggt     480
   tgtctcaact gcagcaaagt gtcagagctg acagagcggc tgaaggtgct ggaggccaag     540
   atgaccatgc tgactgtcat agagcagcca gtacctccaa caccagctac ccctgaggac     600
   cctgccccgc tctggggtcc ccctcctgcc cagggcagcc ccggagatgg aggcctccag     660
   ggagacccat tgctgtccaa caccttcact gagaccaaca accactggcc ccagggaccc     720
   actgggcctc caggccctcc agggcccatg ggtccccctg ggcctcctgg ccccacaggt 780
   gtccctggga gtcctggtca cataggaccc ccaggcccca ctggacccaa aggaatctct 840
   ggccacccag gagagaaggg cgagagagga ctgcgtgggg agcctggccc ccaaggctct 900
   gctgggcagc ggggggaacc tggccctaag ggagaccctg gtgagaagag ccactggggg 960
   gaggggttgc accagctacg cgaggctttg aagattttag ctgagagggt tttaatcttg 1020
   gaaacaatga ttgggctcta tgaaccagag ctggggtctg gggcgggccc tgccggcaca 1080
   ggcaccccca gcctccttcg gggcaagagg ggcggacatg caaccaacta ccggatcgtg 1140
   gcccccagga gccgggacga gagaggctga gggtggtggc ggcccctgag gcagaccagg 1200
   ccaggcttcc cctcctacct ggactcggcc agctgcctcc agggaccgcc cgtccatatt 1260
   tattaatgtc ctcagggtcc cttctgccat ctaggcctta ggggtaagca ggtctcagtc 1320
   ctggcaccat gcacatgtct gaggctgagc aagggctgag aggagaggct tgggcctcag 1380
   tttccctctg tgaagtgggg ggaggcaggc cttcaaggag ggatagaggt acaaggcttc 1440
   gtctcatctg ctgtctgagc atccaggccc aaaggcactg agggagtcag gagctggggc 1500
   tcggcacatg cagagatgac agggcagggg gcagtcttcc tccccctccc cgaccaaacc 1560
   tcggggagcc ctcctgtgcc cctccctcct tgttgtccag tgctgggctc cccaccccga 1620
   ggtcaggctg cccaatcctc tgactggatc accgggggct tcttgcctca gttcttccct 1680
   ctgagccccc aggccctccc gcatctcagg ttggggatgg ggacatggag aggaaggggc 1740
   cgcctactcc tgcaaatgct tgtgacagat gccaggaggt agatgtgtgc tggccaataa 1800
   aggcccctac ctgattcccc gca 1823
<210> 69
   <211> 1976
   <212> DNA
   <213> Homo sapiens
<400> 69
   cgggcaggca ggcggggagg acaggctggg ggcggcgacc gcgaggggcc gcgcgcggag      60
   ggcgcctggt gcagcatggg cggcccgcgg gcttgggcgc tgctctgcct cgggctcctg     120
   ctcccgggag gcggcgctgc gtggagcatc ggggcagctc cgttctccgg acgcaggaac     180
   tggtgctcct atgtggtgac ccgcaccatc tcatgccatg tgcagaatgg cacctacctt     240
   cagcgagtgc tgcagaactg cccctggccc atgagctgtc cggggagcag ctacagaact     300
   gtggtgagac ccacatacaa ggtgatgtac aagatagtga ccgcccgtga gtggaggtgc     360
   tgccctgggc actcaggagt gagctgcgag gaaggttgtc tcaactgcag caaagtgtca     420
   gagctgacag agcggctgaa ggtgctggag gccaagatga ccatgctgac tgtcatagag     480
   cagccagtac ctccaacacc agctacccct gaggaccctg ccccgctctg gggtccccct     540
   cctgcccagg gcagccccgg agatggaggc ctccaggacc aagtcggtgc ttgggggctt     600
   cccgggccca ccggccccaa gggagatgcc ggcagtcggg gcccaatggg gatgagaggc     660
   ccaccaggtc cacagggccc cccagggagc cctggccggg ctggagctgt gggcacccct     720
   ggagagaggg gacctcctgg gccaccaggg cctcctggcc cccctgggcc cccagcccct     780
   gttgggccac cccatgcccg gatctcccag catggagacc cattgctgtc caacaccttc     840
   actgagacca acaaccactg gccccaggga cccactgggc ctccaggccc tccagggccc     900
   atgggtcccc ctgggcctcc tggccccaca ggtgtccctg ggagtcctgg tcacatagga     960
   cccccaggcc ccactggacc caaaggaatc tctggccacc caggagagaa gggcgagaga    1020
   ggactgcgtg gggagcctgg cccccaaggc tctgctgggc agcgggggga acctggccct    1080
   aagggagacc ctggtgagaa gagccactgg ggggaggggt tgcaccagct acgcgaggct    1140
   ttgaagattt tagctgagag ggttttaatc ttggaaacaa tgattgggct ctatgaacca    1200
   gagctggggt ctggggcggg ccctgccggc acaggcaccc ccagcctcct tcggggcaag    1260
   aggggcggac atgcaaccaa ctaccggatc gtggccccca ggagccggga cgagagaggc    1320
   tgagggtggt ggcggcccct gaggcagacc aggccaggct tcccctccta cctggactcg    1380
   gccagctgcc tccagggacc gcccgtccat atttattaat gtcctcaggg tcccttctgc    1440
   catctaggcc ttaggggtaa gcaggtctca gtcctggcac catgcacatg tctgaggctg    1500
   agcaagggct gagaggagag gcttgggcct cagtttccct ctgtgaagtg gggggaggca    1560
   ggccttcaag gagggataga ggtacaaggc ttcgtctcat ctgctgtctg agcatccagg    1620
   cccaaaggca ctgagggagt caggagctgg ggctcggcac atgcagagat gacagggcag    1680
   ggggcagtct tcctccccct ccccgaccaa acctcgggga gccctcctgt gcccctccct    1740
   ccttgttgtc cagtgctggg ctccccaccc cgaggtcagg ctgcccaatc ctctgactgg    1800
   atcaccgggg gcttcttgcc tcagttcttc cctctgagcc cccaggccct cccgcatctc    1860
   aggttgggga tggggacatg gagaggaagg ggccgcctac tcctgcaaat gcttgtgaca    1920
   gatgccagga ggtagatgtg tgctggccaa taaaggcccc tacctgattc cccgca        1976
<210> 70
   <211> 2111
   <212> DNA
   <213> Homo sapiens
<400> 70
   cgggcaggca ggcggggagg acaggctggg ggcggcgacc gcgaggggcc gcgcgcggag 60
   ggcgcctggt gcagcatggg cggcccgcgg gcttgggcgc tgctctgcct cgggctcctg 120
   ctcccgggag gcggcgctgc gtggagcatc ggggcagctc cgttctccgg acgcaggaac 180
   tggtgctcct atgtggtgac ccgcaccatc tcatgccatg tgcagaatgg cacctacctt 240
   cagcgagtgc tgcagaactg cccctggccc atgagctgtc cggggagcag ctacagaact 300
   gtggtgagac ccacatacaa ggtgatgtac aagatagtga ccgcccgtga gtggaggtgc 360
   tgccctgggc actcaggagt gagctgcgag gaagttgcag cttcctctgc ctccttggag 420
   cccatgtggt cgggcagtac catgcggcgg atggcgcttc ggcccacagc cttctcaggt 480
   tgtctcaact gcagcaaagt gtcagagctg acagagcggc tgaaggtgct ggaggccaag 540
   atgaccatgc tgactgtcat agagcagcca gtacctccaa caccagctac ccctgaggac 600
   cctgccccgc tctggggtcc ccctcctgcc cagggcagcc ccggagatgg aggcctccag 660
   gggctgccag gagccataga gagtgtgagg gtcccgctgc ttccccgaaa tgaccaagtc 720
   ggtgcttggg ggcttcccgg gcccaccggc cccaagggag atgccggcag tcggggccca 780
   atggggatga gaggcccacc aggtccacag ggccccccag ggagccctgg ccgggctgga 840
   gctgtgggca cccctggaga gaggggacct cctgggccac cagggcctcc tggcccccct 900
   gggcccccag cccctgttgg gccaccccat gcccggatct cccagcatgg agacccattg 960
   ctgtccaaca ccttcactga gaccaacaac cactggcccc agggacccac tgggcctcca 1020
   ggccctccag ggcccatggg tccccctggg cctcctggcc ccacaggtgt ccctgggagt 1080
   cctggtcaca taggaccccc aggccccact ggacccaaag gaatctctgg ccacccagga 1140
   gagaagggcg agagaggact gcgtggggag cctggccccc aaggctctgc tgggcagcgg 1200
   ggggaacctg gccctaaggg agaccctggt gagaagagcc actgggggga ggggttgcac 1260
   cagctacgcg aggctttgaa gattttagct gagagggttt taatcttgga aacaatgatt 1320
   gggctctatg aaccagagct ggggtctggg gcgggccctg ccggcacagg cacccccagc 1380
   ctccttcggg gcaagagggg cggacatgca accaactacc ggatcgtggc ccccaggagc 1440
   cgggacgaga gaggctgagg gtggtggcgg cccctgaggc agaccaggcc aggcttcccc 1500
   tcctacctgg actcggccag ctgcctccag ggaccgcccg tccatattta ttaatgtcct 1560
   cagggtccct tctgccatct aggccttagg ggtaagcagg tctcagtcct ggcaccatgc 1620
   acatgtctga ggctgagcaa gggctgagag gagaggcttg ggcctcagtt tccctctgtg 1680
   aagtgggggg aggcaggcct tcaaggaggg atagaggtac aaggcttcgt ctcatctgct 1740
   gtctgagcat ccaggcccaa aggcactgag ggagtcagga gctggggctc ggcacatgca 1800
   gagatgacag ggcagggggc agtcttcctc cccctccccg accaaacctc ggggagccct 1860
   cctgtgcccc tccctccttg ttgtccagtg ctgggctccc caccccgagg tcaggctgcc 1920
   caatcctctg actggatcac cgggggcttc ttgcctcagt tcttccctct gagcccccag 1980
   gccctcccgc atctcaggtt ggggatgggg acatggagag gaaggggccg cctactcctg 2040
   caaatgcttg tgacagatgc caggaggtag atgtgtgctg gccaataaag gcccctacct   2100
   gattccccgc a                                                         2111
<210> 71
   <211> 707
   <212> DNA
   <213> Homo sapiens
<400> 71
   agatgaccat gctgactgtc atagagcagc cagtacctcc aacaccagct acccctgagg     60
   accctgcccc gctctggggt ccccctcctg cccagggcag ccccggagat ggaggcctcc    120
   aggggctgcc aggagccata gagagtgtga gggtcccgct gcttccccga aatgaccaag    180
   tcggtgcttg ggggcttccc gggcccaccg gccccaaggg agatgccggc agtcggggcc     240
   caatggggat gagaggccca ccaggtccac agggcccccc agggagccct ggccgggctg     300
   gagctgtggg cacccctgga gagaggggac ctcctgggcc accagggcct cctggccccc    360
   ctgggccccc agcccctgtt gggccacccc atgcccggat ctcccagcat ggagacccat    420
   tgctgtccaa caccttcact gagaccaaca accactggcc ccagggaccc actgggcctc    480
   caggccctcc agggcccatg ggtccccctg ggcctcctgg ccccacaggt gtccctggga    540
   gtcctggtca cataggaccc ccaggcccca ctggacccaa aggaatctct ggccacccag    600
   gagagaaggg cgagagagga ctgcgtgggg agcctggccc ccaaggctct gctgggcagc    660
   ggggggaacc tggccctaag ggagaccctg gtgagaagag ccactgg                  707
<210> 72
   <211> 2034
   <212> DNA
   <213> Homo sapiens
<400> 72
   ggtgagtgcc cgcaatgctg ccccacagct cctctggcca tcccctccac caggtgggcc    60
   cttccctgct cctgacatgg ccaggatgac ctgggccctt tcatctactt gcctcttcac   120
   tcagcacccc accacggagt gccctgccca cgcctgggct ccatgaagtc ctctcttatg   180
   ttcactgacc cacattccct gggcacctac acttatcagg ctctgagctg ggcactgggt   240
   ggggtcagac atgtccctgc ccttctggag cttccatgct gctgggagca gggctgggca   300
   gaggagaagc agcaatgctt gccccatgtg accagggttt ctatgagggg ttttgggggt   360
   ttgggagccc caaggaagga agactcagcc tggacgaggt ggagaactag gtgctgtgct   420
   catccccctg ttagactacc aggcagccta ggcctgtgga ctccggggcc ctctctcatg   480
   cccactgctc caggctgcct tgtcctgtcg ctcaaggcca ccctgggcct ccttgcctcc   540
   tgtataccca caaatccgtg tgattccatt gcaggtccac agggcccccc agggagccct   600
   ggccgggctg gagctgtggg cacccctgga gagaggggac ctcctgggcc accagggcct    660
   cctggccccc ctgggccccc agcccctgtt gggccacccc atgcccggat ctcccagcat    720
   ggtgagtccc cctgggatcc cagcaggtgg aggtgggggt ggagtagcca tcagcacagt    780
   gcccgctacc atctgccacg tgccttctgt gtgccagccc tgctcacgat aggccacatg    840
   tgacccagtc ctccagcagg cgccgttgtc ctcctgtggt tacaggtgag gaacactgag    900
   gaccagagag ggaaggtggc ttgccagggt cccacagcct gggcgtaggg gaacggcttc    960
   aaacccaggc tgcctccaga acctgtgctt agagccaccg ggcatcaggc cctcccaagc   1020
   cttggaactg gctggaatcc agttctcgga acactgggac gcaaaagacc cggcggcagg   1080
   aagtgagtcc tgaactccca aggccacagg cccggcccct cctccaggcc ctgacgtgcg   1140
   tccttggctt cttccctttg gcagcccagc ctgacctgcc catgggctgc caggggtcag   1200
   agtgtggagc gccaggtttc agcctcttct ccactgtgtt tttggtgcac aacccagcac   1260
   accattcatt cattctgcca tcccagcatt cattccatct cactatccat acgatgggga   1320
   caatgacagt gccagcctcc cagagctgcg taacatccat gtacagaagc ctggcacaca   1380
   gtaggtggtg gataaatggt atcttttatt gtcattccca tttgacaggt gacagtacag   1440
   gctctgaaaa gtagaaagtg ttgctggatg tcaccagctg gattgcagtg gggttagaac   1500
   ccacatctcc ctgcctcctg gtcttgcggg accaacactc tccacactcc tcaccctgga   1560
   gcaggtgccc aggtggtacc agccatgctg caggctgccc catagggcag tccaagctgt   1620
   cttggcagag gtggcaggtg aagactaacc accccactct acccagctct actcactcat   1680
   catctttgct cacccaggag acccattgct gtccaacacc ttcactgaga ccaacaacca   1740
   ctggccccag ggacccactg ggcctccagg ccctccaggg cccatgggtc cccctgggcc   1800
   tcctggcccc acaggtgtcc ctgggagtcc tggtcacata gtgagtagtt ctccttgtac   1860
   tctcacccat gtgtctgtcc atctttccat ctatgcatac atccatacat ctgtccatca   1920
   tccacccttg tatccatcta tccatccatc cattcatcct tccattcatt cattcaacaa   1980
   gtatttattg agcacttaat atgcaaacta ccttccataa atcttattca atcc         2034
<210> 73
   <211> 518
   <212> PRT
   <213> Homo sapiens
<400> 73
   Met Leu Ala Ala Ala Ser Lys Tyr Arg His Gly Asn Met Val Phe Phe
   1               5                   10                  15
   Asp Val Leu Gly Leu Phe Val Ile Ala Tyr Pro Ser Arg Ile Gly Ser
               20                  25                  30
   Ile Ile Asn Tyr Met Val Val Met Gly Val Val Leu Tyr Leu Gly Lys
           35                  40                  45
   Lys Phe Leu Gln Pro Lys His Lys Thr Gly Asn Tyr Lys Lys Asp Phe
       50                  55                  60
   Leu Cys Gly Leu Gly Ile Thr Leu Ile Ser Trp Phe Thr Ser Leu Val
   65                  70                  75                  80
   Thr Val Leu Ile Ile Ala Val Phe Ile Ser Leu Ile Gly Gln Ser Leu
                   85                  90                  95
   Ser Trp Tyr Asn His Phe Tyr Val Ser Val Cys Leu Tyr Gly Thr Ala
               100                 105                 110
   Thr Val Ala Lys Ile Ile Leu Ile His Thr Leu Ala Lys Arg Phe Tyr
           115                 120                 125
   Tyr Met Asn Ala Ser Ala Gln Tyr Leu Gly Glu Val Phe Phe Asp Ile
       130                 135                 140
   Ser Leu Phe Val His Cys Cys Phe Leu Val Thr Leu Thr Tyr Gln Gly
   145                 150                 155                 160
   Leu Cys Ser Ala Phe Ile Ser Ala Val Trp Val Ala Phe Pro Leu Leu
                   165                 170                 175
   Thr Lys Leu Cys Val His Lys Asp Phe Lys Gln His Gly Ala Gln Gly
               180                 185                 190
   Lys Phe Ile Ala Phe Tyr Leu Leu Gly Met Phe Ile Pro Tyr Leu Tyr
           195                 200                 205
   Ala Leu Tyr Leu Ile Trp Ala Val Phe Glu Met Phe Thr Pro Ile Leu
       210                 215                 220
   Gly Arg Ser Gly Ser Glu Ile Pro Pro Asp Val Val Leu Ala Ser Ile
   225                 230                 235                 240
   Leu Ala Gly Cys Thr Met Ile Leu Ser Ser Tyr Phe Ile Asn Phe Ile
                   245                 250                 255
   Tyr Leu Ala Lys Ser Thr Lys Lys Thr Met Leu Thr Leu Thr Leu Val
               260                 265                 270
   Cys Ala Ile Thr Phe Leu Leu Val Cys Ser Gly Thr Phe Phe Pro Tyr
           275                 280                 285
   Ser Ser Asn Pro Ala Asn Pro Lys Pro Lys Arg Val Phe Leu Gln His
       290                 295                 300
   Met Thr Arg Thr Phe His Asp Leu Glu Gly Asn Ala Val Lys Arg Asp
   305                 310                 315                 320
   Ser Gly Ile Trp Ile Asn Gly Phe Asp Tyr Thr Gly Ile Ser His Ile
                   325                 330                 335
   Thr Pro His Ile Pro Glu Ile Asn Asp Ser Ile Arg Ala His Cys Glu
               340                 345                 350
   Glu Asn Ala Pro Leu Cys Gly Phe Pro Trp Tyr Leu Pro Val His Phe
           355                 360                 365
   Leu Ile Arg Lys Asn Trp Tyr Leu Pro Ala Pro Glu Val Ser Pro Arg
       370                 375                 380
   Asn Pro Pro His Phe Arg Leu Ile Ser Lys Glu Gln Thr Pro Trp Asp
   385                 390                 395                 400
   Ser Ile Lys Leu Thr Phe Glu Ala Thr Gly Pro Ser His Met Ser Phe
                   405                 410                 415
   Tyr Val Arg Ala His Lys Gly Ser Thr Leu Ser Gln Trp Ser Leu Gly
               420                 425                 430
   Asn Gly Thr Pro Val Thr Ser Lys Gly Gly Asp Tyr Phe Val Phe Tyr
           435                 440                 445
   Ser His Gly Leu Gln Ala Ser Ala Trp Gln Phe Trp Ile Glu Val Gln
       450                 455                 460
   Val Ser Glu Glu His Pro Glu Gly Met Val Thr Val Ala Ile Ala Ala
   465                 470                 475                 480
   His Tyr Leu Ser Gly Glu Asp Lys Arg Ser Pro Gln Leu Asp Ala Leu
                   485                 490                 495
   Lys Glu Lys Phe Pro Asp Trp Thr Phe Pro Ser Ala Trp Val Cys Thr
               500                 505                 510
   Tyr Asp Leu Phe Val Phe
   515
<210> 74
   <211> 904
   <212> PRT
   <213> Homo sapiens
<400> 74
   Met Glu Trp Gly Ser Glu Ser Ala Ala Val Arg Arg His Arg Val Gly
   1               5                   10                  15
   Val Glu Arg Arg Glu Gly Ala Ala Ala Ala Pro Pro Pro Glu Arg Glu
               20                  25                  30
   Ala Arg Ala Gln Glu Pro Leu Val Asp Gly Cys Ser Gly Gly Gly Arg
           35                  40                  45
   Thr Arg Lys Arg Ser Pro Gly Gly Ser Gly Gly Ala Ser Arg Gly Ala
       50                  55                  60
   Gly Thr Gly Leu Ser Glu Val Arg Ala Ala Leu Gly Leu Ala Leu Tyr
   65                  70                  75                  80
   Leu Ile Ala Leu Arg Thr Leu Val Gln Leu Ser Leu Gln Gln Leu Val
                   85                  90                  95
   Leu Arg Gly Ala Ala Gly His Arg Gly Glu Phe Asp Ala Leu Gln Ala
               100                 105                 110
   Arg Asp Tyr Leu Glu His Ile Thr Ser Ile Gly Pro Arg Thr Thr Gly
           115                 120                 125
   Ser Pro Glu Asn Glu Ile Leu Thr Val His Tyr Leu Leu Glu Gln Ile
       130                 135                 140
   Lys Leu Ile Glu Val Gln Ser Asn Ser Leu His Lys Ile Ser Val Asp
   145                 150                 155                 160
   Val Gln Arg Pro Thr Gly Ser Phe Ser Ile Asp Phe Leu Gly Gly Phe
                   165                 170                 175
   Thr Ser Tyr Tyr Asp Asn Ile Thr Asn Val Val Val Lys Leu Glu Pro
               180                 185                 190
   Arg Asp Gly Ala Gln His Ala Val Leu Ala Asn Cys His Phe Asp Ser
           195                 200                 205
   Val Ala Asn Ser Pro Gly Ala Ser Asp Asp Ala Val Ser Cys Ser Val
       210                 215                 220
   Met Leu Glu Val Leu Arg Val Leu Ser Thr Ser Ser Glu Ala Leu His
   225                 230                 235                 240
   His Ala Val Ile Phe Leu Phe Asn Gly Ala Glu Glu Asn Val Leu Gln
                   245                 250                 255
   Ala Ser His Gly Phe Ile Thr Gln His Pro Trp Ala Ser Leu Ile Arg
               260                 265                 270
   Ala Phe Ile Asn Leu Glu Ala Ala Gly Val Gly Gly Lys Glu Leu Val
           275                 280                 285
   Phe Gln Thr Gly Pro Glu Asn Pro Trp Leu Val Gln Ala Tyr Val Ser
       290                 295                 300
   Ala Ala Lys His Pro Phe Ala Ser Val Val Ala Gln Glu Val Phe Gln
   305                 310                 315                 320
   Ser Gly Ile Ile Pro Ser Asp Thr Asp Phe Arg Ile Tyr Arg Asp Phe
                   325                 330                 335
   Gly Asn Ile Pro Gly Ile Asp Leu Ala Phe Ile Glu Asn Gly Tyr Ile
               340                 345                 350
   Tyr His Thr Lys Tyr Asp Thr Ala Asp Arg Ile Leu Thr Asp Ser Ile
           355                 360                 365
   Gln Arg Ala Gly Asp Asn Ile Leu Ala Val Leu Lys His Leu Ala Thr
       370                 375                 380
   Ser Asp Met Leu Ala Ala Ala Ser Lys Tyr Arg His Gly Asn Met Val
   385                 390                 395                 400
   Phe Phe Asp Val Leu Gly Leu Phe Val Ile Ala Tyr Pro Ser Arg Ile
                   405                 410                 415
   Gly Ser Ile Ile Asn Tyr Met Val Val Met Gly Val Val Leu Tyr Leu
               420                 425                 430
   Gly Lys Lys Phe Leu Gln Pro Lys His Lys Thr Gly Asn Tyr Lys Lys
           435                 440                 445
   Asp Phe Leu Cys Gly Leu Gly Ile Thr Leu Ile Ser Trp Phe Thr Ser
       450                 455                 460
   Leu Val Thr Val Leu Ile Ile Ala Val Phe Ile Ser Leu Ile Gly Gln
   465                 470                 475                 480
   Ser Leu Ser Trp Tyr Asn His Phe Tyr Val Ser Val Cys Leu Tyr Gly
                   485                 490                 495
   Thr Ala Thr Val Ala Lys Ile Ile Leu Ile His Thr Leu Ala Lys Arg
               500                 505                 510
   Phe Tyr Tyr Met Asn Ala Ser Ala Gln Tyr Leu Gly Glu Val Phe Phe
           515                 520                 525
   Asp Ile Ser Leu Phe Val His Cys Cys Phe Leu Val Thr Leu Thr Tyr
       530                 535                 540
   Gln Gly Leu Cys Ser Ala Phe Ile Ser Ala Val Trp Val Ala Phe Pro
   545                 550                 555                 560
   Leu Leu Thr Lys Leu Cys Val His Lys Asp Phe Lys Gln His Gly Ala
                   565                 570                 575
   Gln Gly Lys Phe Ile Ala Phe Tyr Leu Leu Gly Met Phe Ile Pro Tyr
               580                 585                 590
   Leu Tyr Ala Leu Tyr Leu Ile Trp Ala Val Phe Glu Met Phe Thr Pro
           595                 600                 605
   Ile Leu Gly Arg Ser Gly Ser Glu Ile Pro Pro Asp Val Val Leu Ala
       610                 615                 620
   Ser Ile Leu Ala Gly Cys Thr Met Ile Leu Ser Ser Tyr Phe Ile Asn
   625                 630                 635                 640
   Phe Ile Tyr Leu Ala Lys Ser Thr Lys Lys Thr Met Leu Thr Leu Thr
                   645                 650                 655
   Leu Val Cys Ala Ile Thr Phe Leu Leu Val Cys Ser Gly Thr Phe Phe
               660                 665                 670
   Pro Tyr Ser Ser Asn Pro Ala Asn Pro Lys Pro Lys Arg Val Phe Leu
           675                 680                 685
   Gln His Met Thr Arg Thr Phe His Asp Leu Glu Gly Asn Ala Val Lys
       690                 695                 700
   Arg Asp Ser Gly Ile Trp Ile Asn Gly Phe Asp Tyr Thr Gly Ile Ser
   705                 710                 715                 720
   His Ile Thr Pro His Ile Pro Glu Ile Asn Asp Ser Ile Arg Ala His
                   725                 730                 735
   Cys Glu Glu Asn Ala Pro Leu Cys Gly Phe Pro Trp Tyr Leu Pro Val
               740                 745                 750
   His Phe Leu Ile Arg Lys Asn Trp Tyr Leu Pro Ala Pro Glu Val Ser
           755                 760                 765
   Pro Arg Asn Pro Pro His Phe Arg Leu Ile Ser Lys Glu Gln Thr Pro
       770                 775                 780
   Trp Asp Ser Ile Lys Leu Thr Phe Glu Ala Thr Gly Pro Ser His Met
   785                 790                 795                 800
   Ser Phe Tyr Val Arg Ala His Lys Gly Ser Thr Leu Ser Gln Trp Ser
                   805                 810                 815
   Leu Gly Asn Gly Thr Pro Val Thr Ser Lys Gly Gly Asp Tyr Phe Val
               820                 825                 830
   Phe Tyr Ser His Gly Leu Gln Ala Ser Ala Trp Gln Phe Trp Ile Glu
           835                 840                 845
   Val Gln Val Ser Glu Glu His Pro Glu Gly Met Val Thr Val Ala Ile
       850                 855                 860
   Ala Ala His Tyr Leu Ser Gly Glu Asp Lys Arg Ser Pro Gln Leu Asp
   865                 870                 875                 880
   Ala Leu Lys Glu Lys Phe Pro Asp Trp Thr Phe Pro Ser Ala Trp Val
                   885                 890                 895
   Cys Thr Tyr Asp Leu Phe Val Phe
               900
<210> 75
   <211> 419
   <212> PRT
   <213> Homo sapiens
<400> 75
   Met Val Val Met Gly Val Val Leu Tyr Leu Gly Lys Lys Phe Leu Gln
   1               5                   10                  15
   Pro Lys His Lys Thr Gly Asn Tyr Lys Lys Asp Phe Leu Cys Gly Leu
               20                  25                  30
   Gly Ile Thr Leu Ile Ser Trp Phe Thr Ser Leu Val Thr Val Leu Ile
           35                  40                  45
   Ile Ala Val Phe Ile Ser Leu Ile Gly Gln Ser Leu Ser Trp Tyr Asn
       50                  55                  60
   His Phe Tyr Val Ser Val Cys Leu Tyr Gly Thr Ala Thr Val Ala Lys
   65                  70                  75                  80
   Ile Ile Leu Ile His Thr Leu Ala Lys Arg Phe Tyr Tyr Met Asn Ala
                   85                  90                  95
   Ser Ala Gln Tyr Leu Gly Glu Val Phe Phe Asp Ile Ser Leu Phe Val
               100                 105                 110
   His Cys Cys Phe Leu Val Thr Leu Thr Tyr Gln Gly Leu Cys Ser Ala
           115                 120                 125
   Phe Ile Ser Ala Val Trp Val Ala Phe Pro Leu Leu Thr Lys Leu Cys
       130                 135                 140
   Val His Lys Asp Phe Lys Gln His Gly Ala Gln Gly Lys Phe Ile Ala
   145                 150                 155                 160
   Phe Tyr Leu Leu Gly Met Phe Ile Pro Tyr Leu Tyr Ala Leu Tyr Leu
                   165                 170                 175
   Ile Trp Ala Val Phe Glu Met Phe Thr Pro Ile Leu Gly Arg Ser Gly
               180                 185                 190
   Ser Glu Ile Pro Pro Asp Val Val Leu Ala Ser Ile Leu Ala Gly Cys
           195                 200                 205
   Thr Met Ile Leu Ser Ser Tyr Phe Ile Asn Phe Ile Tyr Leu Ala Lys
       210                 215                 220
   Ser Thr Lys Lys Thr Met Leu Thr Leu Thr Leu Val Cys Ala Ile Thr
   225                 230                 235                 240
   Phe Leu Leu Val Cys Ser Gly Thr Phe Phe Pro Tyr Ser Ser Asn Pro
                   245                 250                 255
   Ala Asn Pro Lys Pro Lys Arg Val Phe Leu Gln His Met Thr Arg Thr
               260                 265                 270
   Phe His Asp Leu Glu Gly Asn Ala Val Lys Arg Asp Ser Gly Ile Trp
           275                 280                 285
   Ile Asn Gly Phe Asp Tyr Thr Gly Ile Ser His Ile Thr Pro His Ile
       290                 295                 300
   Pro Glu Ile Asn Asp Ser Ile Arg Ala His Cys Glu Glu Asn Ala Pro
   305                 310                 315                 320
   Leu Cys Gly Phe Pro Trp Tyr Leu Pro Val His Phe Leu Ile Arg Lys
                   325                 330                 335
   Asn Trp Tyr Leu Pro Ala Pro Glu Val Ser Pro Arg Asn Pro Pro His
               340                 345                 350
   Phe Arg Leu Ile Ser Lys Glu Gln Thr Pro Trp Asp Ser Ile Lys Leu
           355                 360                 365
   Thr Phe Glu Ala Thr Ala Cys Leu Pro Ile Leu Gln Ile Leu Asp Leu
       370                 375                 380
   Pro Ala Ser Thr Ile Met Thr Lys Pro Tyr Val Leu Leu Cys Ser Ser
   385                 390                 395                 400
   Pro Gln Arg Val Asn Thr Phe Ser Val Val Ser Trp Gln Trp His Pro
                   405                 410                 415
   Ser His Lys
<210> 76
   <211> 4974
   <212> DNA
   <213> Homo sapiens
<400> 76
   ggcgcgggga ccgggctgtc tgaggtgcgc gccgcgctgg ggctcgcgct ctacctgatc      60
   gcgctgcgga cgctggtgca gctctcgctg cagcagctcg tgctacgcgg ggccgctgga     120
   caccgcgggg agttcgacgc gctccaagcc agggattatc ttgaacacat aacctccatt     180
   ggccccagga ctacaggaag tccagaaaat gaaattctga ccgtgcacta ccttttggaa     240
   cagattaaac tgattgaagt gcaaagcaac agccttcata agatttcagt agatgtacaa     300
   cggcccacag gctcttttag cattgatttc ttgggaggtt ttacaagcta ttatgacaac     360
   atcaccaatg ttgtggtaaa gctggaaccc agagatggag cccagcatgc tgtcttggct     420
   aattgtcatt ttgactcagt agcaaactca ccaggccagt catggtttca ttactcagca    480
   cccctgggct agcttgattc gtgcattcat taacctagag gcagcaggtg taggagggaa    540
   agaacttgta ttccaaacag gtcctgaaaa tccttggttg gttcaagctt atgtttcagc    600
   agctaaacac ccttttgctt ctgtggtggc tcaggaggtt tttcagagtg gaatcattcc     660
   ttcagatact gactttcgta tctacaggga ttttgggaac attccaggaa tagacttagc    720
   ttttattgag aatggataca tttatcacac caagtatgac acagcggaca gaattctaac    780
   agattccatt cagagagcag gtgacaacat tttagcagtt cttaagcatc tagctacatc    840
   tgatatgctg gctgctgctt ctaagtatcg acatggaaac atggtcttct ttgatgtgct    900
   gggcctgttt gtcattgcct acccctctcg tattggctca atcataaact acatggtggt    960
   aatgggtgtt gttttgtacc tgggcaaaaa atttttgcag cccaaacata agactggtaa   1020
   ctacaagaag gacttcttgt gtggacttgg catcactttg atcagctggt tcactagcct   1080
   tgttaccgtt ctcattatag cagtgttcat ctctcttatt ggacagtctc tctcatggta   1140
   taaccacttc tatgtctccg tttgtctgta tggaactgca actgtagcca aaataatact   1200
   tatacatact cttgcgaaaa gattttatta catgaatgcc agtgcccagt atctgggaga   1260
   agtatttttt gacatttcgc tgtttgtcca ttgctgtttt cttgttaccc tcacttacca   1320
   aggactttgc tcggcgttta ttagtgctgt ctgggtagca ttcccattgc tcacaaagct   1380
   ctgtgtgcat aaggacttca agcagcatgg tgcccaagga aaatttattg ctttttacct   1440
   tttggggatg tttattcctt atctttatgc attgtacctc atctgggcag tatttgagat   1500
   gtttacccct atcctcggga gaagtggttc tgaaatccca cctgatgttg tgctggcatc   1560
   cattttggct ggctgtacaa tgattctctc gtcctatttt attaacttca tctaccttgc   1620
   caagagcaca aaaaaaacca tgctaacttt aactttggta tgtgcaatta cattcctcct   1680
   tgtttgcagt ggaacatttt ttccatatag ctccaatcct gctaatccga agccaaagag   1740
   agtgtttctt cagcatatga ctagaacatt ccatgacttg gaaggaaatg cagttaaacg   1800
   ggactctgga atatggatca atgggtttga ttatactgga atttctcaca taacccctca   1860
   cattcctgag atcaatgata gtatccgagc tcactgtgag gagaatgcac ctctttgtgg   1920
   ttttccttgg tatcttccag tgcactttct gatcaggaaa aactggtatc ttcctgcccc   1980
   agaagtttct ccaagaaatc ctcctcattt ccgactcata tccaaagaac agacaccttg   2040
   ggattctata aaattgactt ttgaagcaac aggaccaagc catatgtcct tctatgttcg   2100
   agcccacaaa gggtcaacac tttctcagtg gtctcttggc aatggcaccc cagtcacaag   2160
   taaaggagga gactactttg tcttttactc ccatggactc caggcctctg catggcagtt   2220
   ctggatagaa gtgcaggttt cagaagaaca tcctgaagga atggtcaccg tggccattgc   2280
   tgcccactat ctgtctgggg aagacaagag atcccctcaa ctggatgctc tgaaggaaaa   2340
   gttcccagat tggacatttc cctctgcctg ggtgtgcacc tacgatctct ttgtatttta   2400
   atcttgtgga tgagctctaa gtacatgccc agtggatact ccatgtgaca tggtttctcc   2460
   ctatgttacg tggatgtttg taacgtaagt caatgaattt taatgatcat atgttcaaag   2520
   agctttctgg gttaacgctt ttcagggcca agcactataa gggtttagct gtggcgcagt   2580
   gatgcatggc ctgttgacac ttgaaaatgc cagtcttttg gcacttcagc acatgtgggt   2640
   actgccacta cacacacgtc attttatatg accttaagga caaagccaac aatccacttc   2700
   aatagctgcc cctttaggat caagaaagat gtacactgtc agagcattgt taatgagaca   2760
   aaagttgttt ccaatttaag ccccaaaacc atttgttgta ttagtggatg gtgggtaaaa   2820
   tatcattcac tgaggtaatg attccccttg agaatataac tctgtgtagg tcactggaaa   2880
   gtgattgcca tagggctggg agagaagcat tgcactcttg aggctgtagc ctgtgtcaag   2940
   ctgtttcttc aggcagcctc tcaaatgtgc tttgtctctc tgtgctgagg cctggaccct   3000
   gtgctgagct ggtgactcac tgtcctgaca agtggacaca cagatgcact gctgtgctgc   3060
   tttcctgagg tggttttcta tgcctgtttt cctctgaaac atgtctgtta cccctctcca   3120
   tcttaccaag ttgaaaaggg gaatatttgg ccacataccc ctctggtttt cgtaggttct   3180
   tttggttcag aatattgttt gtgccagtac atgaccttaa cttccttcct cagagcactg   3240
   agctgccatc tgggctattc tggggtagaa ggaaggctgg gagtggtggg aattttataa   3300
   atatttattc tcttttcttt gtttcatagg agtcttgtgt tatacaaggt tagtccttca   3360
   tggtataatc ttactgatgc actgggccta tctttttgtt ttccagccag ttgaatagat   3420
   tagtttttct cagtaactta ctatccagca gactggcttt cctgagactt gaggttgtgg   3480
   cttatactgg aatgagacca ctgtacgtgt aggtggttca gatcctgcgt aatggcagca   3540
   tgaggactta aaaggtggtt ttcattttga agatggctat gtagcttgta aggtgtatca   3600
   cagcagtacc tctcatggct ttttggttcc agcagtgagg gcattggtga gatcaatggt   3660
   aaactgtgca agctttcttt ttatcattag gaaatgtgaa acgttggaca aattttgagt   3720
   tttaacaagg acaaaaagtt gaaagaaaag gcacagttaa caaaaaaggg tggctagatt   3780
   tatcttgggt gatggaggaa atgagagagg aatgctcttg aaaggtggtc tgtggatctg   3840
   tctgaataga aagagcacag taagtatgca ttgccggaga aaacgtcctt gaagctgctt   3900
   gtctcatgtg tatgatgtgc tttttaaatc atgcccctcg ttgcctgcct aatctgtgac   3960
   tccctaaaaa ctaactgggc ccatgtagat ggggctgcaa ccagagctga ataacatgtt   4020
   aggctcacac atgcatcagc actgcacact ggaatcattg ctcttcctgg actttgtaga   4080
   aatcagtctc aagtgcttca agagtctggc tcctgctact tttatctgtc aggtagcaca   4140
   taaggtttgc agggtttata ttttgtatag aatcacagtt gtggagaaaa agtaataatt 4200
   tctcaatgaa ttttaaaaat gggcctattt tctatccccg tggttcatct gatataatta 4260
   gtgttccctg tgaattcccc ccctctatgg gaaggatgcc tttactcttt atcagtaata 4320
   aattatgact gttttcatat tgccttaggg ttatttccct gtgtaaacca ttgtcttttg 4380
   ttttggtttt ctttagcatt atgaagcttt ggtattgtac aaggtcagta gtaagatgct 4440
   cactagtctc agggcttgtg taatattctg ggaggtcatt taaatgccag aaatggtcaa 4500
   gcaattatac acagtattta tgactctgtt aagcataccg tttgtctgtc acattagtag 4560
   attctgagat taaaaaaaat ttttaaagag tgatcattta aataatttct aaaagggtct 4620
   tttcaagctc taacaaagtc actaacaaat gcattatttt ctacagaatt agatgttagt 4680
   agtacagtac tgcatattca gggaaaaagt gtgaggaatt gatttcaaaa tagttcgttc 4740
   ttgtgtttga cctaagaatg attgtcgcat gaagtgtttg tttttacagt ttagcatata 4800
   taaacaaaca tgataggatt ccttaagatg ttaccaccca gggggccaca agccagcctg 4860
   ctgtctcagg aagctgtaga aggagtgttt gtcaatttct tgtcactggt ttgctgactt 4920
   actgaggatt aattgttgcc ttacaatgtt actgaaataa actgtttaat atac 4974
<210> 77
   <211> 5338
   <212> DNA
   <213> Homo sapiens
<400> 77
   ggccggggct gtcgcgggtt ggggcggttg ggctggcagc tgaggctcgt ggccatggag      60
   tggggttctg agtcggctgc tgtgaggcgg caccgcgtcg gagtagagcg tcgagaggga     120
   gcggcggccg cgccaccgcc ggagagggag gcccgagcgc aggagcctct ggtggatggg     180
   tgcagcggcg gcgggaggac gcggaagagg agccccgggg gtagcggcgg cgcgagcagg     240
   ggcgcgggga ccgggctgtc tgaggtgcgc gccgcgctgg ggctcgcgct ctacctgatc     300
   gcgctgcgga cgctggtgca gctctcgctg cagcagctcg tgctacgcgg ggccgctgga     360
   caccgcgggg agttcgacgc gctccaagcc agggattatc ttgaacacat aacctccatt     420
   ggccccagga ctacaggaag tccagaaaat gaaattctga ccgtgcacta ccttttggaa     480
   cagattaaac tgattgaagt gcaaagcaac agccttcata agatttcagt agatgtacaa     540
   cggcccacag gctcttttag cattgatttc ttgggaggtt ttacaagcta ttatgacaac     600
   atcaccaatg ttgtggtaaa gctggaaccc agagatggag cccagcatgc tgtcttggct     660
   aattgtcatt ttgactcagt agcaaactca ccaggtgcca gtgatgatgc agttagctgc     720
   tcagtgatgc tggaagtcct tcgcgtcttg tcaacatctt cagaagcctt gcatcatgct     780
   gtcatatttc tctttaatgg tgctgaggaa aatgtcttgc aagccagtca tggtttcatt 840
   actcagcacc cctgggctag cttgattcgt gcattcatta acctagaggc agcaggtgta 900
   ggagggaaag aacttgtatt ccaaacaggt cctgaaaatc cttggttggt tcaagcttat 960
   gtttcagcag ctaaacaccc ttttgcttct gtggtggctc aggaggtttt tcagagtgga 1020
   atcattcctt cagatactga ctttcgtatc tacagggatt ttgggaacat tccaggaata 1080
   gacttagctt ttattgagaa tggatacatt tatcacacca agtatgacac agcggacaga 1140
   attctaacag attccattca gagagcaggt gacaacattt tagcagttct taagcatcta 1200
   gctacatctg atatgctggc tgctgcttct aagtatcgac atggaaacat ggtcttcttt 1260
   gatgtgctgg gcctgtttgt cattgcctac ccctctcgta ttggctcaat cataaactac 1320
   atggtggtaa tgggtgttgt tttgtacctg ggcaaaaaat ttttgcagcc caaacataag 1380
   actggtaact acaagaagga cttcttgtgt ggacttggca tcactttgat cagctggttc 1440
   actagccttg ttaccgttct cattatagca gtgttcatct ctcttattgg acagtctctc 1500
   tcatggtata accacttcta tgtctccgtt tgtctgtatg gaactgcaac tgtagccaaa 1560
   ataatactta tacatactct tgcgaaaaga ttttattaca tgaatgccag tgcccagtat 1620
   ctgggagaag tattttttga catttcgctg tttgtccatt gctgttttct tgttaccctc 1680
   acttaccaag gactttgctc ggcgtttatt agtgctgtct gggtagcatt cccattgctc 1740
   acaaagctct gtgtgcataa ggacttcaag cagcatggtg cccaaggaaa atttattgct 1800
   ttttaccttt tggggatgtt tattccttat ctttatgcat tgtacctcat ctgggcagta 1860
   tttgagatgt ttacccctat cctcgggaga agtggttctg aaatcccacc tgatgttgtg 1920
   ctggcatcca ttttggctgg ctgtacaatg attctctcgt cctattttat taacttcatc 1980
   taccttgcca agagcacaaa aaaaaccatg ctaactttaa ctttggtatg tgcaattaca 2040
   ttcctccttg tttgcagtgg aacatttttt ccatatagct ccaatcctgc taatccgaag 2100
   ccaaagagag tgtttcttca gcatatgact agaacattcc atgacttgga aggaaatgca 2160
   gttaaacggg actctggaat atggatcaat gggtttgatt atactggaat ttctcacata 2220
   acccctcaca ttcctgagat caatgatagt atccgagctc actgtgagga gaatgcacct 2280
   ctttgtggtt ttccttggta tcttccagtg cactttctga tcaggaaaaa ctggtatctt 2340
   cctgccccag aagtttctcc aagaaatcct cctcatttcc gactcatatc caaagaacag 2400
   acaccttggg attctataaa attgactttt gaagcaacag gaccaagcca tatgtccttc 2460
   tatgttcgag cccacaaagg gtcaacactt tctcagtggt ctcttggcaa tggcacccca 2520
   gtcacaagta aaggaggaga ctactttgtc ttttactccc atggactcca ggcctctgca 2580
   tggcagttct ggatagaagt gcaggtttca gaagaacatc ctgaaggaat ggtcaccgtg 2640
   gccattgctg cccactatct gtctggggaa gacaagagat cccctcaact ggatgctctg 2700
   aaggaaaagt tcccagattg gacatttccc tctgcctggg tgtgcaccta cgatctcttt 2760
   gtattttaat cttgtggatg agctctaagt acatgcccag tggatactcc atgtgacatg 2820
   gtttctccct atgttacgtg gatgtttgta acgtaagtca atgaatttta atgatcatat 2880
   gttcaaagag ctttctgggt taacgctttt cagggccaag cactataagg gtttagctgt 2940
   ggcgcagtga tgcatggcct gttgacactt gaaaatgcca gtcttttggc acttcagcac 3000
   atgtgggtac tgccactaca cacacgtcat tttatatgac cttaaggaca aagccaacaa 3060
   tccacttcaa tagctgcccc tttaggatca agaaagatgt acactgtcag agcattgtta 3120
   atgagacaaa agttgtttcc aatttaagcc ccaaaaccat ttgttgtatt agtggatggt 3180
   gggtaaaata tcattcactg aggtaatgat tccccttgag aatataactc tgtgtaggtc 3240
   actggaaagt gattgccata gggctgggag agaagcattg cactcttgag gctgtagcct 3300
   gtgtcaagct gtttcttcag gcagcctctc aaatgtgctt tgtctctctg tgctgaggcc 3360
   tggaccctgt gctgagctgg tgactcactg tcctgacaag tggacacaca gatgcactgc 3420
   tgtgctgctt tcctgaggtg gttttctatg cctgttttcc tctgaaacat gtctgttacc 3480
   cctctccatc ttaccaagtt gaaaagggga atatttggcc acatacccct ctggttttcg 3540
   taggttcttt tggttcagaa tattgtttgt gccagtacat gaccttaact tccttcctca 3600
   gagcactgag ctgccatctg ggctattctg gggtagaagg aaggctggga gtggtgggaa 3660
   ttttataaat atttattctc ttttctttgt ttcataggag tcttgtgtta tacaaggtta 3720
   gtccttcatg gtataatctt actgatgcac tgggcctatc tttttgtttt ccagccagtt 3780
   gaatagatta gtttttctca gtaacttact atccagcaga ctggctttcc tgagacttga 3840
   ggttgtggct tatactggaa tgagaccact gtacgtgtag gtggttcaga tcctgcgtaa 3900
   tggcagcatg aggacttaaa aggtggtttt cattttgaag atggctatgt agcttgtaag 3960
   gtgtatcaca gcagtacctc tcatggcttt ttggttccag cagtgagggc attggtgaga 4020
   tcaatggtaa actgtgcaag ctttcttttt atcattagga aatgtgaaac gttggacaaa 4080
   ttttgagttt taacaaggac aaaaagttga aagaaaaggc acagttaaca aaaaagggtg 4140
   gctagattta tcttgggtga tggaggaaat gagagaggaa tgctcttgaa aggtggtctg 4200
   tggatctgtc tgaatagaaa gagcacagta agtatgcatt gccggagaaa acgtccttga 4260
   agctgcttgt ctcatgtgta tgatgtgctt tttaaatcat gcccctcgtt gcctgcctaa 4320
   tctgtgactc cctaaaaact aactgggccc atgtagatgg ggctgcaacc agagctgaat 4380
   aacatgttag gctcacacat gcatcagcac tgcacactgg aatcattgct cttcctggac 4440
   tttgtagaaa tcagtctcaa gtgcttcaag agtctggctc ctgctacttt tatctgtcag 4500
   gtagcacata aggtttgcag ggtttatatt ttgtatagaa tcacagttgt ggagaaaaag 4560
   taataatttc tcaatgaatt ttaaaaatgg gcctattttc tatccccgtg gttcatctga 4620
   tataattagt gttccctgtg aattcccccc ctctatggga aggatgcctt tactctttat 4680
   cagtaataaa ttatgactgt tttcatattg ccttagggtt atttccctgt gtaaaccatt 4740
   gtcttttgtt ttggttttct ttagcattat gaagctttgg tattgtacaa ggtcagtagt 4800
   aagatgctca ctagtctcag ggcttgtgta atattctggg aggtcattta aatgccagaa 4860
   atggtcaagc aattatacac agtatttatg actctgttaa gcataccgtt tgtctgtcac 4920
   attagtagat tctgagatta aaaaaaattt ttaaagagtg atcatttaaa taatttctaa 4980
   aagggtcttt tcaagctcta acaaagtcac taacaaatgc attattttct acagaattag 5040
   atgttagtag tacagtactg catattcagg gaaaaagtgt gaggaattga tttcaaaata 5100
   gttcgttctt gtgtttgacc taagaatgat tgtcgcatga agtgtttgtt tttacagttt 5160
   agcatatata aacaaacatg ataggattcc ttaagatgtt accacccagg gggccacaag 5220
   ccagcctgct gtctcaggaa gctgtagaag gagtgtttgt caatttcttg tcactggttt 5280
   gctgacttac tgaggattaa ttgttgcctt acaatgttac tgaaataaac tgtttaat 5338
<210> 78
   <211> 5387
   <212> DNA
   <213> Homo sapiens
<400> 78
   ggccggggct gtcgcgggtt ggggcggttg ggctggcagc tgaggctcgt ggccatggag      60
   tggggttctg agtcggctgc tgtgaggcgg caccgcgtcg gagtagagcg tcgagaggga     120
   gcggcggccg cgccaccgcc ggagagggag gcccgagcgc aggagcctct ggtggatggg     180
   tgcagcggcg gcgggaggac gcggaagagg agccccgggg gtagcggcgg cgcgagcagg     240
   ggcgcgggga ccgggctgtc tgaggtgcgc gccgcgctgg ggctcgcgct ctacctgatc     300
   gcgctgcgga cgctggtgca gctctcgctg cagcagctcg tgctacgcgg ggccgctgga     360
   caccgcgggg agttcgacgc gctccaagcc agggattatc ttgaacacat aacctccatt     420
   ggccccagga ctacaggaag tccagaaaat gaaattctga ccgtgcacta ccttttggaa     480
   cagattaaac tgattgaagt gcaaagcaac agccttcata agatttcagt agatgtacaa     540
   cggcccacag gctcttttag cattgatttc ttgggaggtt ttacaagcta ttatgacaac     600
   atcaccaatg ttgtggtaaa gctggaaccc agagatggag cccagcatgc tgtcttggct     660
   aattgtcatt ttgactcagt agcaaactca ccaggtgcca gtgatgatgc agttagctgc     720
   tcagtgatgc tggaagtcct tcgcgtcttg tcaacatctt cagaagcctt gcatcatgct     780
   gtcatatttc tctttaatgg tgctgaggaa aatgtcttgc aagccagtca tggtttcatt     840
   actcagcacc cctgggctag cttgattcgt gcattcatta acctagaggc agcaggtgta    900
   ggagggaaag aacttgtatt ccaaacaggt cctgaaaatc cttggttggt tcaagcttat    960
   gtttcagcag ctaaacaccc ttttgcttct gtggtggctc aggaggtttt tcagagtgga   1020
   atcattcctt cagatactga ctttcgtatc tacagggatt ttgggaacat tccaggaata   1080
   gacttagctt ttattgagaa tggatacatt tatcacacca agtatgacac agcggacaga   1140
   attctaacag attccattca gagagcaggt gacaacattt tagcagttct taagcatcta   1200
   gctacatctg atatgctggc tgctgcttct aagtatcgac atggaaacat ggtcttcttt   1260
   gatgtgctgg gcctgtttgt cattgcctac ccctctcgta ttggctcaat cataaactac   1320
   atggtggtaa tgggtgttgt tttgtacctg ggcaaaaaat ttttgcagcc caaacataag   1380
   actggtaact acaagaagga cttcttgtgt ggacttggca tcactttgat cagctggttc   1440
   actagccttg ttaccgttct cattatagca gtgttcatct ctcttattgg acagtctctc   1500
   tcatggtata accacttcta tgtctccgtt tgtctgtatg gaactgcaac tgtagccaaa   1560
   ataatactta tacatactct tgcgaaaaga ttttattaca tgaatgccag tgcccagtat   1620
   ctgggagaag tattttttga catttcgctg tttgtccatt gctgttttct tgttaccctc   1680
   acttaccaag gactttgctc ggcgtttatt agtgctgtct gggtagcatt cccattgctc   1740
   acaaagctct gtgtgcataa ggacttcaag cagcatggtg cccaaggaaa atttattgct   1800
   ttttaccttt tggggatgtt tattccttat ctttatgcat tgtacctcat ctgggcagta   1860
   tttgagatgt ttacccctat cctcgggaga agtggttctg aaatcccacc tgatgttgtg   1920
   ctggcatcca ttttggctgg ctgtacaatg attctctcgt cctattttat taacttcatc   1980
   taccttgcca agagcacaaa aaaaaccatg ctaactttaa ctttggtatg tgcaattaca   2040
   ttcctccttg tttgcagtgg aacatttttt ccatatagct ccaatcctgc taatccgaag   2100
   ccaaagagag tgtttcttca gcatatgact agaacattcc atgacttgga aggaaatgca   2160
   gttaaacggg actctggaat atggatcaat gggtttgatt atactggaat ttctcacata   2220
   acccctcaca ttcctgagat caatgatagt atccgagctc actgtgagga gaatgcacct   2280
   ctttgtggtt ttccttggta tcttccagtg cactttctga tcaggaaaaa ctggtatctt   2340
   cctgccccag aagtttctcc aagaaatcct cctcatttcc gactcatatc caaagaacag   2400
   acaccttggg attctataaa attgactttt gaagcaacag cctgcctgcc tatccttcag   2460
   attttggact tgccagcctc aacaatcatg accaagccat atgtccttct atgttcgagc   2520
   ccacaaaggg tcaacacttt ctcagtggtc tcttggcaat ggcaccccag tcacaagtaa   2580
   aggaggagac tactttgtct tttactccca tggactccag gcctctgcat ggcagttctg   2640
   gatagaagtg caggtttcag aagaacatcc tgaaggaatg gtcaccgtgg ccattgctgc   2700
   ccactatctg tctggggaag acaagagatc ccctcaactg gatgctctga aggaaaagtt 2760
   cccagattgg acatttccct ctgcctgggt gtgcacctac gatctctttg tattttaatc 2820
   ttgtggatga gctctaagta catgcccagt ggatactcca tgtgacatgg tttctcccta 2880
   tgttacgtgg atgtttgtaa cgtaagtcaa tgaattttaa tgatcatatg ttcaaagagc 2940
   tttctgggtt aacgcttttc agggccaagc actataaggg tttagctgtg gcgcagtgat 3000
   gcatggcctg ttgacacttg aaaatgccag tcttttggca cttcagcaca tgtgggtact 3060
   gccactacac acacgtcatt ttatatgacc ttaaggacaa agccaacaat ccacttcaat 3120
   agctgcccct ttaggatcaa gaaagatgta cactgtcaga gcattgttaa tgagacaaaa 3180
   gttgtttcca atttaagccc caaaaccatt tgttgtatta gtggatggtg ggtaaaatat 3240
   cattcactga ggtaatgatt ccccttgaga atataactct gtgtaggtca ctggaaagtg 3300
   attgccatag ggctgggaga gaagcattgc actcttgagg ctgtagcctg tgtcaagctg 3360
   tttcttcagg cagcctctca aatgtgcttt gtctctctgt gctgaggcct ggaccctgtg 3420
   ctgagctggt gactcactgt cctgacaagt ggacacacag atgcactgct gtgctgcttt 3480
   cctgaggtgg ttttctatgc ctgttttcct ctgaaacatg tctgttaccc ctctccatct 3540
   taccaagttg aaaaggggaa tatttggcca catacccctc tggttttcgt aggttctttt 3600
   ggttcagaat attgtttgtg ccagtacatg accttaactt ccttcctcag agcactgagc 3660
   tgccatctgg gctattctgg ggtagaagga aggctgggag tggtgggaat tttataaata 3720
   tttattctct tttctttgtt tcataggagt cttgtgttat acaaggttag tccttcatgg 3780
   tataatctta ctgatgcact gggcctatct ttttgttttc cagccagttg aatagattag 3840
   tttttctcag taacttacta tccagcagac tggctttcct gagacttgag gttgtggctt 3900
   atactggaat gagaccactg tacgtgtagg tggttcagat cctgcgtaat ggcagcatga 3960
   ggacttaaaa ggtggttttc attttgaaga tggctatgta gcttgtaagg tgtatcacag 4020
   cagtacctct catggctttt tggttccagc agtgagggca ttggtgagat caatggtaaa 4080
   ctgtgcaagc tttcttttta tcattaggaa atgtgaaacg ttggacaaat tttgagtttt 4140
   aacaaggaca aaaagttgaa agaaaaggca cagttaacaa aaaagggtgg ctagatttat 4200
   cttgggtgat ggaggaaatg agagaggaat gctcttgaaa ggtggtctgt ggatctgtct 4260
   gaatagaaag agcacagtaa gtatgcattg ccggagaaaa cgtccttgaa gctgcttgtc 4320
   tcatgtgtat gatgtgcttt ttaaatcatg cccctcgttg cctgcctaat ctgtgactcc 4380
   ctaaaaacta actgggccca tgtagatggg gctgcaacca gagctgaata acatgttagg 4440
   ctcacacatg catcagcact gcacactgga atcattgctc ttcctggact ttgtagaaat 4500
   cagtctcaag tgcttcaaga gtctggctcc tgctactttt atctgtcagg tagcacataa 4560
   ggtttgcagg gtttatattt tgtatagaat cacagttgtg gagaaaaagt aataatttct 4620
   caatgaattt taaaaatggg cctattttct atccccgtgg ttcatctgat ataattagtg 4680
   ttccctgtga attccccccc tctatgggaa ggatgccttt actctttatc agtaataaat 4740
   tatgactgtt ttcatattgc cttagggtta tttccctgtg taaaccattg tcttttgttt 4800
   tggttttctt tagcattatg aagctttggt attgtacaag gtcagtagta agatgctcac 4860
   tagtctcagg gcttgtgtaa tattctggga ggtcatttaa atgccagaaa tggtcaagca 4920
   attatacaca gtatttatga ctctgttaag cataccgttt gtctgtcaca ttagtagatt 4980
   ctgagattaa aaaaaatttt taaagagtga tcatttaaat aatttctaaa agggtctttt 5040
   caagctctaa caaagtcact aacaaatgca ttattttcta cagaattaga tgttagtagt 5100
   acagtactgc atattcaggg aaaaagtgtg aggaattgat ttcaaaatag ttcgttcttg 5160
   tgtttgacct aagaatgatt gtcgcatgaa gtgtttgttt ttacagttta gcatatataa 5220
   acaaacatga taggattcct taagatgtta ccacccaggg ggccacaagc cagcctgctg 5280
   tctcaggaag ctgtagaagg agtgtttgtc aatttcttgt cactggtttg ctgacttact 5340
   gaggattaat tgttgcctta caatgttact gaaataaact gtttaat 5387
<210> 79
   <211> 245
   <212> PRT
   <213> Homo sapiens
<400> 79
   Met Thr Leu Phe Pro Val Leu Leu Phe Leu Val Ala Gly Leu Leu Pro
   1               5                   10                  15
   Ser Phe Pro Ala Asn Glu Asp Lys Asp Pro Ala Phe Thr Ala Leu Leu
               20                  25                  30
   Thr Thr Gln Thr Gln Val Gln Arg Glu Ile Val Asn Lys His Asn Glu
           35                  40                  45
   Leu Arg Arg Ala Val Ser Pro Pro Ala Arg Asn Met Leu Lys Met Glu
       50                  55                  60
   Trp Asn Lys Glu Ala Ala Ala Asn Ala Gln Lys Trp Ala Asn Gln Cys
   65                  70                  75                  80
   Asn Tyr Arg His Ser Asn Pro Lys Asp Arg Met Thr Ser Leu Lys Cys
                   85                  90                  95
   Gly Glu Asn Leu Tyr Met Ser Ser Ala Ser Ser Ser Trp Ser Gln Ala
   100 105 110
   Ile Gln Ser Trp Phe Asp Glu Tyr Asn Asp Phe Asp Phe Gly Val Gly
   115 120 125
   Pro Lys Thr Pro Asn Ala Val Val Gly His Tyr Thr Gln Val Val Trp
   130 135 140
   Tyr Ser Ser Tyr Leu Val Gly Cys Gly Asn Ala Tyr Cys Pro Asn Gln
   145 150 155 160
   Lys Val Leu Lys Tyr Tyr Tyr Val Cys Gln Tyr Cys Pro Ala Gly Asn
   165 170 175
   Trp Ala Asn Arg Leu Tyr Val Pro Tyr Glu Gln Gly Ala Pro Cys Ala
   180 185 190
   Ser Cys Pro Asp Asn Cys Asp Asp Gly Leu Cys Thr Asn Gly Cys Lys
   195 200 205
   Tyr Glu Asp Leu Tyr Ser Asn Cys Lys Ser Leu Lys Leu Thr Leu Thr
   210 215 220
   Cys Lys His Gln Leu Val Arg Asp Ser Cys Lys Ala Ser Cys Asn Cys
   225 230 235 240
   Ser Asn Ser Ile Tyr
   245
<210> 80
   <211> 245
   <212> PRT
   <213> Homo sapiens
<400> 80
   Met Thr Leu Phe Pro Val Leu Leu Phe Leu Val Ala Gly Leu Leu Pro
   1 5 10 15
   Ser Phe Pro Ala Asn Glu Asp Lys Asp Pro Ala Phe Thr Ala Leu Leu
   20 25 30
   Thr Thr Gln Thr Gln Val Gln Arg Glu Ile Val Asn Lys His Asn Glu
   35 40 45
   Leu Arg Arg Ala Val Ser Pro Pro Ala Arg Asn Met Leu Lys Met Glu
   50 55 60
   Trp Asn Lys Glu Ala Ala Ala Asn Ala Gln Lys Trp Ala Asn Gln Cys
   65                  70                  75                  80
   Asn Tyr Arg His Ser Asn Pro Lys Asp Arg Met Thr Ser Leu Lys Cys
                   85                  90                  95
   Gly Glu Asn Leu Tyr Met Ser Ser Ala Ser Ser Ser Trp Ser Gln Ala
               100                 105                 110
   Ile Gln Ser Trp Phe Asp Glu Tyr Asn Asp Phe Asp Phe Gly Val Gly
           115                 120                 125
   Pro Lys Thr Pro Asn Ala Val Val Gly His Tyr Thr Gln Val Val Trp
       130                 135                 140
   Tyr Ser Ser Tyr Leu Val Gly Cys Gly Asn Ala Tyr Cys Pro Asn Gln
   145                 150                 155                 160
   Lys Val Leu Lys Tyr Tyr Tyr Val Cys Gln Tyr Cys Pro Ala Gly Asn
                   165                 170                 175
   Trp Ala Asn Arg Leu Tyr Val Pro Tyr Glu Gln Gly Ala Pro Cys Ala
               180                 185                 190
   Ser Cys Pro Asp Asn Cys Asp Asp Gly Leu Cys Thr Asn Gly Cys Lys
           195                 200                 205
   Tyr Glu Asp Leu Tyr Ser Asn Cys Lys Ser Leu Lys Leu Thr Leu Thr
       210                 215                 220
   Cys Lys His Gln Leu Val Arg Asp Ser Cys Lys Ala Ser Cys Asn Cys
   225                 230                 235                 240
   Ser Asn Ser Ile Tyr
                   245
<210> 81
   <211> 245
   <212> PRT
   <213> Homo sapiens
<400> 81
   Met Thr Leu Phe Pro Val Leu Leu Phe Leu Val Ala Gly Leu Leu Pro
   1               5                   10                  15
   Ser Phe Pro Ala Asn Glu Asp Lys Asp Pro Ala Phe Thr Ala Leu Leu
               20                  25                  30
   Thr Thr Gln Thr Gln Val Gln Arg Glu Ile Val Asn Lys His Asn Glu
           35                  40                  45
   Leu Arg Arg Ala Val Ser Pro Pro Ala Arg Asn Met Leu Lys Met Glu
       50                  55                  60
   Trp Asn Lys Glu Ala Ala Ala Asn Ala Gln Lys Trp Ala Asn Gln Cys
   65                  70                  75                  80
   Asn Tyr Arg His Ser Asn Pro Lys Asp Arg Met Thr Ser Leu Lys Cys
                   85                  90                  95
   Gly Glu Asn Leu Tyr Met Ser Ser Ala Ser Ser Ser Trp Ser Gln Ala
               100                 105                 110
   Ile Gln Ser Trp Phe Asp Glu Tyr Asn Asp Phe Asp Phe Gly Val Gly
           115                 120                 125
   Pro Lys Thr Pro Asn Ala Val Val Gly His Tyr Thr Gln Val Val Trp
       130                 135                 140
   Tyr Ser Ser Tyr Leu Val Gly Cys Gly Asn Ala Tyr Cys Pro Asn Gln
   145                 150                 155                 160
   Lys Val Leu Lys Tyr Tyr Tyr Val Cys Gln Tyr Cys Pro Ala Gly Asn
                   165                 170                 175
   Trp Ala Asn Arg Leu Tyr Val Pro Tyr Glu Gln Gly Ala Pro Cys Ala
               180                 185                 190
   Ser Cys Pro Asp Asn Cys Asp Asp Gly Leu Cys Thr Asn Gly Cys Lys
           195                 200                 205
   Tyr Glu Asp Leu Tyr Ser Asn Cys Lys Ser Leu Lys Leu Thr Leu Thr
       210                 215                 220
   Cys Lys His Gln Leu Val Arg Asp Ser Cys Lys Ala Ser Cys Asn Cys
   225                 230                 235                 240
   Ser Asn Ser Ile Tyr
                   245
<210> 82
   <211> 2073
   <212> DNA
   <213> Homo sapiens
<400> 82
   cccagcaatg acattattcc cagtgctgtt gttcctggtt gctgggctgc ttccatcttt      60
   tccagcaaat gaagataagg atcccgcttt tactgctttg ttaaccaccc aaacacaagt     120
   gcaaagggag attgtgaata agcacaatga actgaggaga gcagtatctc cccctgccag     180
   aaacatgctg aagatggaat ggaacaaaga ggctgcagca aatgcccaaa agtgggcaaa     240
   ccagtgcaat tacagacaca gtaacccaaa ggatcgaatg acaagtctaa aatgtggtga     300
   gaatctctac atgtcaagtg cctccagctc atggtcacaa gcaatccaaa gctggtttga     360
   tgagtacaat gattttgact ttggtgtagg gccaaagact cccaacgcag tggttggaca     420
   ttatacacag gttgtttggt actcttcata cctcgttgga tgtggaaatg cctactgtcc     480
   caatcaaaaa gttctaaaat actactatgt ttgccaatat tgtcctgctg gtaattgggc     540
   taatagacta tatgtccctt atgaacaagg agcaccttgt gccagttgcc cagataactg     600
   tgacgatgga ctatgcacca atggttgcaa gtacgaagat ctctatagta actgtaaaag     660
   tttgaagctc acattaacct gtaaacatca gttggtcagg gacagttgca aggcctcctg     720
   caattgttca aacagcattt attaaatacg cattacacac cgagtagggc tatgtagaga     780
   ggagtcagat tatctactta gatttggcat ctacttagat ttaacatata ctagctgaga     840
   aattgtaggc atgtttgata cacatttgat ttcaaatgtt tttcttctgg atctgctttt     900
   tattttacaa aaatattttt catacaaatg gttaaaaaga aacaaaatct ataacaacaa     960
   ctttggattt ttatatataa actttgtgat ttaaatttac tgaatttaat tagggtgaaa    1020
   attttgaaag ttgtattctc atatgactaa gttcactaaa accctggatt gaaagtgaaa    1080
   attatgttcc tagaacaaaa tgtacaaaaa gaacaatata attttcacat gaacccttgg    1140
   ctgtagttgc ctttcctagc tccactctaa ggctaagcat cttcaaagac gttttcccat    1200
   atgctgtctt aattcttttc actcattcac ccttcttccc aatcatctgg ctggcatcct    1260
   cacaattgag ttgaagctgt tcctcctaaa acaatcctga cttttatttt gccaaaatca    1320
   atacaatcct ttgaattttt tatctgcata aattttacag tagaatatga tcaaaccttc    1380
   atttttaaac ctctcttctc tttgacaaaa cttccttaaa aaagaataca agataatata    1440
   ggtaaatacc ctccactcaa ggaggtagaa ctcagtcctc tcccttgtga gtcttcacta    1500
   aaatcagtga ctcacttcca aagagtggag tatggaaagg gaaacatagt aactttacag    1560
   gggagaaaaa tgacaaatga cgtcttcacc aagtgatcaa aattaacgtc accagtgata    1620
   agtcattcag atttgttcta gataatcttt ctaaaaattc ataatcccaa tctaattatg    1680
   agctaaaaca tccagcaaac tcaagttgaa ggacattcta caaaatatcc ctggggtatt    1740
   ttagagtatt cctcaaaact gtaaaaatca tggaaaataa gggaatcctg agaaacaatc    1800
   acagaccaca tgagactaag gagacatgtg agccaaatgc aatgtgcttc ttggatcaga    1860
   tcctggaaca gaaaaagatc agtaatgaaa aaactgatga agtctgaata gaatctggag   1920
   tatttttaac agtagtgttg atttcttaat cttgataaat atagcagggt aatgtaagat   1980
   gataacgtta gagaaactga aactgggtga gggctatcta ggaattctct gtactatctt   2040
   accaaatttt cggtaagtct aagaaagcaa tgc                                2073
<210> 83
   <211> 2081
   <212> DNA
   <213> Homo sapiens
<400> 83
   ctggaaacca ctgcaatgac attattccca gtgctgttgt tcctggttgc tgggctgctt     60
   ccatcttttc cagcaaatga agataaggat cccgctttta ctgctttgtt aaccacccaa    120
   acacaagtgc aaagggagat tgtgaataag cacaatgaac tgaggagagc agtatctccc    180
   cctgccagaa acatgctgaa gatggaatgg aacaaagagg ctgcagcaaa tgcccaaaag    240
   tgggcaaacc agtgcaatta cagacacagt aacccaaagg atcgaatgac aagtctaaaa    300
   tgtggtgaga atctctacat gtcaagtgcc tccagctcat ggtcacaagc aatccaaagc    360
   tggtttgatg agtacaatga ttttgacttt ggtgtagggc caaagactcc caacgcagtg    420
   gttggacatt atacacaggt tgtttggtac tcttcatacc tcgttggatg tggaaatgcc    480
   tactgtccca atcaaaaagt tctaaaatac tactatgttt gccaatattg tcctgctggt    540
   aattgggcta atagactata tgtcccttat gaacaaggag caccttgtgc cagttgccca    600
   gataactgtg acgatggact atgcaccaat ggttgcaagt acgaagatct ctatagtaac    660
   tgtaaaagtt tgaagctcac attaacctgt aaacatcagt tggtcaggga cagttgcaag    720
   gcctcctgca attgttcaaa cagcatttat taaatacgca ttacacaccg agtagggcta    780
   tgtagagagg agtcagatta tctacttaga tttggcatct acttagattt aacatatact    840
   agctgagaaa ttgtaggcat gtttgataca catttgattt caaatgtttt tcttctggat    900
   ctgcttttta ttttacaaaa atatttttca tacaaatggt taaaaagaaa caaaatctat    960
   aacaacaact ttggattttt atatataaac tttgtgattt aaatttactg aatttaatta   1020
   gggtgaaaat tttgaaagtt gtattctcat atgactaagt tcactaaaac cctggattga   1080
   aagtgaaaat tatgttccta gaacaaaatg tacaaaaaga acaatataat tttcacatga   1140
   acccttggct gtagttgcct ttcctagctc cactctaagg ctaagcatct tcaaagacgt   1200
   tttcccatat gctgtcttaa ttcttttcac tcattcaccc ttcttcccaa tcatctggct   1260
   ggcatcctca caattgagtt gaagctgttc ctcctaaaac aatcctgact tttattttgc   1320
   caaaatcaat acaatccttt gaatttttta tctgcataaa ttttacagta gaatatgatc   1380
   aaaccttcat ttttaaacct ctcttctctt tgacaaaact tccttaaaaa agaatacaag   1440
   ataatatagg taaataccct ccactcaagg aggtagaact cagtcctctc ccttgtgagt   1500
   cttcactaaa atcagtgact cacttccaaa gagtggagta tggaaaggga aacatagtaa   1560
   ctttacaggg gagaaaaatg acaaatgacg tcttcaccaa gtgatcaaaa ttaacgtcac   1620
   cagtgataag tcattcagat ttgttctaga taatctttct aaaaattcat aatcccaatc   1680
   taattatgag ctaaaacatc cagcaaactc aagttgaagg acattctaca aaatatccct   1740
   ggggtatttt agagtattcc tcaaaactgt aaaaatcatg gaaaataagg gaatcctgag   1800
   aaacaatcac agaccacatg agactaagga gacatgtgag ccaaatgcaa tgtgcttctt   1860
   ggatcagatc ctggaacaga aaaagatcag taatgaaaaa actgatgaag tctgaataga   1920
   atctggagta tttttaacag tagtgttgat ttcttaatct tgataaatat agcagggtaa   1980
   tgtaagatga taacgttaga gaaactgaaa ctgggtgagg gctatctagg aattctctgt   2040
   actatcttac caaattttcg gtaagtctaa gaaagcaatg c                         2081
<210> 84
   <211> 2122
   <212> DNA
   <213> Homo sapiens
<400> 84
   gatgaaacaa atacttcatc ctgctctgga aaccactgat gacattattc ccagtgctgt     60
   tgttcctggt tgctgggctg cttccatctt ttccagcaaa tgaagataag gatcccgctt    120
   ttactgcttt gttaaccacc caaacacaag tgcaaaggga gattgtgaat aagcacaatg    180
   aactgaggag agcagtatct ccccctgcca gaaacatgct gaagatggaa tggaacaaag    240
   aggctgcagc aaatgcccaa aagtgggcaa accagtgcaa ttacagacac agtaacccaa    300
   aggatcgaat gacaagtcta aaatgtggtg agaatctcta catgtcaagt gcctccagct    360
   catggtcaca agcaatccaa agctggtttg atgagtacaa tgattttgac tttggtgtag    420
   ggccaaagac tcccaacgca gtggttggac attatacaca ggttgtttgg tactcttcat    480
   acctcgttgg atgtggaaat gcctactgtc ccaatcaaaa agttctaaaa tactactatg    540
   tttgccaata ttgtcctgct ggtaattggg ctaatagact atatgtccct tatgaacaag    600
   gagcaccttg tgccagttgc ccagataact gtgacgatgg actatgcacc aatggttgca    660
   agtacgaaga tctctatagt aactgtaaaa gtttgaagct cacattaacc tgtaaacatc    720
   agttggtcag ggacagttgc aaggcctcct gcaattgttc aaacagcatt tattaaatac    780
   gcattacaca ccgagtaggg ctatgtagag aggagtcaga ttatctactt agatttggca    840
   tctacttaga tttaacatat actagctgag aaattgtagg catgtttgat acacatttga    900
   tttcaaatgt ttttcttctg gatctgcttt ttattttaca aaaatatttt tcatacaaat    960
   ggttaaaaag aaacaaaatc tataacaaca actttggatt tttatatata aactttgtga   1020
   tttaaattta ctgaatttaa ttagggtgaa aattttgaaa gttgtattct catatgacta   1080
   agttcactaa aaccctggat tgaaagtgaa aattatgttc ctagaacaaa atgtacaaaa   1140
   agaacaatat aattttcaca tgaacccttg gctgtagttg cctttcctag ctccactcta   1200
   aggctaagca tcttcaaaga cgttttccca tatgctgtct taattctttt cactcattca   1260
   cccttcttcc caatcatctg gctggcatcc tcacaattga gttgaagctg ttcctcctaa   1320
   aacaatcctg acttttattt tgccaaaatc aatacaatcc tttgaatttt ttatctgcat   1380
   aaattttaca gtagaatatg atcaaacctt catttttaaa cctctcttct ctttgacaaa   1440
   acttccttaa aaaagaatac aagataatat aggtaaatac cctccactca aggaggtaga   1500
   actcagtcct ctcccttgtg agtcttcact aaaatcagtg actcacttcc aaagagtgga   1560
   gtatggaaag ggaaacatag taactttaca ggggagaaaa atgacaaatg acgtcttcac   1620
   caagtgatca aaattaacgt caccagtgat aagtcattca gatttgttct agataatctt   1680
   tctaaaaatt cataatccca atctaattat gagctaaaac atccagcaaa ctcaagttga   1740
   aggacattct acaaaatatc cctggggtat tttagagtat tcctcaaaac tgtaaaaatc   1800
   atggaaaata agggaatcct gagaaacaat cacagaccac atgagactaa ggagacatgt   1860
   gagccaaatg caatgtgctt cttggatcag atcctggaac agaaaaagat cagtaatgaa   1920
   aaaactgatg aagtctgaat agaatctgga gtatttttaa cagtagtgtt gatttcttaa   1980
   tcttgataaa tatagcaggg taatgtaaga tgataacgtt agagaaactg aaactgggtg   2040
   agggctatct aggaattctc tgtactatct taccaaattt tcggtaagtc taagaaagca   2100
   atgcaaaata aaaagtgtct tg                                             2122
<210> 85
   <211> 409
   <212> PRT
   <213> Homo sapiens
<400> 85
   Met Glu Glu Ser Trp Glu Ala Ala Pro Gly Gly Gln Ala Gly Ala Glu
   1               5                   10                  15
   Leu Pro Met Glu Pro Val Gly Ser Leu Val Pro Thr Leu Glu Gln Pro
               20                  25                  30
   Gln Val Pro Ala Lys Val Arg Gln Pro Glu Gly Pro Glu Ser Ser Pro
           35                  40                  45
   Ser Pro Ala Gly Ala Val Glu Lys Ala Ala Gly Ala Gly Leu Glu Pro
   50 55 60
   Ser Ser Lys Lys Lys Pro Pro Ser Pro Arg Pro Gly Ser Pro Arg Val
   65 70 75 80
   Pro Pro Leu Ser Leu Gly Tyr Gly Val Cys Pro Glu Pro Pro Ser Pro
   85 90 95
   Gly Pro Ala Leu Val Lys Leu Pro Arg Asn Gly Glu Ala Pro Gly Ala
   100 105 110
   Glu Pro Ala Pro Ser Ala Trp Ala Pro Met Glu Leu Gln Val Asp Val
   115 120 125
   Arg Val Lys Pro Val Gly Ala Ala Gly Gly Ser Ser Thr Pro Ser Pro
   130 135 140
   Arg Pro Ser Thr Arg Phe Leu Lys Val Pro Val Pro Glu Ser Pro Ala
   145 150 155 160
   Phe Ser Arg His Ala Asp Pro Ala His Gln Leu Leu Leu Arg Ala Pro
   165 170 175
   Ser Gln Gly Gly Thr Trp Gly Arg Arg Ser Pro Leu Ala Ala Ala Arg
   180 185 190
   Thr Glu Ser Gly Cys Asp Ala Glu Gly Arg Ala Ser Pro Ala Glu Gly
   195 200 205
   Ser Ala Gly Ser Pro Gly Ser Pro Thr Cys Cys Arg Cys Lys Glu Leu
   210 215 220
   Gly Leu Glu Lys Glu Asp Ala Ala Leu Leu Pro Arg Ala Gly Leu Asp
   225 230 235 240
   Gly Asp Glu Lys Leu Pro Arg Ala Val Thr Leu Thr Gly Leu Pro Met
   245 250 255
   Tyr Val Lys Ser Leu Tyr Trp Ala Leu Ala Phe Met Ala Val Leu Leu
   260 265 270
   Ala Val Ser Gly Val Val Ile Val Val Leu Ala Ser Arg Ala Gly Ala
   275 280 285
   Arg Cys Gln Gln Cys Pro Pro Gly Trp Val Leu Ser Glu Glu His Cys
   290 295 300
   Tyr Tyr Phe Ser Ala Glu Ala Gln Ala Trp Glu Ala Ser Gln Ala Phe
   305                 310                 315                 320
   Cys Ser Ala Tyr His Ala Thr Leu Pro Leu Leu Ser His Thr Gln Asp
                   325                 330                 335
   Phe Leu Gly Arg Tyr Pro Val Ser Arg His Ser Trp Val Gly Ala Trp
               340                 345                 350
   Arg Gly Pro Gln Gly Trp His Trp Ile Asp Glu Ala Pro Leu Pro Pro
           355                 360                 365
   Gln Leu Leu Pro Glu Asp Gly Glu Asp Asn Leu Asp Ile Asn Cys Gly
       370                 375                 380
   Ala Leu Glu Glu Gly Thr Leu Val Ala Ala Asn Cys Ser Thr Pro Arg
   385                 390                 395                 400
   Pro Trp Val Cys Ala Lys Gly Thr Gln
                   405
<210> 86
   <211> 314
   <212> PRT
   <213> Homo sapiens
<400> 86
   Met Glu Glu Ser Trp Glu Ala Ala Pro Gly Gly Gln Ala Gly Ala Glu
   1               5                   10                  15
   Leu Pro Met Glu Pro Val Gly Ser Leu Val Pro Thr Leu Glu Gln Pro
               20                  25                  30
   Gln Val Pro Ala Lys Val Arg Gln Pro Glu Gly Pro Glu Ser Ser Pro
           35                  40                  45
   Ser Pro Ala Gly Ala Val Glu Lys Ala Ala Gly Ala Gly Leu Glu Pro
       50                  55                  60
   Ser Ser Lys Lys Lys Pro Pro Ser Pro Arg Pro Gly Ser Pro Arg Val
   65                  70                  75                  80
   Pro Pro Leu Ser Leu Gly Tyr Gly Val Cys Pro Glu Pro Pro Ser Pro
                   85                  90                  95
   Gly Pro Ala Leu Val Lys Leu Pro Arg Asn Gly Glu Ala Pro Gly Ala
               100                 105                 110
   Glu Pro Ala Pro Ser Ala Trp Ala Pro Met Glu Leu Gln Val Asp Val
           115                 120                 125
   Arg Val Lys Pro Val Gly Ala Ala Gly Gly Ser Ser Thr Pro Ser Pro
       130                 135                 140
   Arg Pro Ser Thr Arg Phe Leu Lys Val Pro Val Pro Glu Ser Pro Ala
   145                 150                 155                 160
   Phe Ser Arg His Ala Asp Pro Ala His Gln Leu Leu Leu Arg Ala Pro
                   165                 170                 175
   Ser Gln Gly Gly Thr Trp Gly Arg Arg Ser Pro Leu Ala Ala Ala Arg
               180                 185                 190
   Thr Glu Ser Gly Cys Asp Ala Glu Gly Arg Ala Ser Pro Ala Glu Gly
           195                 200                 205
   Ser Ala Gly Ser Pro Gly Ser Pro Thr Cys Cys Arg Cys Lys Glu Leu
       210                 215                 220
   Gly Leu Glu Lys Glu Asp Ala Ala Leu Leu Pro Arg Ala Gly Leu Asp
   225                 230                 235                 240
   Gly Asp Glu Lys Leu Pro Arg Ala Val Thr Leu Thr Asp Ser Leu Arg
                   245                 250                 255
   Thr Ala Arg Thr Ile Trp Ile Ser Thr Val Gly Pro Trp Arg Lys Ala
               260                 265                 270
   Arg Trp Trp Leu Gln Thr Ala Ala Leu Gln Asp Pro Gly Ser Val Pro
           275                 280                 285
   Arg Gly Pro Ser Asp Leu Gly Ser Ala Trp Ser Ser Ala Cys Gln Ala
       290                 295                 300
   Asp Ala Ala Pro Pro Thr Gly Glu Ala Ser
   305                 310
<210> 87
   <211> 1544
   <212> DNA
   <213> Homo sapiens
<400> 87
   gagagcgaag ctcctctgca ctgggcccag gtgcgctcct cagcgtctcc gggtggcggg      60
   gcgcgcggga tggaggagtc ttgggaggct gcgcccggag gccaagccgg ggcagagctc     120
   ccaatggagc ccgtgggaag cctggtcccc acgctggagc agccgcaggt gcccgcgaag     180
   gtgcgacaac ctgaaggtcc cgaaagcagc ccaagtccgg ccggggccgt ggagaaggcg     240
   gcgggcgcag gcctggagcc ctcgagcaag aaaaagccgc cttcgcctcg ccccgggtcc     300
   ccgcgcgtgc cgccgctcag cctgggctac ggggtctgcc ccgagccgcc gtcaccgggc     360
   cctgccttgg tcaagctgcc ccggaatggc gaggcgcccg gggctgagcc tgcgcccagc     420
   gcctgggcgc ccatggagct gcaggtagat gtgcgcgtga agcccgtggg cgcggccggt     480
   ggcagcagca cgccatcgcc caggccctcc acgcgcttcc tcaaggtgcc ggtgcccgag     540
   tcccctgcct tctcccgcca cgcggacccg gcgcaccagc tcctgctgcg cgcaccatcc     600
   cagggcggca cgtggggccg ccgctcgccg ctggctgcag cccggacgga gagcggctgc     660
   gacgcagagg gccgggccag ccccgcggaa ggaagcgccg gctccccggg ctcccccacg     720
   tgctgccgct gcaaggagct ggggctggag aaggaggatg cggcgctgtt gccccgcgcg     780
   gggttggacg gcgacgagaa gctgccccgg gccgtaacgc ttacggggct acccatgtac     840
   gtgaagtccc tgtactgggc cctggcgttc atggctgtgc tcctggcagt ctctggggtt     900
   gtcattgtgg tcctggcctc aagagcagga gccagatgcc agcagtgccc cccaggctgg     960
   gtgttgtccg aggagcactg ttactacttc tctgcagaag cgcaggcctg ggaagccagc    1020
   caggctttct gctcagccta ccacgctacc ctccccctgc taagccacac ccaggacttc   1080
   ctgggcagat acccagtctc caggcactcc tgggtggggg cctggcgagg cccccagggc    1140
   tggcactgga tcgacgaggc cccactcccg ccccagctac tccctgagga cggcgaggac    1200
   aatctggata tcaactgtgg ggccctggag gaaggcacgc tggtggctgc aaactgcagc    1260
   actccaagac cctgggtctg tgccaagggg acccagtgat ctgggctctg cctggtcctc    1320
   agcctgccag gcagatgcag caccccctac aggggaggcc agttgagagc ttgggcagcc   1380
   tcttcctgga cccagttatc caggtcttca tgctctgctc aagggggcca catgagcgag    1440
   cctaggagct ggacttcaac ccaggaagat gcatccgagg gaaaggagat tttctatggc    1500
   ctcaggcctg agtgccaata ttagtctcca gcttctgtgg atga                      1544
<210> 88
   <211> 1192
   <212> DNA
   <213> Homo sapiens
<400> 88
   gagagcgaag ctcctctgca ctgggcccag gtgcgctcct cagcgtctcc gggtggcggg      60
   gcgcgcggga tggaggagtc ttgggaggct gcgcccggag gccaagccgg ggcagagctc     120
   ccaatggagc ccgtgggaag cctggtcccc acgctggagc agccgcaggt gcccgcgaag     180
   gtgcgacaac ctgaaggtcc cgaaagcagc ccaagtccgg ccggggccgt ggagaaggcg     240
   gcgggcgcag gcctggagcc ctcgagcaag aaaaagccgc cttcgcctcg ccccgggtcc     300
   ccgcgcgtgc cgccgctcag cctgggctac ggggtctgcc ccgagccgcc gtcaccgggc     360
   cctgccttgg tcaagctgcc ccggaatggc gaggcgcccg gggctgagcc tgcgcccagc     420
   gcctgggcgc ccatggagct gcaggtagat gtgcgcgtga agcccgtggg cgcggccggt     480
   ggcagcagca cgccatcgcc caggccctcc acgcgcttcc tcaaggtgcc ggtgcccgag     540
   tcccctgcct tctcccgcca cgcggacccg gcgcaccagc tcctgctgcg cgcaccatcc     600
   cagggcggca cgtggggccg ccgctcgccg ctggctgcag cccggacgga gagcggctgc     660
   gacgcagagg gccgggccag ccccgcggaa ggaagcgccg gctccccggg ctcccccacg     720
   tgctgccgct gcaaggagct ggggctggag aaggaggatg cggcgctgtt gccccgcgcg     780
   gggttggacg gcgacgagaa gctgccccgg gccgtaacgc ttacggactc cctgaggacg     840
   gcgaggacaa tctggatatc aactgtgggg ccctggagga aggcacgctg gtggctgcaa     900
   actgcagcac tccaagaccc tgggtctgtg ccaaggggac ccagtgatct gggctctgcc     960
   tggtcctcag cctgccaggc agatgcagca ccccctacag gggaggccag ttgagagctt    1020
   gggcagcctc ttcctggacc cagttatcca ggtcttcatg ctctgctcaa gggggccaca    1080
   tgagcgagcc taggagctgg acttcaaccc aggaagatgc atccgaggga aaggagattt    1140
   tctatggcct caggcctgag tgccaatatt agtctccagc ttctgtggat ga            1192
<210> 89
   <211> 21
   <212> DNA
   <213> Homo sapiens
<400> 89
   cgaggacaat ctggatatca a 21
<210> 90
   <211> 21
   <212> DNA
   <213> Homo sapiens
<400> 90
   ctggagccct cgagcaagaa a 21
<210> 91
   <211> 21
   <212> DNA
   <213> Homo sapiens
<400> 91
   cccgtggttc atctgatata a 21
<210> 92
   <211> 21
   <212> DNA
   <213> Homo sapiens
<400> 92
   aaggactttg ctcggcgttt a 21
<210> 93
   <211> 21
   <212> DNA
   <213> Homo sapiens
<400> 93
   tacgtggatg tttgtaacgt a 21
<210> 94
   <211> 21
   <212> DNA
   <213> Homo sapiens
<400> 94
   ctcgtattgg ctcaatcata a 21